# EUROPEAN PATENT APPLICATION

(11) **EP 4 810 057 A1**
(43) Date of publication of application: **23.09.2026**
(21) Application number: 24890742.0
(22) Date of filing: 13.11.2024
(51) Int. Cl.: A61K 39/155, C07K 14/135, C07K 16/10, C12N 15/45, A61P 31/14

(54) **RSV VACCINE COMPOSITION, METHOD, AND USE THEREOF**

(30) Priority: 13.11.2023 CN 202311591607
(71) Applicant: SICHUAN CLOVER BIOPHARMACEUTICALS, INC., Chengdu, Sichuan 610041 (CN)
(72) Inventor: LIANG, Joshua, Chengdu, Sichuan 610041 (CN); SU, Danmei, Chengdu, Sichuan 610041 (CN); LIANG, Peng, Chengdu, Sichuan 610041 (CN); TAN, Wei, Chengdu, Sichuan 610041 (CN)
(74) Representative: Kuttenkeuler, David
(86) International application number: PCT/CN2024/131885
(87) International publication number: WO 2025/103387

(57) **Abstract**

The present invention provides a recombinant peptide and an immunogenic composition comprising the recombinant peptide. The recombinant peptide comprises a soluble respiratory syncytial virus (RSV) antigen, the soluble RSV antigen comprises a recombinant F protein peptide, and the soluble RSV antigen is linked to the C-terminal part of collagen by means of in-frame fusion to form a disulfide-connected trimeric fusion protein. The immunogenic composition may be used to produce an immune response, for example, for treating or preventing an RSV infection. The immunogenic composition can be used in a vaccine composition, for example, as a part of a prophylactic and/or therapeutic vaccine.

## Description

### Technical Field

The present invention relates in some aspects to an immunogenic composition comprising a recombinant peptide and protein, wherein the recombinant peptide and protein comprise a respiratory syncytial virus (RSV) viral antigen and immunogen, e.g., an RSV F protein peptide, and the immunogenic composition is used for treating and/or preventing RSV infection.

### Background Art

Respiratory syncytial virus (RSV) causes respiratory tract infections in adults and children, and is a leading cause of lower respiratory tract infections and hospitalization during infancy and childhood. Despite the high rate of RSV infection, treatments, including prophylactics, therapeutics, and vaccines, are limited or unavailable. Improved approaches are needed for the treatment of RSV infections. Provided herein are compositions, methods, uses, and articles of manufacture that meet such and other needs.

### Summary of the Invention

Provided herein is a fusion protein (which may also be referred to herein as recombinant polypeptide) comprising a soluble RSV viral surface antigen linked to a collagen trimerization domain, wherein the soluble RSV viral surface antigen comprises a recombinant F peptide, or a fragment or epitope thereof. In some embodiments, RSV virus is of subtype A and/or subtype B. In some embodiments, the soluble RSV viral surface antigen is linked to the collagen trimerization domain by in-frame fusion. In some embodiments, the fusion protein is capable of forming a disulfide-linked trimeric fusion protein. In some embodiments, the fusion protein exists in a trimeric form.

In some embodiments, the collagen trimerization domain is a collagen C-terminal propeptide. In some embodiments, the collagen trimerization domain comprises a C-terminal polypeptide of proα1(I), proα1(II), proα1(III), proα1(V), proα1(XI), proα2(I), proα2(V), proα2(XI), or proα3(XI), or a fragment thereof. In some embodiments, the collagen trimerization domain comprises the sequence of any one of SEQ ID NOs: 103-118, or an amino acid sequence having at least 90% identity thereto, capable of forming inter-polypeptide disulfide bonds and trimerizing the fusion protein.

In some embodiments, the recombinant F peptide, or a fragment or epitope thereof has one or more mutation sites, and the F peptide, or a fragment or epitope thereof comprises a sulfur-containing amino acid or a sulfur-containing amino acid derivative at the one or more mutation sites. In some embodiments, the recombinant F peptide, or a fragment or epitope thereof has at least about 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 11, 16, 31, or 36.

In some embodiments, the recombinant F peptide, or a fragment or epitope thereof comprises an F2 peptide, or a fragment or epitope thereof, and an F1 peptide, or a fragment or epitope thereof. In some embodiments, the recombinant F peptide, or a fragment or epitope thereof comprises a pep27 peptide. In some embodiments, the F2 peptide, or a fragment or epitope thereof is located at the N-terminus of the F1 peptide, or a fragment or epitope thereof.

In some embodiments, the recombinant F peptide, or a fragment or epitope thereof comprises one or more mutation sites in the F2 peptide and/or pep27 peptide. In some embodiments, the recombinant F peptide, or a fragment or epitope thereof comprises an amino acid substitution at one or more or all of amino acid residues 106, 108, 133, 135, and 136. In some embodiments, the sulfur-containing amino acid is cysteine. In some embodiments, the recombinant F peptide, or a fragment or epitope thereof comprises the amino acid substitutions R106C, R108C, R133C, R135C, and R136C.

In some embodiments, the recombinant F peptide, or a fragment or epitope thereof comprises the sequence of any one of SEQ ID NOs: 11-20 and 31-40, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 11-20 and 31-40.

In some embodiments, the fusion protein comprises the sequence of any one of SEQ ID NOs: 1-10 and 21-30, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 1-10 and 21-30.

In some embodiments, the recombinant F peptide, or a fragment or epitope thereof comprises an F1 peptide and an F2 peptide of the F peptide, wherein the F1 peptide and the F2 peptide are linked by an artificially introduced linker, the artificially introduced linker being the amino acid sequence CGGG (SEQ ID NO: 138). In some embodiments, the F2 peptide, or a fragment or epitope thereof is located at the N-terminus of the F1 peptide, or a fragment or epitope thereof.

In some embodiments, the F2 peptide comprises an amino acid sequence from amino acid residues at position 26 to position 105 of the F0 precursor, or an amino acid sequence from amino acid residues at position 1 to position 105 of the F0 precursor. In some embodiments, the F1 peptide comprises an amino acid sequence from amino acid residue at position 105 to any one selected from amino acid residues at positions 513-524 of the F0 precursor.

In some embodiments, the F0 precursor comprises the sequence of SEQ ID NO: 67 or 69, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 67 or 69.

In some embodiments, the recombinant F peptide, or a fragment or epitope thereof comprises the sequence of any one of SEQ ID NOs: 49-50 and 62-63, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 49-50 and 62-63.

In some embodiments, the fusion protein comprises the sequence of any one of SEQ ID NOs: 41-48 and 54-61, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 41-48 and 54-61.

Also provided herein is a polynucleotide encoding any one of the fusion proteins described herein. Also provided herein is a vector comprising any one of the polynucleotides described herein. Also provided herein is a host cell comprising any one of the polynucleotides described herein or any one of the vectors described herein.

Also provided herein is an immunogenic composition (which may also be referred to as recombinant subunit vaccine) comprising any one of the fusion proteins described herein. In some embodiments, the immunogenic composition comprises one or more adjuvants, or does not comprise an adjuvant. In some embodiments, the adjuvant is alum. In some embodiments, the alum comprises aluminum hydroxide.

In some embodiments, the immunogenic composition is multivalent, and comprises two or more of the fusion proteins, each fusion protein being a fusion protein formed by linking a soluble RSV viral surface antigen of a different RSV virus subtype or strain to a collagen trimerization domain.

In some embodiments, the immunogenic composition comprises a fusion protein formed by linking a soluble RSV viral surface antigen of RSV subtype A to a collagen trimerization domain, and a fusion protein formed by linking a soluble RSV viral surface antigen of RSV subtype B to a collagen trimerization domain.

In some embodiments, the fusion protein formed by linking a soluble RSV viral surface antigen of RSV subtype A to a collagen trimerization domain comprises the sequence of any one of SEQ ID NOs: 1-10 and 41-48, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 1-10 and 41-48.

In some embodiments, the fusion protein formed by linking a soluble RSV viral surface antigen of RSV subtype B to a collagen trimerization domain comprises the sequence of any one of SEQ ID NOs: 21-30 and 54-61, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 21-30 and 54-61.

In some embodiments, the soluble RSV viral surface antigen of RSV subtype A comprises the sequence of any one of SEQ ID NOs: 11-20 and 49-50, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 11-20 and 49-50.

In some embodiments, the soluble RSV viral surface antigen of RSV subtype B comprises the sequence of any one of SEQ ID NOs: 31-40 and 62-63, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 11-20 and 49-50.

In some embodiments, the soluble RSV viral surface antigen of RSV subtype A comprises SEQ ID NO: 12 or 17, and the soluble RSV viral surface antigen of RSV subtype B comprises SEQ ID NO: 32 or 37.

In some embodiments, the soluble RSV viral surface antigen of RSV subtype A comprises SEQ ID NO: 49 or 50, and the soluble RSV viral surface antigen of RSV subtype B comprises SEQ ID NO: 62 or 63.

In some embodiments, the fusion protein formed by linking a soluble RSV viral surface antigen of RSV subtype A to a collagen trimerization domain comprises SEQ ID NO: 2 or 7, and the fusion protein formed by linking a soluble RSV viral surface antigen of RSV subtype B to a collagen trimerization domain comprises SEQ ID NO: 22 or 27.

In some embodiments, the fusion protein formed by linking a soluble RSV viral surface antigen of RSV subtype A to a collagen trimerization domain comprises SEQ ID NO: 41 or 45, and the fusion protein formed by linking a soluble RSV viral surface antigen of RSV subtype B to a collagen trimerization domain comprises SEQ ID NO: 54 or 58.

Provided herein is a method for preventing or treating respiratory syncytial virus (RSV) infection, comprising administering to a subject any one of the fusion proteins described herein, any one of the polynucleotides described herein, any one of the vectors described herein, any one of the host cells described herein, or any one of the immunogenic compositions described herein.

Provided herein is a method for generating an immune response against the RSV F peptide, or a fragment or epitope thereof in a subject, comprising administering to the subject any one of the fusion proteins described herein, any one of the polynucleotides described herein, any one of the vectors described herein, any one of the host cells described herein, or any one of the immunogenic compositions described herein.

Provided herein is a method for producing neutralizing antibodies against RSV in a subject, comprising administering to the subject any one of the fusion proteins described herein, any one of the polynucleotides described herein, any one of the vectors described herein, any one of the host cells described herein, or any one of the immunogenic compositions described herein.

In some embodiments, the methods comprise administration by intramuscular injection (for immunization or vaccination) or by intranasal spray (for immunization or vaccination).

In some embodiments, the agent used in the methods is administered in a single dose or in a series of doses separated by intervals of weeks or months (for immunization or vaccination).

Also provided herein is use of any one of the fusion proteins described herein, any one of the polynucleotides described herein, any one of the vectors described herein, any one of the host cells described herein, or any one of the immunogenic compositions described herein in the manufacture of a medicament used in a method for preventing or treating respiratory syncytial virus (RSV) infection, a method for generating an immune response against the RSV F peptide, or a fragment or epitope thereof in a subject, and a method for producing neutralizing antibodies against RSV in a subject.

Provided herein is a protein comprising a plurality of recombinant polypeptides, each recombinant polypeptide comprising a respiratory syncytial virus (RSV) F protein peptide, or a fragment or epitope thereof linked to a collagen C-terminal propeptide, wherein the C-terminal propeptides of the recombinant polypeptides form inter-polypeptide disulfide bonds. In some embodiments, the RSV is of subtype A or/and subtype B. In some embodiments, the epitope is a linear epitope or a conformational epitope.

In some embodiments, the RSV is soluble. The soluble RSV viral surface antigen comprises an F1 peptide, or a fragment or epitope thereof having one or more mutation sites, and the F1 peptide, or a fragment or epitope thereof comprises a sulfur-containing amino acid or a sulfur-containing amino acid derivative at the one or more mutation sites.

In some embodiments, disclosed herein is a recombinant subunit vaccine comprising an extracellular domain (e.g., without a transmembrane domain and a cytoplasmic domain) of an RSV F protein, or a fragment thereof, wherein the extracellular domain is fused in-frame to a collagen C-terminal propeptide that is capable of forming a disulfide-linked homotrimer. The resulting recombinant subunit vaccine, such as an F-trimer, can be expressed and purified from transfected cells, and is expected to be in native-like conformation in a trimeric form. This solves the misfolding problem of viral antigens often encountered when they are expressed as recombinant peptides or proteins in soluble forms without the transmembrane domain and/or cytoplasmic domain. Such misfolded viral antigens do not faithfully retain the native viral antigen conformation, and often fail to elicit neutralizing antibodies.

In some embodiments, the F protein peptide comprises an F1 subunit peptide, an F2 subunit peptide, or any combination thereof, and the protein comprises three recombinant polypeptides. In some embodiments, the F protein peptide comprises a signal peptide, a heptad-repeat C (HRC) peptide, a pep27 peptide, a fusion peptide (FP), a heptad-repeat A (HRA) peptide, a Domain I peptide, a Domain II peptide, or a heptad-repeat B (HRB) peptide, or any combination thereof. In some embodiments, the F protein peptide comprises an F1 subunit but not an F2 subunit of the F protein, or vice versa. In some embodiments, the F protein peptide comprises an F1 subunit and an F2 subunit of the F protein, optionally without pep27, and optionally wherein the F1 subunit and the F2 subunit are linked by a disulfide bond or an artificially introduced linker. In some embodiments, pep27 is absent, and wherein the F1 subunit and the F2 subunit are linked by the artificially introduced linker.

In some embodiments, the F protein peptide does not comprise a transmembrane (TM) domain peptide and/or a cytoplasmic (CP) domain peptide. In some embodiments, the F protein peptide comprises a protease cleavage site, wherein the protease is optionally furin, trypsin, factor Xa, thrombin, or cathepsin L. In some embodiments, the F protein peptide does not comprise a protease cleavage site, wherein the protease is optionally furin, trypsin, factor Xa, thrombin, or cathepsin L.

In some embodiments, the F protein peptide is soluble, or does not bind directly to a lipid bilayer, e.g., a membrane or viral envelope. In some embodiments, the F protein peptides are the same or different among the recombinant polypeptides of the protein. In some embodiments, the F protein peptide is fused directly to the C-terminal propeptide, or is linked to the C-terminal propeptide via a linker, such as a linker comprising glycine-X-Y repeats, wherein X and Y are independently any amino acid, optionally proline or hydroxyproline.

In some embodiments, the protein is soluble, or does not bind directly to a lipid bilayer, e.g., a membrane or viral envelope. In some embodiments, the protein is capable of forming a rosette-like oligomer comprising an F protein peptide trimer. In some embodiments, the protein is capable of binding to a cell surface attachment factor or receptor of a subject, optionally wherein the subject is a mammal, such as a primate, e.g., human.

In some embodiments, the C-terminal propeptide is of human collagen. In some embodiments, the C-terminal propeptide comprises a C-terminal polypeptide of proα1(I), proα1(II), proα1(III), proα1(V), proα1(XI), proα2(I), proα2(V), proα2(XI), or proα3(XI), or a fragment thereof. In some embodiments, the C-terminal propeptides are the same or different among the recombinant polypeptides. In some embodiments, the C-terminal propeptide comprises any one of SEQ ID NOs: 103-118, or an amino acid sequence having at least 90% identity thereto, capable of forming inter-polypeptide disulfide bonds and trimerizing the recombinant polypeptides.

In some embodiments, the F protein peptide in each recombinant polypeptide is in a pre-fusion conformation or a post-fusion conformation, optionally wherein the protein comprises a rosette-like oligomer comprising an F protein peptide trimer as a crutch-shaped rod. In any one of the preceding embodiments, the F protein peptide in each recombinant polypeptide can comprise any one of SEQ ID NOs: 11-20, 31-40, 49-50, 62-63, and 67-70, or an amino acid sequence having at least 80% identity thereto.

In any one of the preceding embodiments, the recombinant polypeptide can comprise any one of SEQ ID NOs: 1-10, 21-30, 41-48, and 54-61, or an amino acid sequence having at least 80% identity thereto. In any one of the preceding embodiments, the recombinant polypeptide can comprise any one of SEQ ID NOs: 11-20, 31-40, 49-53, and 62-66, or an amino acid sequence having at least 80% identity thereto, which is linked directly or indirectly to any one of SEQ ID NOs: 103-118, or an amino acid sequence having at least 90% identity thereto.

Also provided herein is an immunogen comprising the protein provided herein. Provided herein is a protein nanoparticle comprising the protein provided herein linked directly or indirectly to a nanoparticle. Provided herein is a virus-like particle (VLP) comprising the protein provided herein.

Also provided herein is an isolated nucleic acid encoding one, two, three, or more recombinant polypeptides of the protein provided herein. In some embodiments, a polypeptide encoding the F protein peptide is fused in-frame to a polypeptide encoding the collagen C-terminal propeptide. In some embodiments, the isolated nucleic acid provided herein is operably linked to a promoter.

In some embodiments, the isolated nucleic acid provided herein is a DNA molecule. In some embodiments, the isolated nucleic acid provided herein is an RNA molecule, optionally an mRNA molecule, such as a nucleoside-modified mRNA, a non-amplifying mRNA, a self-amplifying mRNA, or a trans-amplifying mRNA.

Also provided herein is a vector comprising the isolated nucleic acid provided herein. In some embodiments, the vector is a viral vector.

In some aspects, provided herein is a virus, pseudovirus, or cell comprising the vector provided herein, optionally wherein the virus or cell has a recombinant genome. In some aspects, provided herein is an immunogenic composition comprising the protein, immunogen, protein nanoparticle, VLP, isolated nucleic acid, vector, virus, pseudovirus, or cell provided herein, and a pharmaceutically acceptable carrier.

Also provided herein is a vaccine comprising the immunogenic composition provided herein and optionally an adjuvant, wherein the vaccine is optionally a subunit vaccine. In some embodiments, the vaccine is a prophylactic and/or therapeutic vaccine.

In some aspects, provided herein is a method for producing a protein, comprising: expressing the isolated nucleic acid or vector provided herein in a host cell to produce the protein provided herein; and purifying the protein. Provided herein is a protein produced by the method provided herein.

Provided herein is a method for generating an immune response against an RSV F protein peptide, or a fragment or epitope thereof in a subject, comprising administering to the subject an effective amount of the protein, immunogen, protein nanoparticle, VLP, isolated nucleic acid, vector, virus, pseudovirus, cell, immunogenic composition, or vaccine provided herein to generate the immune response. In some embodiments, the method provided herein is used for treating or preventing RSV infection. In some embodiments, the immune response generated inhibits or reduces the replication of RSV in the subject. In some embodiments, the immune response comprises a cell-mediated response and/or a humoral response, and optionally comprises the production of one or more neutralizing antibodies, such as polyclonal antibodies or monoclonal antibodies. In some embodiments, the immune response is directed against the RSV F protein peptide, or a fragment or epitope thereof, but not against the C-terminal propeptide. In some embodiments, administration to the subject does not result in antibody-dependent enhancement

(ADE) in the subject due to prior exposure to one or more strains of RSV. In some embodiments, the administration does not result in antibody-dependent enhancement (ADE) in the subject when subsequently exposed to one or more strains of RSV. In some embodiments, the method further comprises a priming step and/or a boosting step. In some embodiments, the administering step is performed via topical, transdermal, subcutaneous, intradermal, oral, intranasal (e.g., intranasal spray), intratracheal, sublingual, buccal, rectal, vaginal, inhaled, intravenous (e.g., intravenous injection), intraarterial, intramuscular (e.g., intramuscular injection), intracardiac, intraosseous, intraperitoneal, transmucosal, intravitreal, subretinal, intraarticular, periarticular, local, or transepidermal administration. In some embodiments, the effective amount is administered in a single dose or in a series of doses separated by one or more intervals. In some embodiments, the effective amount is administered without an adjuvant. In some embodiments, the effective amount is administered with an adjuvant.

Provided herein is a method comprising administering to a subject an effective amount of the protein provided herein to produce in the subject neutralizing antibodies or neutralizing antisera against RSV. In some embodiments, the subject is a mammal, optionally a human or a non-human primate. In some embodiments, the method further comprises isolating the neutralizing antibodies or neutralizing antisera from the subject. In some embodiments, the method further comprises administering to a human subject an effective amount of the isolated neutralizing antibodies or neutralizing antisera via passive immunization to prevent or treat RSV infection. In some embodiments, the neutralizing antibodies or neutralizing antisera against RSV comprise polyclonal antibodies against the RSV F protein peptide, or a fragment or epitope thereof, optionally wherein the neutralizing antibodies or neutralizing antisera are free or substantially free of antibodies against the collagen C-terminal propeptide. In some embodiments, the neutralizing antibodies comprise monoclonal antibodies against the RSV F protein peptide, or a fragment or epitope thereof, optionally wherein the neutralizing antibodies are free or substantially free of antibodies against the collagen C-terminal propeptide.

In some aspects, the protein, immunogen, protein nanoparticle, VLP, isolated nucleic acid, vector, virus, pseudovirus, cell, immunogenic composition, or vaccine provided herein is used for inducing an immune response against RSV in a subject, and/or for treating or preventing RSV infection.

In some aspects, provided herein is use of the protein, immunogen, protein nanoparticle, VLP, isolated nucleic acid, vector, virus, pseudovirus, cell, immunogenic composition, or vaccine provided herein for inducing an immune response against RSV in a subject, and/or for treating or preventing RSV infection. In some aspects, provided herein is use of the protein, immunogen, protein nanoparticle, VLP, isolated nucleic acid, vector, virus, pseudovirus, cell, immunogenic composition, or vaccine provided herein in the manufacture of a medicament or a prophylactic agent for inducing an immune response against RSV virus in a subject, and/or for treating or preventing RSV infection.

Also provided herein is a method for analyzing a sample, comprising: contacting the sample with the protein provided herein, and detecting binding between the protein and an analyte capable of specific binding to the RSV F protein peptide, or a fragment or epitope thereof. In some embodiments, the analyte is an antibody, receptor, or cell recognizing the F protein peptide, or a fragment or epitope thereof. In some embodiments, the binding indicates the presence of the analyte in the sample, and/or the presence of RSV infection in the subject from which the sample is derived.

Provided herein is a kit comprising the protein provided herein and a substrate, pad, or vial containing or immobilizing the protein, optionally wherein the kit is a kit for ELISA or lateral flow assay.

### Brief Description of the Drawings

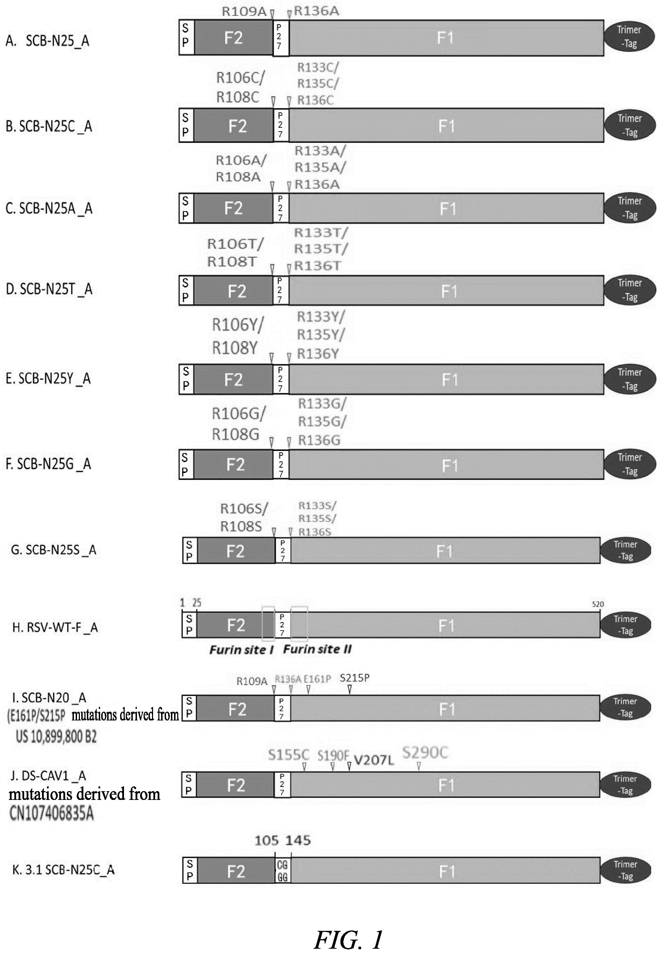

Figure 1 shows a schematic diagram of exemplary fusion peptides comprising the extracellular F domain of the A2 strain fused to a trimerization peptide.

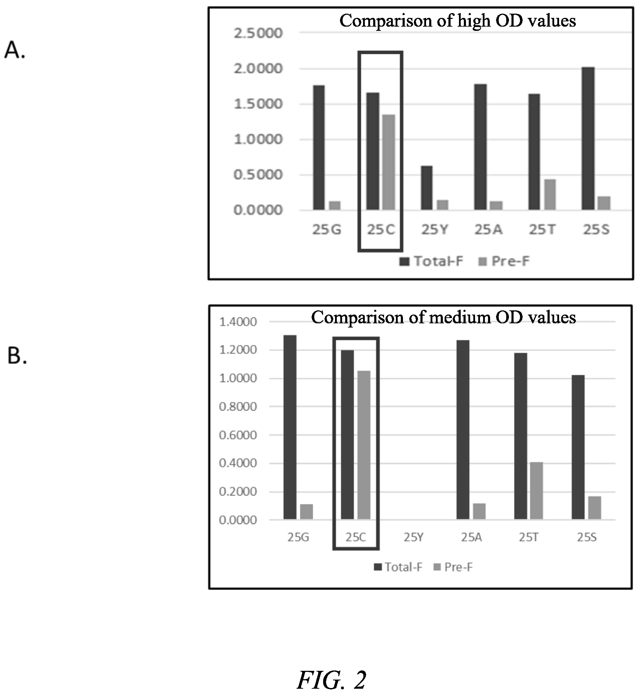

Figure 2 shows the OD values in the supernatant of 293T cells on Day 3. On Day 3, the amounts of total protein and pre-fusion F protein in the supernatant were determined by ELISA.

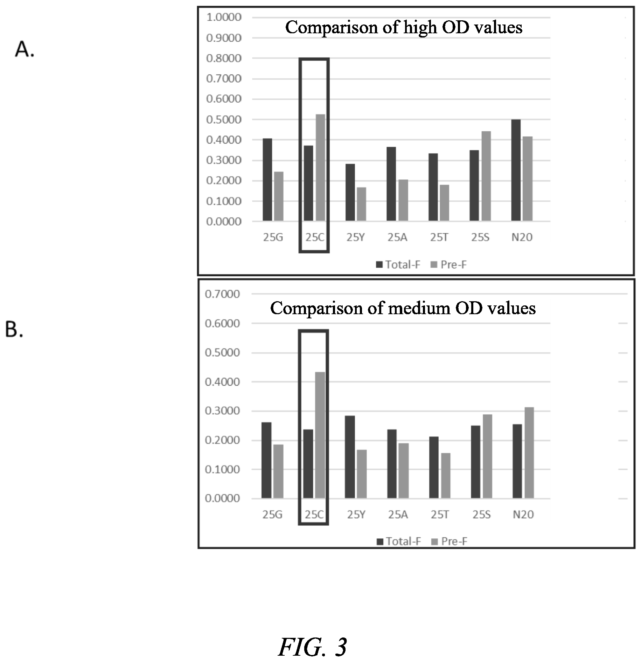

Figure 3 shows the OD values in the supernatant of CHO cells on Day 3. On Day 3, the amounts of total protein and pre-fusion F protein in the supernatant were determined by ELISA.

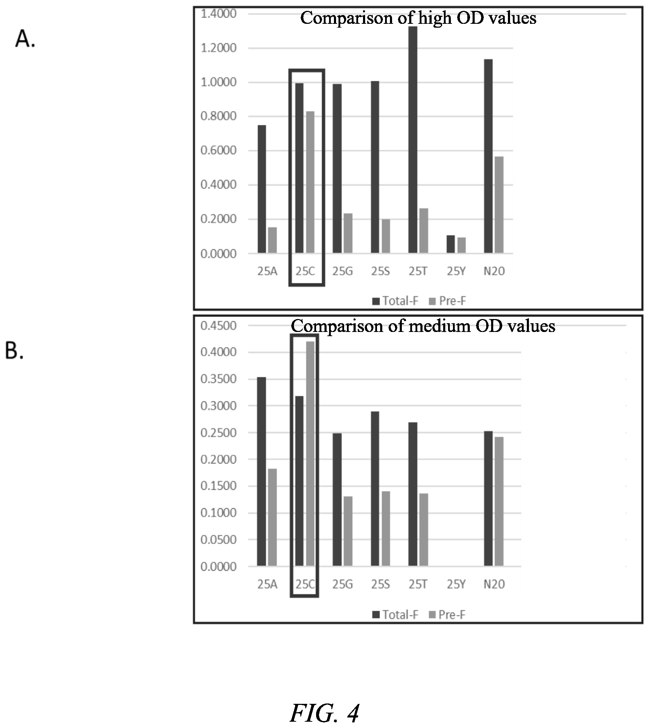

Figure 4 shows the OD values in the supernatant of CHO cells on Day 7. On Day 7, the amounts of total protein and pre-fusion F protein in the supernatant were determined by ELISA.

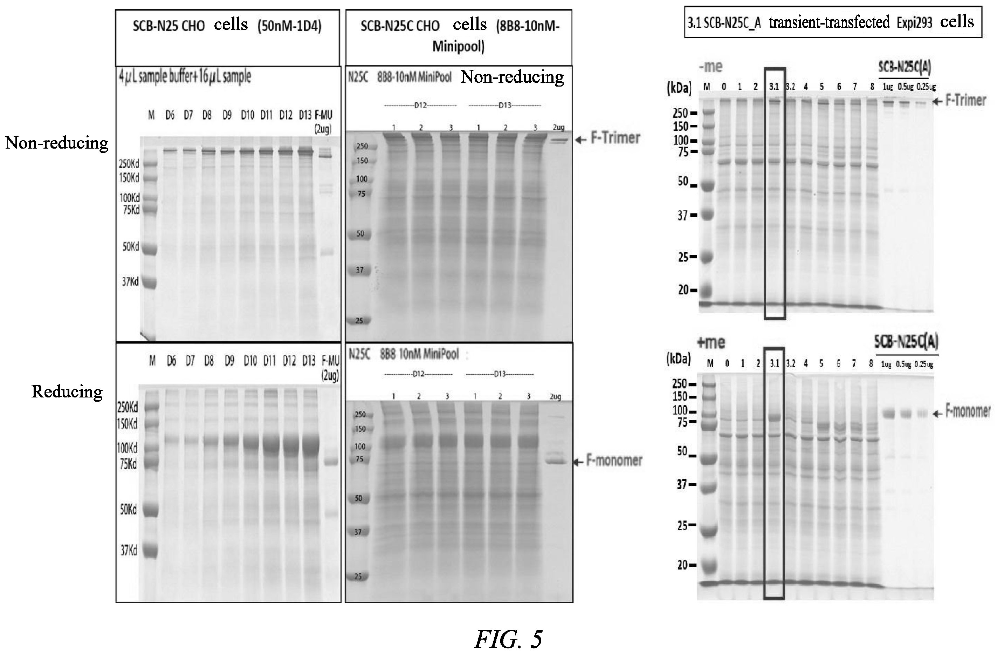

Figure 5 shows the expression levels of the peptides expressed from exemplary fusion peptides as analyzed by 8% SDS-PAGE in serum-free fed-batch cell cultures. SCB-N25 (i.e., SCB-N25_A): cell-free conditioned media from Day 1 to Day 13 were separated under non-reducing and reducing conditions, followed by Coomassie brilliant blue staining, with a loading volume of 16 µl; SCB-N25C (i.e., SCB-N25C_A): cell-free conditioned media from Day 1 to Day 13 were separated under non-reducing and reducing conditions, followed by Coomassie brilliant blue staining, with a loading volume of 16 µl; and 3.1 SCB-N25_A: transient transfection conditioned media were subjected to SDS-PAGE under non-reducing conditions, followed by Coomassie brilliant blue staining, with a loading volume of 13.5 µl.

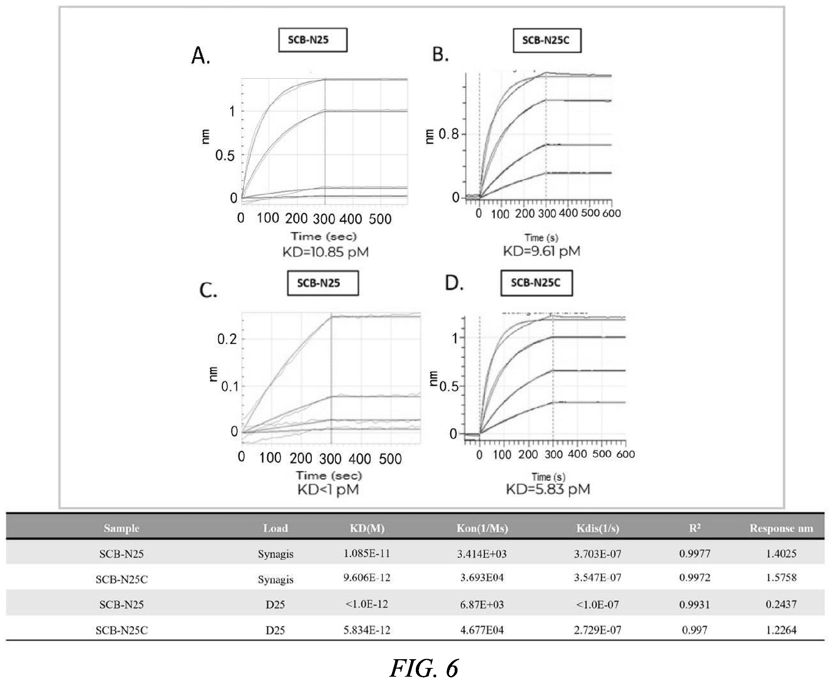

Figure 6 shows the purity evaluation of exemplary purified fusion peptides by SEC-HPLC. The main peak area of the exemplary SCB-N25 (i.e., SCB-N25_A) fusion protein was 92.8%, the main peak area of the SCB-N25C (i.e., SCB-N25C_A) protein was 81.7%, and the main peak area of the 3.1 SCB-N25C_A protein was 88.58%.

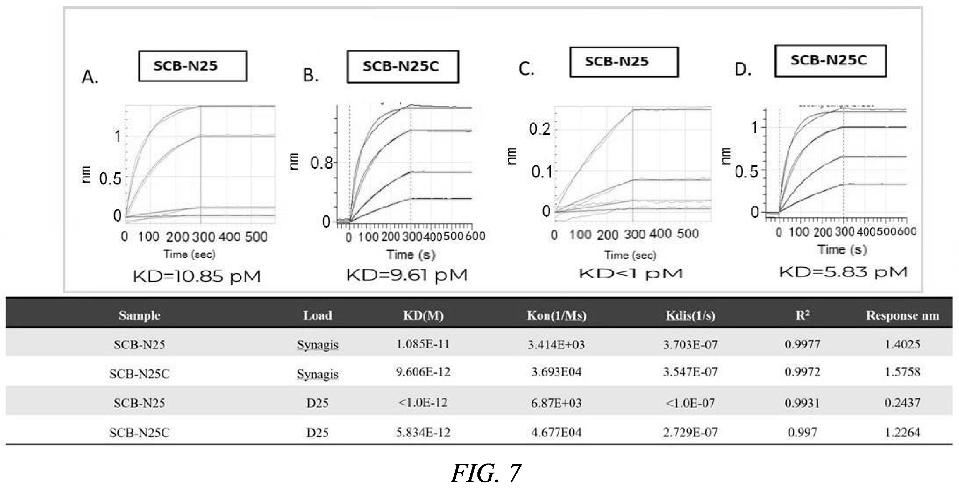

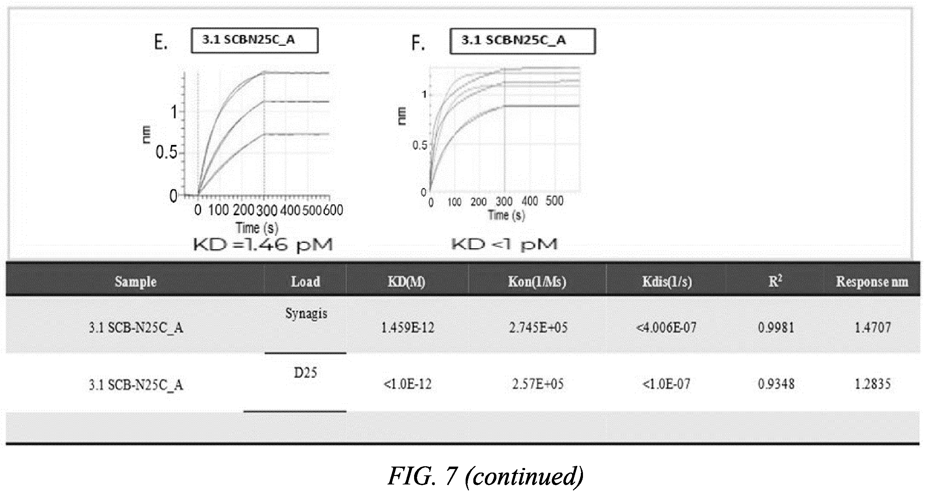

Figure 7 shows affinity kinetic assays. Panels A, B, and E show binding studies by biolayer interferometry between palivizumab and exemplary fusion peptides comprising the RSV F protein peptide. First, 5 µg/mL palivizumab was immobilized on Protein A sensors, and the sensors were then immersed in the exemplary fusion peptides at different concentrations to measure binding kinetics. The resulting curves were fitted to a 1:1 binding model by subtracting buffer reference values to obtain the Kₒₙ and K_{dis} values, as shown in the table below. Panels C, D, and F show binding studies by binding between antibody D25_A and exemplary fusion peptides comprising the RSV F protein peptide. The corresponding K_{D}, Kₒₙ, and K_{dis} values from the antibody D25 binding assays are shown in the labels below. SCB-N25C (i.e., SCB-N25C_A) exhibited high affinity for both palivizumab and antibody D25 at 9.61 pM and 5.83 pM, respectively.

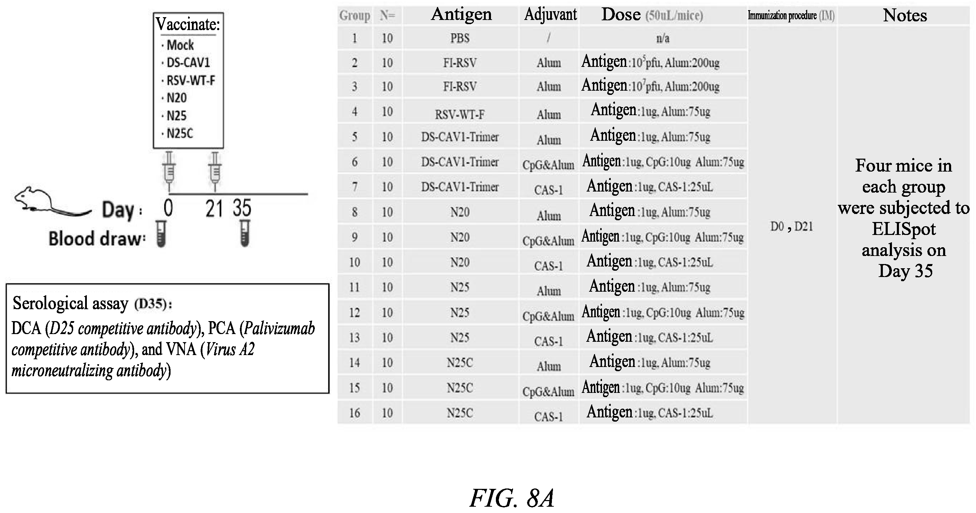

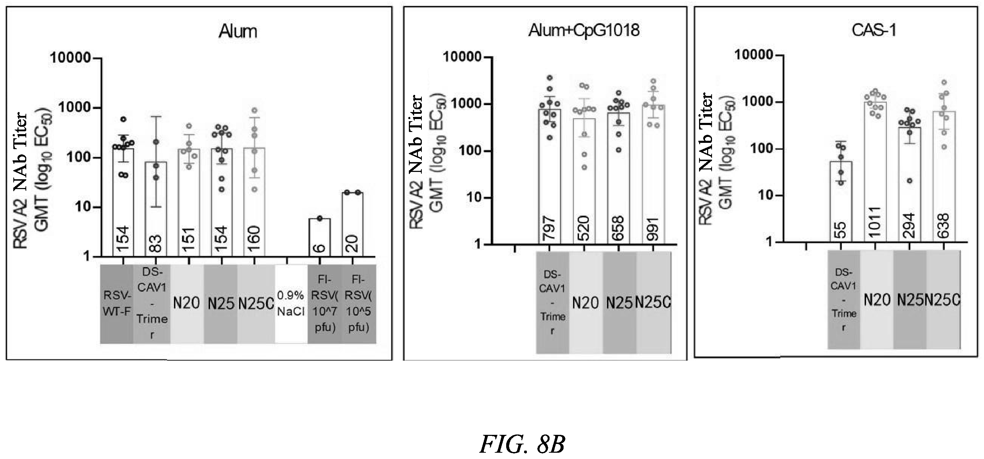

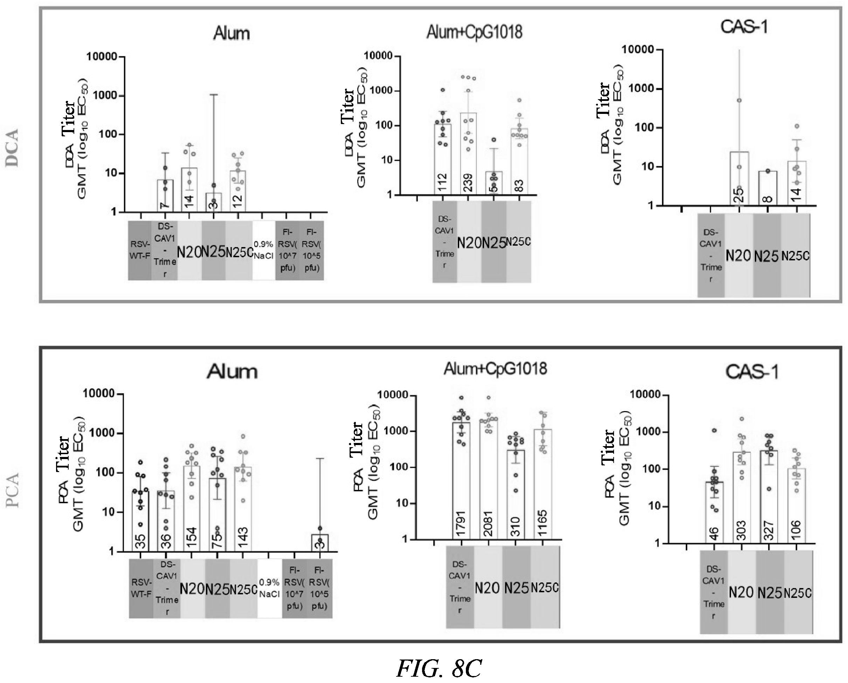

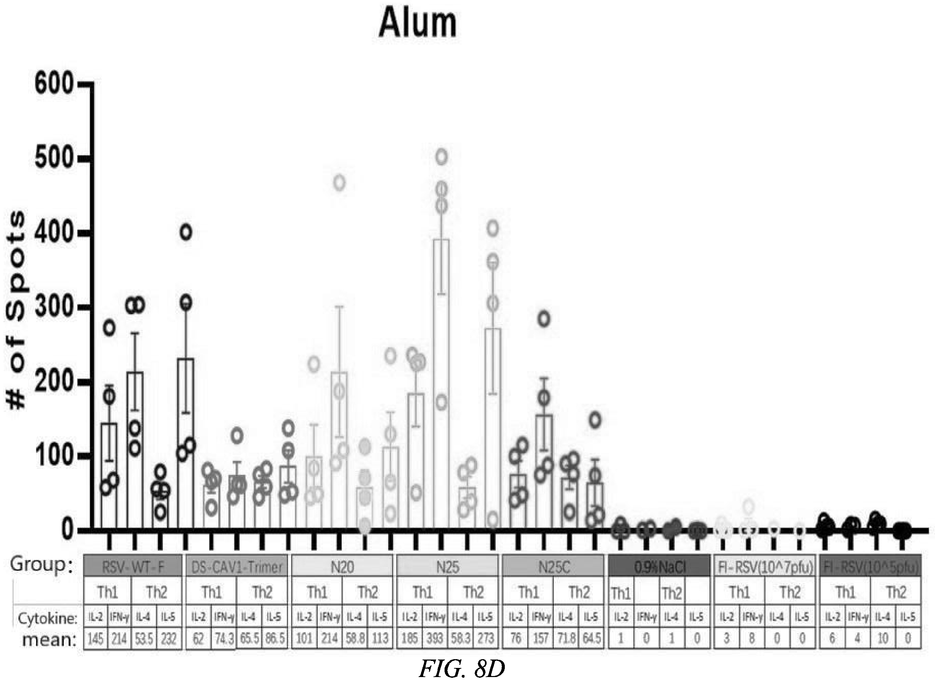

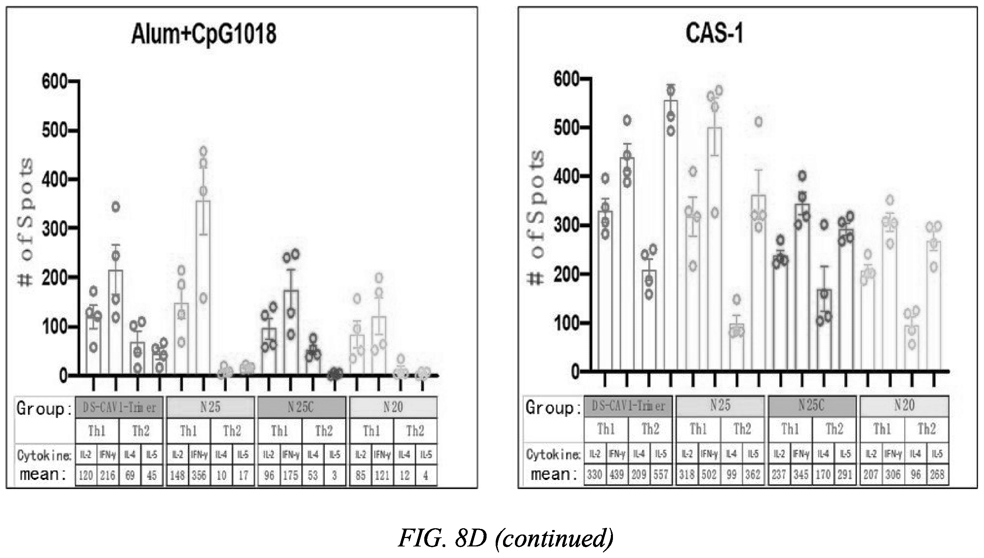

Figures 8A-8D show the results of immunization assays using exemplary fusion peptides comprising the RSV F protein peptide. Figure 8A shows a schematic diagram of the experimental method: mice were immunized on Day 0 and Day 21, and sera were collected on Day 0 and Day 35, respectively. Figure 8B shows serological assays (D35) for RSV A2 strain microneutralizing antibody titers against purified exemplary fusion peptides comprising the RSV F protein peptide and the adjuvant Alum, Alum + CpG 1018, or CAS-1. The fusion proteins adjuvanted with Alum + CpG 1018 showed higher titer values than the corresponding fusion proteins adjuvanted with Alum or CAS-1. SCB-N25C (i.e., SCB-N25C_A) showed higher titer values than other antigens. Figure 8C shows the titers of D25 and palivizumab competitive IgG that provide 50% inhibition of the binding of D25 and palivizumab to heat-inactivated RSV (HI-RSV) particles, as determined by serum sample dilutions. Values are expressed as log₂ and mean ± SEM. All antigens adjuvanted with Alum showed lower antibody (DCA: D25 competitive antibody; and PCA: palivizumab competitive antibody) responses than those with the other two adjuvants. When used in combination with the Alum + CpG1018 adjuvant, both DS-CAV1-Trimer and N20 (i.e., SCB-N20_A) showed high DCA and PCA titers, with no significant difference between them. N25 (i.e., SCB-N25_A) performed the worst. None of the four antigen candidates used in combination with the CAS-1 adjuvant had effective DCA titers. N20 and N25 had high PCA titers and the DS-CAV1-Trimer had the lowest titers. Figure 8D shows the results of the ELISpot assay. The N25C (i.e., SCB-N25C_A) antigen in combination with the Alum adjuvant generated a strong Th1-type cellular immune response. All antigens in combination with the Alum + CpG1018 adjuvant generated a low Th2-type cellular immune response. All antigens in combination with the CAS-1 adjuvant generated high Th1-type and Th2-type cellular immune responses. CAS-1 comprises squalene, α-tocopherol, and Tween-80.

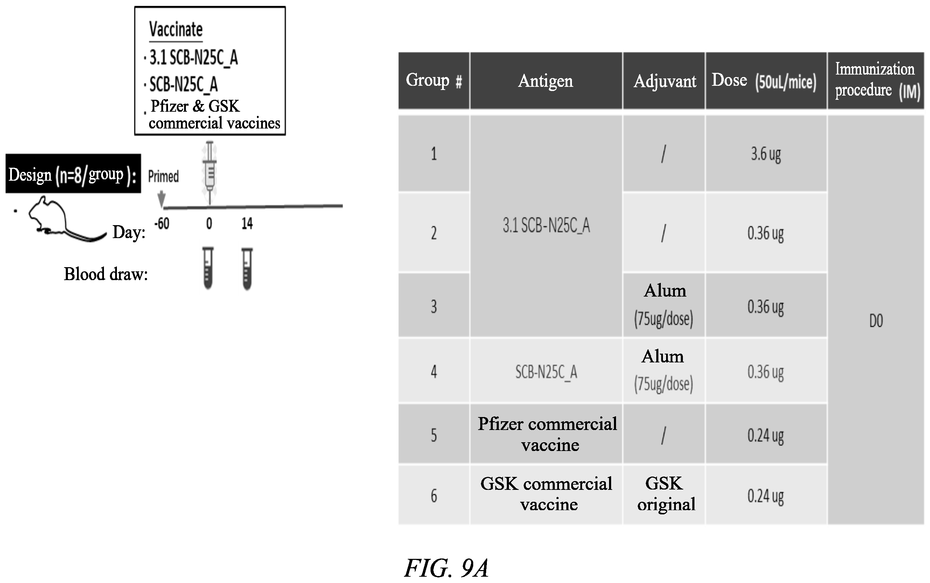

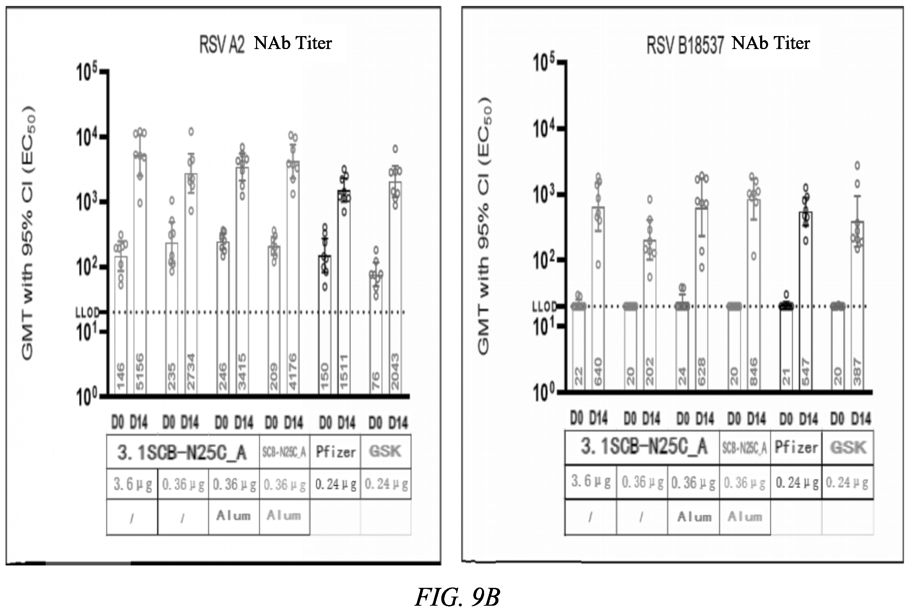

Figures 9A and 9B show the immunogenicity assays of the vaccine candidate based on 3.1 SCB-N25C_A in RSV-infected mice. Figure 9A shows a schematic diagram of the experimental method: 6- to 8-week-old SPF female BALB/c mice were intranasally infected with RSV virus to develop natural immunity; after 60 days, the mice were randomly divided into 6 groups of 8 mice each, and the mice in each group were immunized once intramuscularly with 50 µL of the RSV virus vaccine candidate or control vaccine according to the table below; and blood samples were collected by retroorbital venipuncture on Day 0 and Day 14, and sera were obtained by centrifugation. Figure 9B shows the experimental results of the immunogenicity assays.

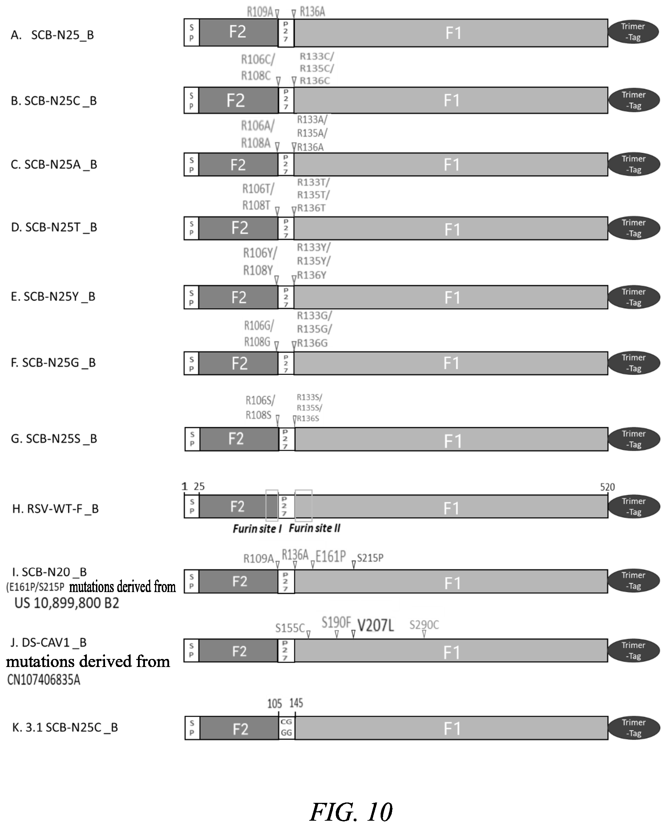

Figure 10 shows a schematic diagram of exemplary fusion peptides comprising the extracellular F domain of the B strain fused to a trimerization peptide.

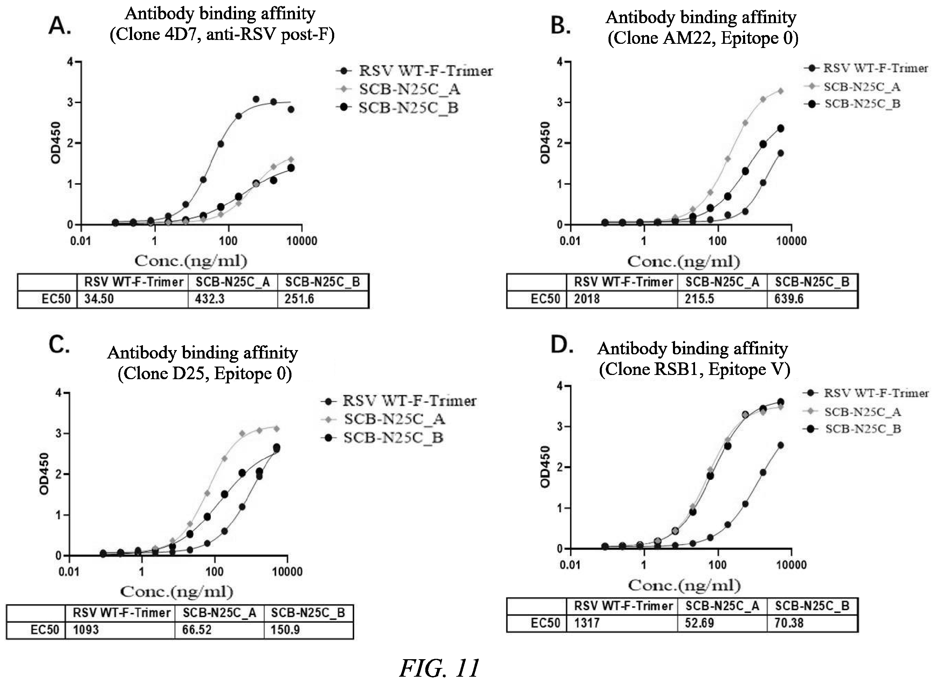

Figure 11 shows the ELISA-based affinity assays of multiple antibodies against RSV vaccine candidate antigens. A: anti-RSV post-F (4D7) antibody; B: human anti-RSV F (AM22) antibody; C: D25 antibody; and D: anti-RSV F (RSB1) antibody.

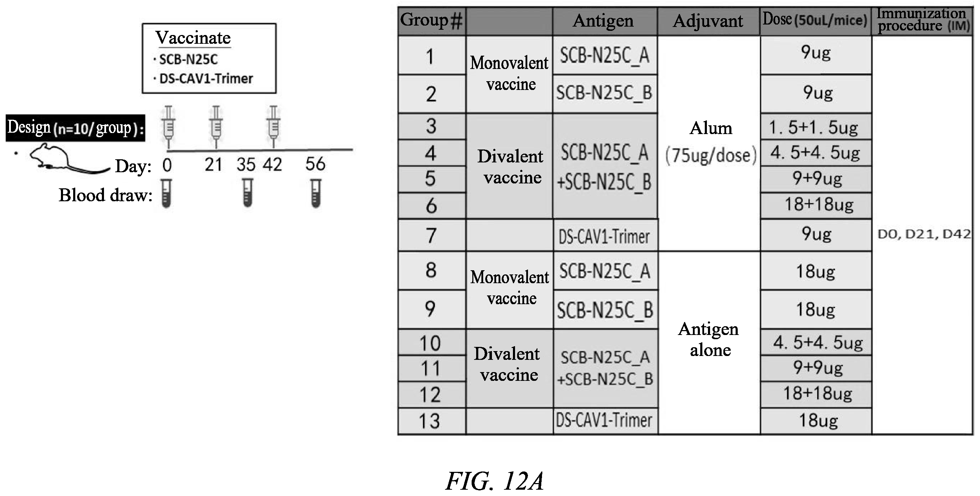

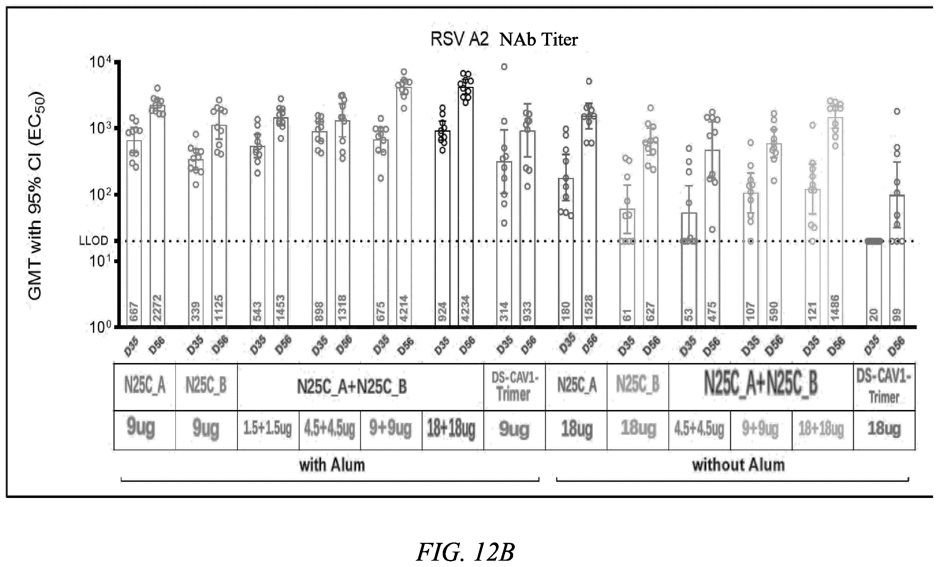

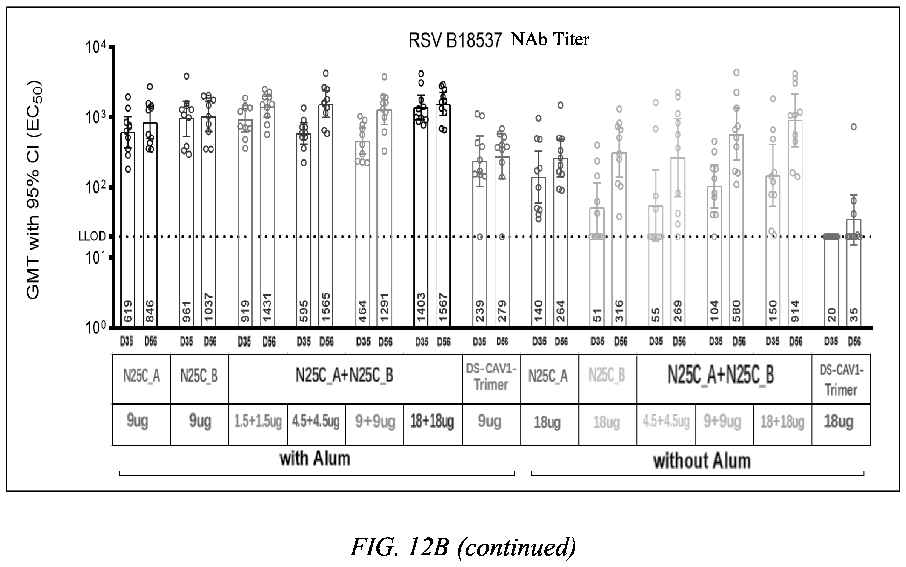

Figure 12 shows the titer detection of neutralizing antibodies against the RSV A2 and B18537 strains. A: Titer detection results of neutralizing antibodies against live hRSV A2 virus; and B: titer detection results of neutralizing antibodies against live hRSV B18537 virus.

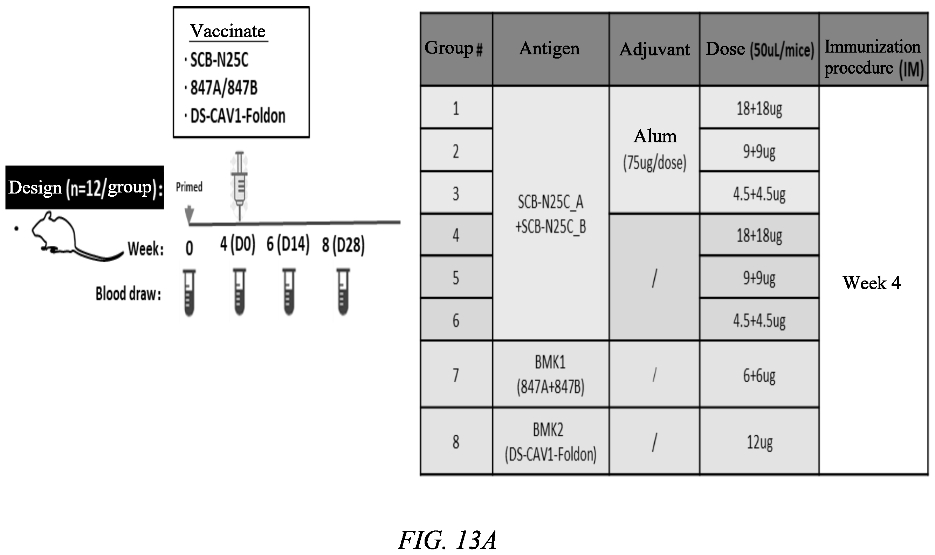

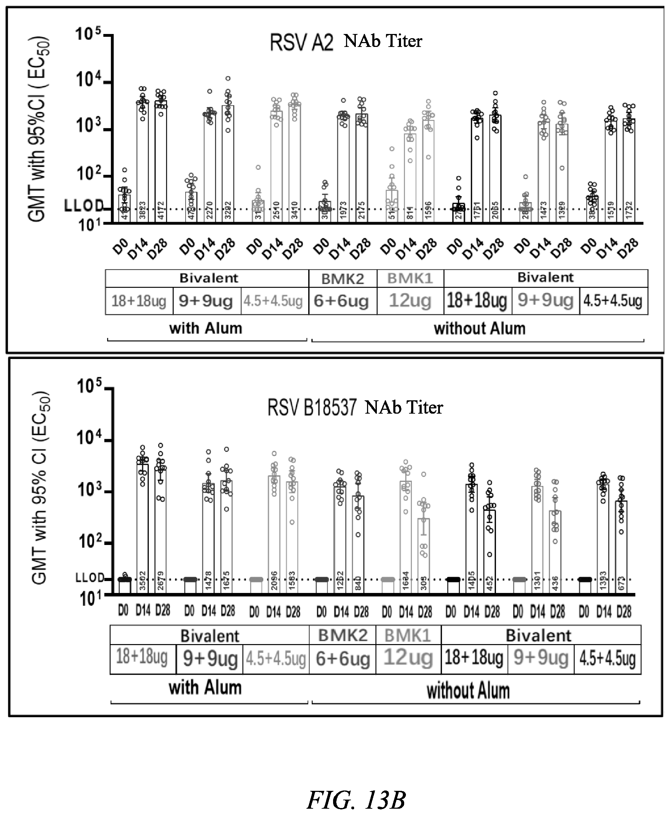

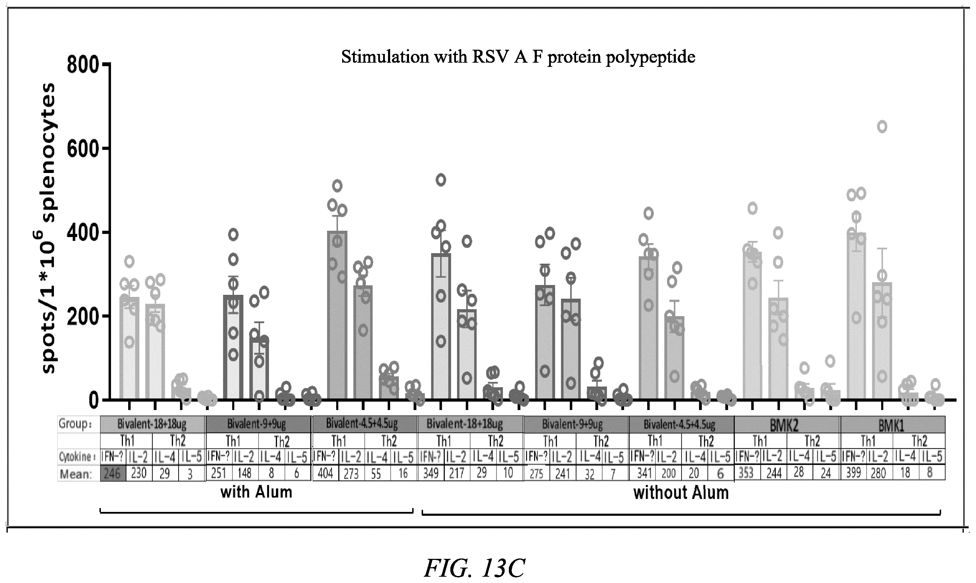

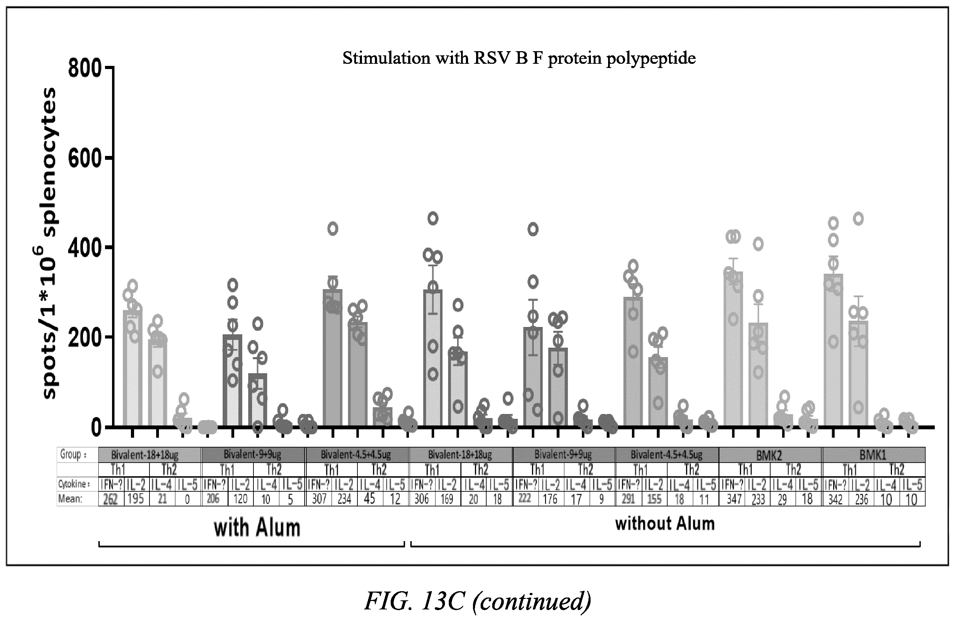

Figures 13A-13C show the immunogenicity assays of an exemplary trimerized fusion peptide bivalent vaccine in primed mice. Figure 13A shows a schematic diagram of the experimental method: 6- to 8-week-old SPF female BALB/c mice were intranasally infected with RSV virus to develop natural immunity; after 28 days, the mice were randomly divided into 8 groups of 12 mice each, and the mice in each group were immunized once intramuscularly with 50 µl of the RSV virus vaccine candidate or control vaccine according to the table below; and blood samples were collected by retroorbital venipuncture on Day 0, Day 14, and Day 28, and sera were obtained by centrifugation. The bivalent vaccine was SCB-N25C_A + SCB-N25C_B at a weight ratio of 1 : 1, with 75 µg of Alum per dose. Figure 13B shows the titer values of neutralizing antibodies induced by the bivalent trimerized fusion peptide vaccines against live hRSV A2 and hRSV B18537 viruses. Figure 13C shows the data obtained after stimulation with the peptide pools of F protein from RSV subtype A and subtype B.

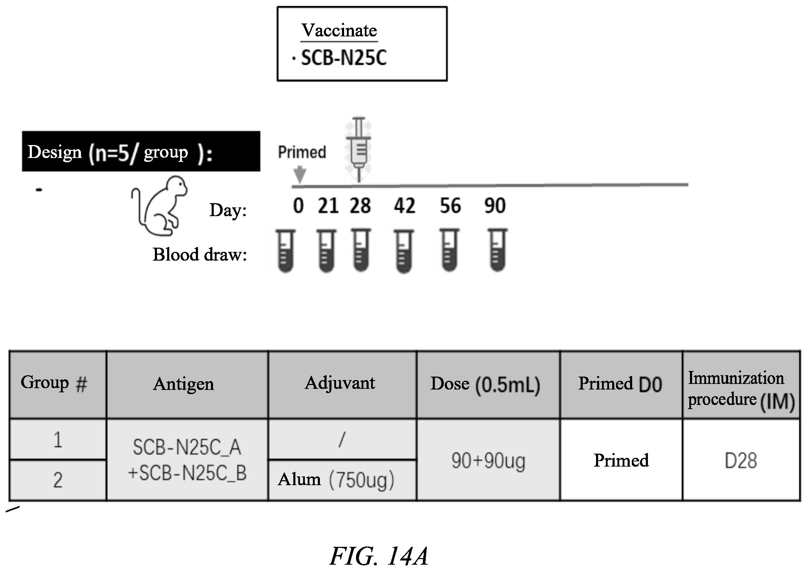

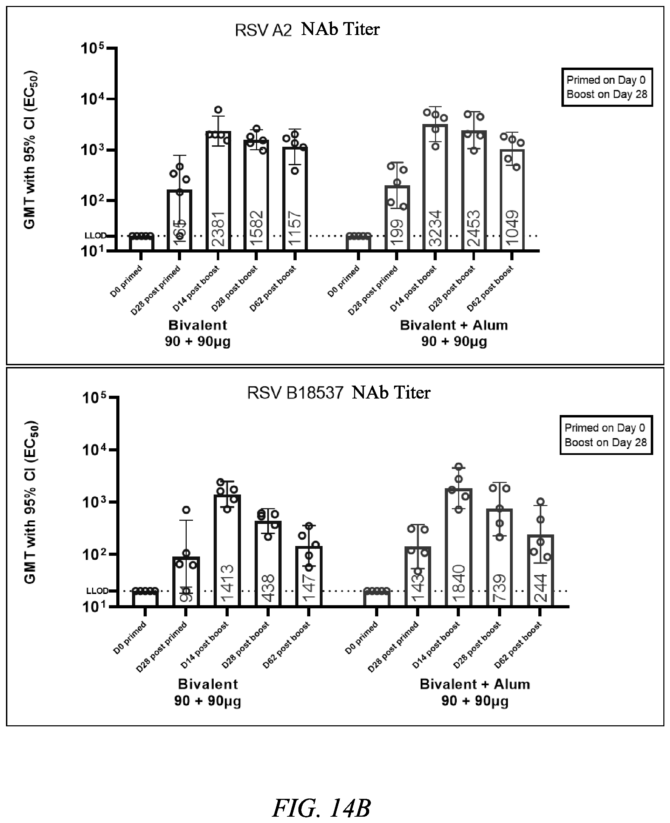

Figures 14A-14B show the immunogenicity assays of an exemplary trimerized fusion peptide bivalent vaccine in non-human primates. Figure 14A shows a schematic diagram of the experimental method: 10 male rhesus monkeys were immunized intramuscularly with a mixture of SCB-N25C_A and SCB-N25C_B with the squalene emulsion adjuvant to develop an immune response; after 28 days, the rhesus monkeys were randomly divided into 2 groups of 5 monkeys each, and the test monkeys in each group were immunized once intramuscularly with 500 µL of the RSV virus vaccine candidate according to the table below; and blood samples were collected on Day 0, Day 14, Day 28, and Day 62 post-immunization, and sera were obtained by centrifugation. The bivalent vaccine was SCB-N25C_A + SCB-N25C_B at a weight ratio of 1 : 1, with a dose of 90 µg per antigen and 750 µg of Alum per dose. Figure 14B shows the titer values of neutralizing antibodies induced by the bivalent trimerized fusion peptide vaccine against live hRSV A2 and hRSV B18537 viruses.

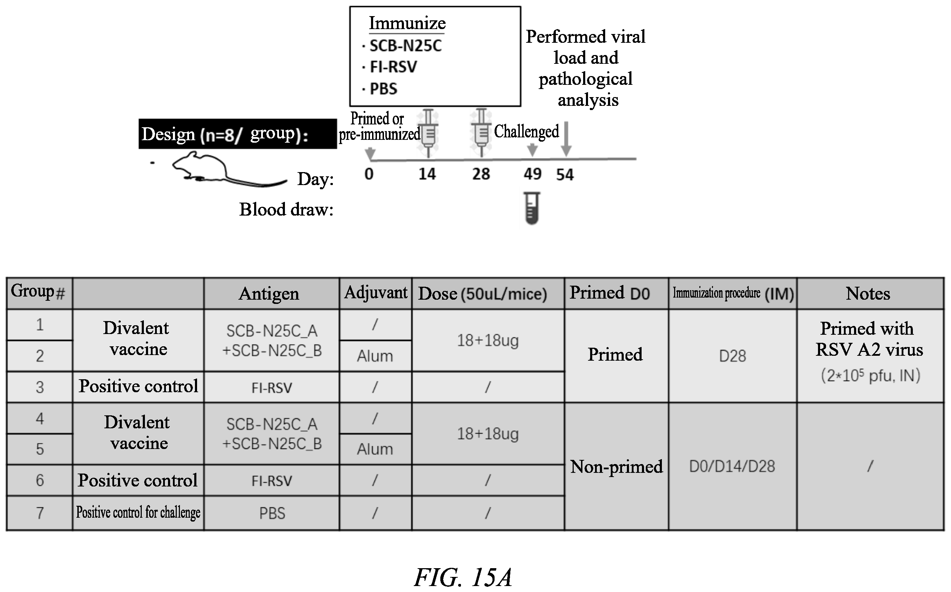

Figures 15A-15B show mouse challenge assays. Figure 15A shows a schematic diagram of the experimental method: a total of 56 mice were used, and 24 6- to 8-week-old SPF female BALB/c mice were intranasally infected with RSV virus to develop natural immunity; the mice were randomly divided into 7 groups (3 groups of primed mice and 4 groups of naive mice, with 8 mice per group), and the mice in each group were immunized intramuscularly with 50 µL of the RSV virus vaccine candidate or control vaccine according to the table below; and blood samples were collected by retroorbital venipuncture on Day 49 and sera were obtained by centrifugation, and mouse viral load and lung pathological sections were detected on Day 54. The bivalent vaccine was SCB-N25C_A + SCB-N25C_B at a weight ratio of 1 : 1, with a dose of 18 µg per antigen and 75 µg of Alum per dose. Figure 13B shows the titer values of neutralizing antibodies induced by the bivalent trimerized fusion peptide vaccines against live hRSV A2 and hRSV B18537 viruses.

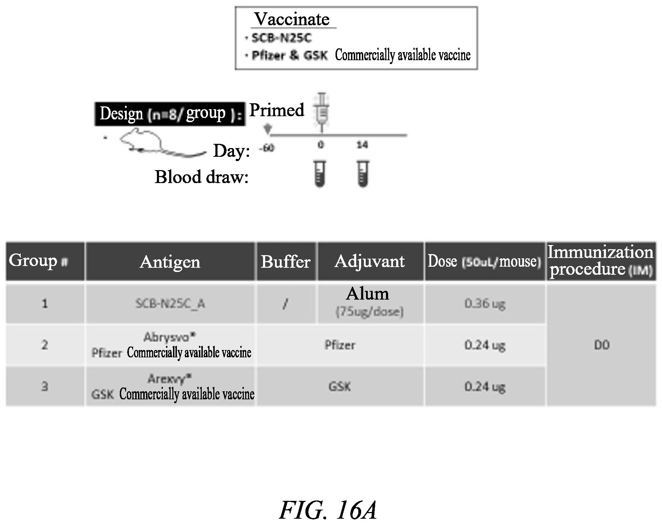

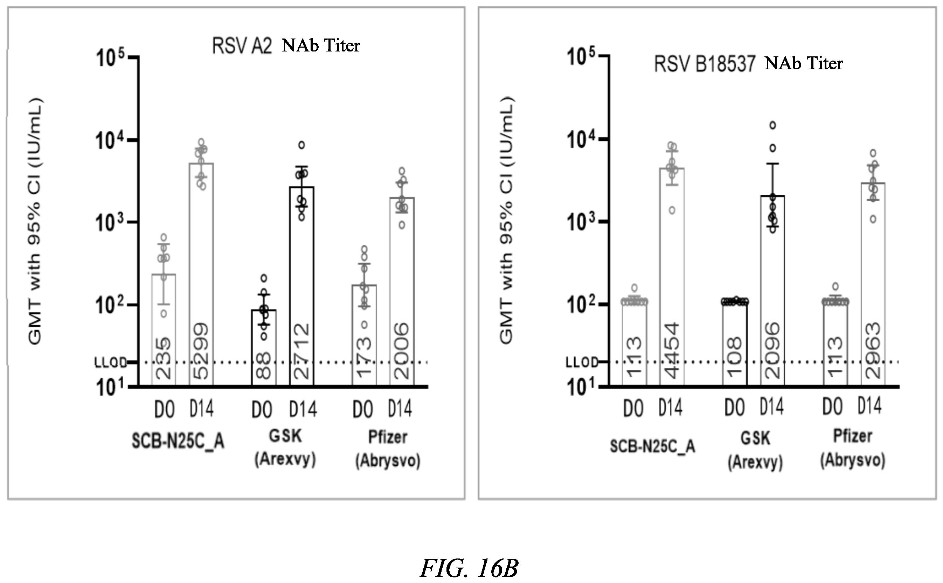

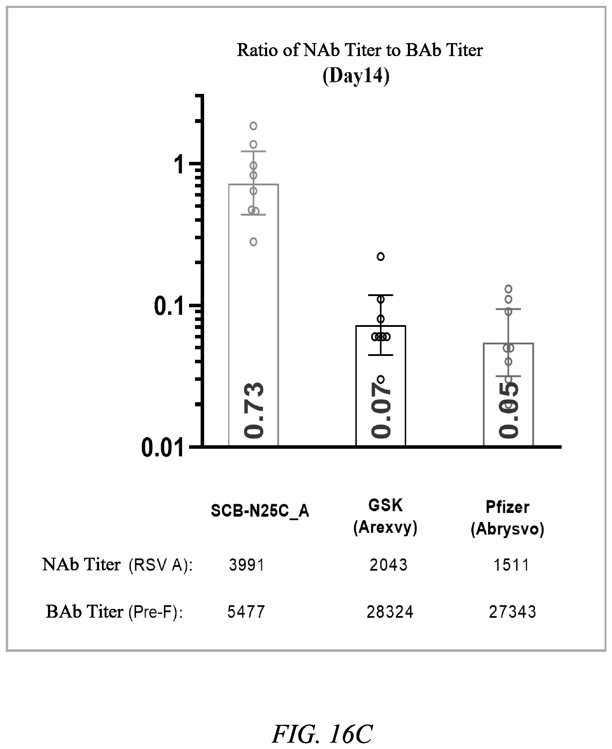

Figures 16A-16C show head-to-head immunogenicity comparison assays of an exemplary trimerized fusion peptide vaccine versus commercially available RSV vaccines. Figure 16A shows a schematic diagram of the experimental method: 6- to 8-week-old SPF female BALB/c mice were intranasally infected with RSV virus to develop natural immunity; after 60 days, the mice were randomly divided into 3 groups of 8 mice each, and the mice in each group were immunized once intramuscularly with 50 µL of the RSV virus vaccine candidate or control vaccine according to the table below; and blood samples were collected by retroorbital venipuncture on Day 0, Day 14, and Day 28, and sera were obtained by centrifugation. Figure 16B shows the head-to-head comparison of the titers of neutralizing antibodies against the trimerized fusion peptide vaccine versus commercially available RSV vaccines. Figure 16C shows the head-to-head comparison of the ratio of neutralizing antibody titer to binding antibody titer against the trimerized fusion peptide vaccine versus commercially available RSV vaccines.

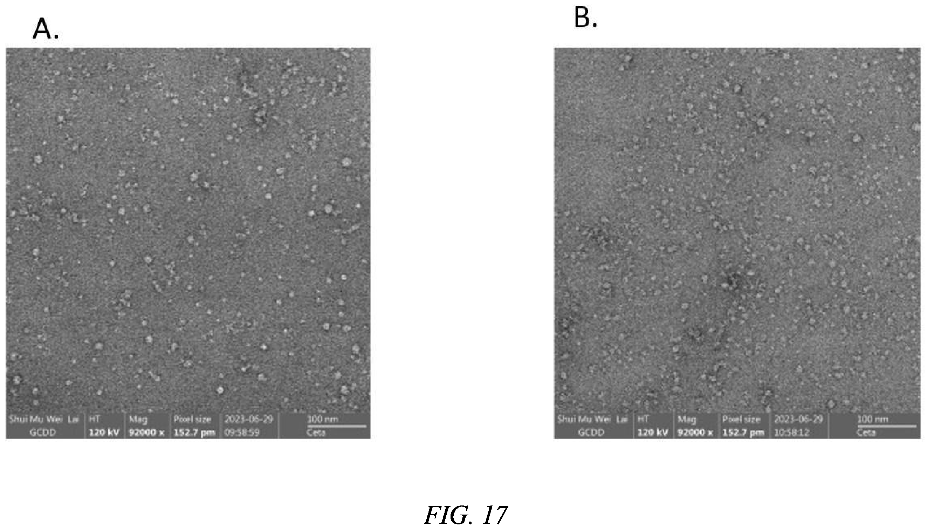

Figure 17 shows electron microscopy images of the RSV A2 and B strains.

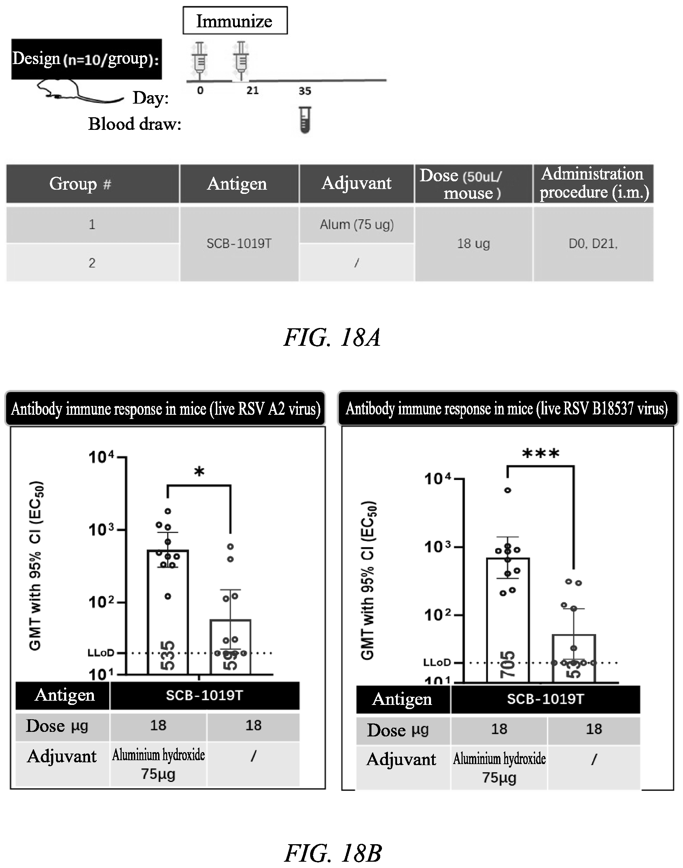

Figures 18A-18B show the immunogenicity assays of an exemplary trimerized fusion peptide bivalent vaccine in normal healthy naive mice. Figure 18A shows a schematic diagram of the experimental method: the mice were randomly divided into 2 groups of 10 mice each, and the mice in each group were immunized twice intramuscularly with the RSV virus vaccine candidate on D0 and D21 according to the table below; and blood samples were collected by retroorbital venipuncture on Day 35, and sera were obtained by centrifugation. The bivalent vaccine was SCB-1019T(A) + SCB-1019T(B) at a weight ratio of 1 : 1, with 75 µg of Alum per dose. Figure 18B shows the titer values of neutralizing antibodies induced by the bivalent trimerized fusion peptide vaccine against live hRSV A2 and hRSV B18537 viruses.

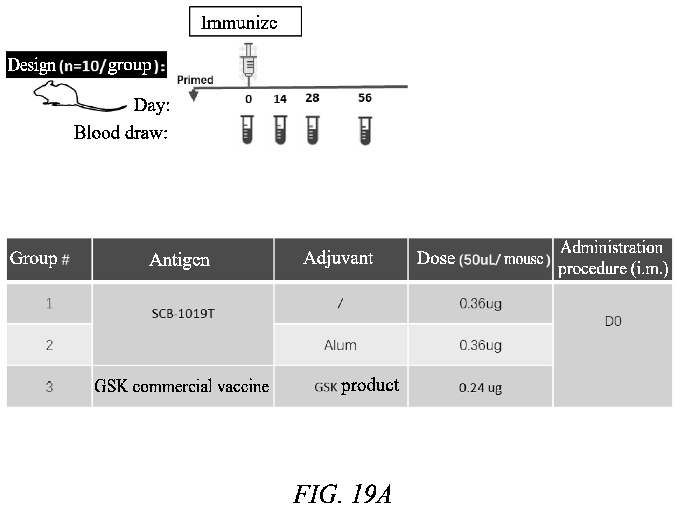

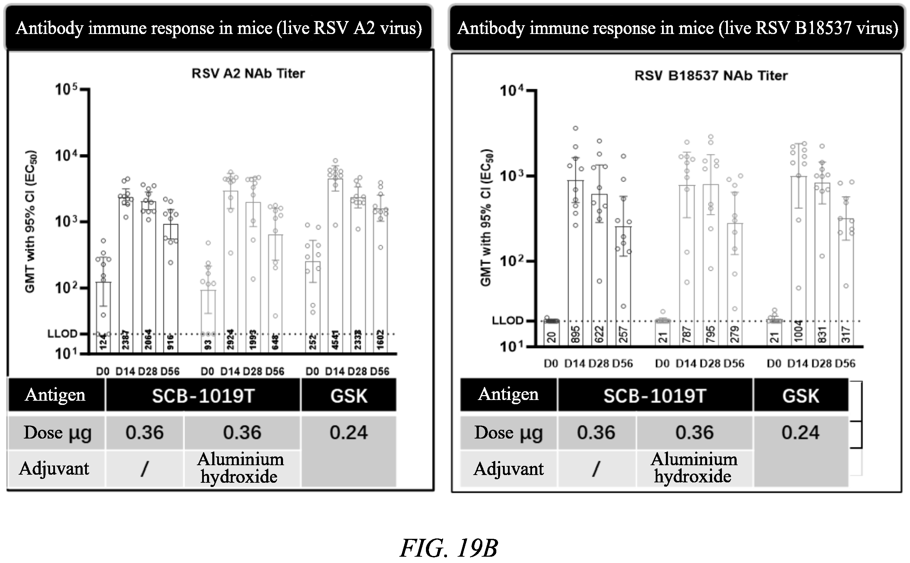

Figures 19A-19B show the immunogenicity assays of an exemplary trimerized fusion peptide bivalent vaccine in primed mice. Figure 19A shows a schematic diagram of the experimental method: 6- to 8-week-old SPF female BALB/c mice were intranasally infected with RSV virus to develop natural immunity; after 28 days, the mice were randomly divided into 3 groups of 10 mice each, and the mice in each group were immunized once intramuscularly with 50 µl of the RSV virus vaccine candidate or commercial GSK control vaccine according to the table below; and blood samples were collected by retroorbital venipuncture on Day 0, Day 14, Day 28, and Day 56, and sera were obtained by centrifugation. The bivalent vaccine was SCB-1019T(A) + SCB-1019T(B) at a weight ratio of 1 : 1, with 75 µg of Alum per dose. Figure 19B shows the titer values of neutralizing antibodies induced by the bivalent trimerized fusion peptide vaccine against live hRSV A2 and hRSV B18537 viruses.

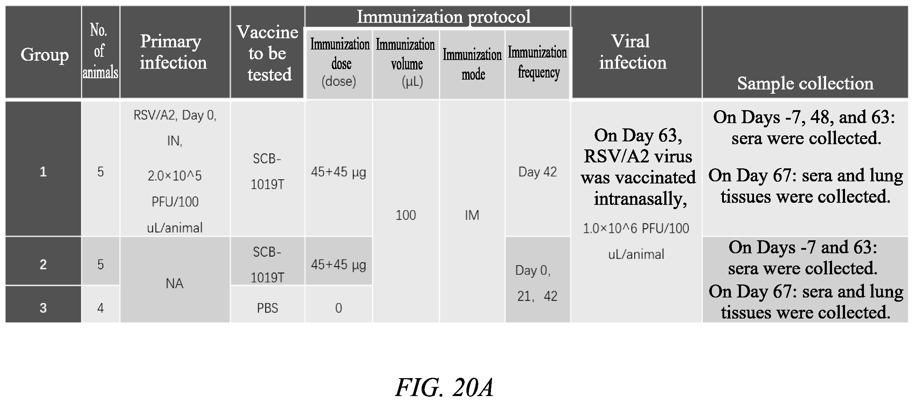

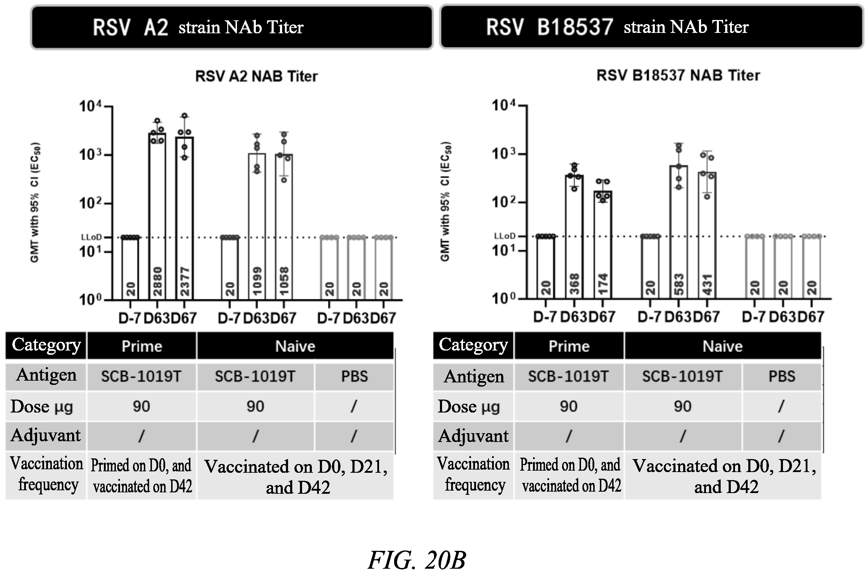

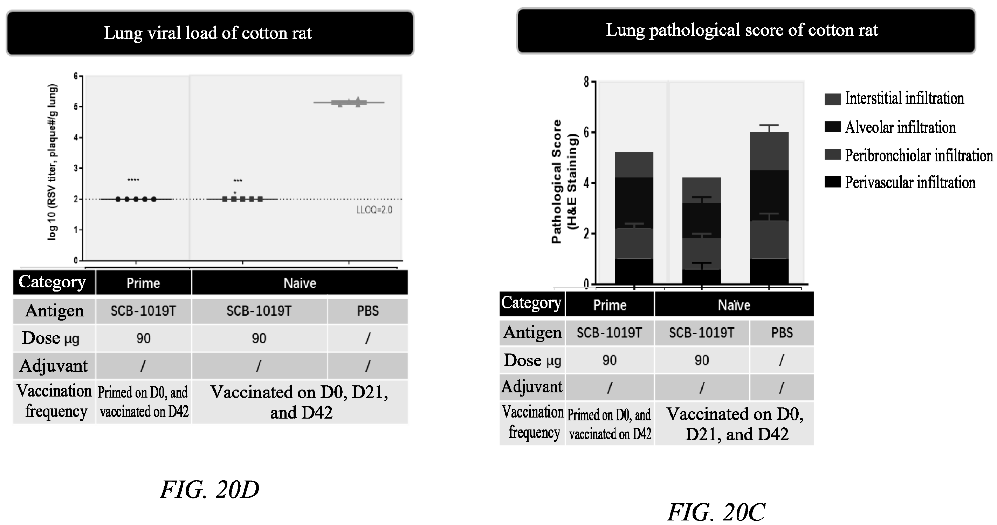

Figures 20A-20D show cotton rat challenge assays. Figure 20A shows a schematic diagram of the experimental method: a total of 14 cotton rats were used, and 5 cotton rats were intranasally infected with RSV virus to develop natural immunity, designated as Group 1; there were 2 groups of naive mice, and the mice in each group were immunized intramuscularly with 50 µL of the RSV virus vaccine candidate or control vaccine according to the table below; and blood samples were collected by retroorbital venipuncture at Week 9 (i.e., D21 post-boost) and 5 days post-challenge and sera were obtained by centrifugation, and mouse viral load and lung pathological sections were detected on Day 68. The bivalent vaccine was SCB-1019T(A) + SCB-1019T(B) at a weight ratio of 1 : 1, with a dose of 90 µg per antigen. Figure 20B shows the titer values of neutralizing antibodies induced by the bivalent trimerized fusion peptide vaccine against live hRSV A2 and hRSV B18537 viruses. Figure 20C shows pathological scores. Figure 20D shows lung viral load.

### Detailed Description of Embodiments

In some embodiments, a composition and a method of use of a recombinant soluble surface antigen from RNA viruses in a covalently linked trimeric form are disclosed. In some embodiments, the resulting fusion protein is secreted as a disulfide-linked homotrimer, which is more stable in structure, while retaining the conformation of the native-like trimeric viral antigens, and thus can be used as a more effective vaccine against these dangerous pathogens.

In some embodiments, disclosed herein is a method for using a viral antigen trimer as a vaccine or as part of a multivalent vaccine to prevent viral infections, with or without an adjuvant, or with more than one adjuvant, optionally via either intramuscular injection or intranasal administration.

In some embodiments, disclosed herein is a method for using a viral antigen trimer as an antigen to diagnose viral infections through detection of antibodies, e.g., IgM or IgG, that recognize the viral antigen, such as neutralizing antibodies.

In some embodiments, disclosed herein is a method for using a viral antigen trimer as an antigen to produce polyclonal or monoclonal antibodies which can be used for passive immunization, e.g., neutralizing mAbs for treating RSV infection in infants.

In some embodiments, disclosed herein is a viral antigen trimer as a vaccine or as part of a multivalent vaccine, wherein the vaccine comprises a plurality of trimeric subunit vaccines comprising viral antigens of the same protein of a virus or comprising viral antigens of two or more different proteins of one or more viruses or one or more strains of the same virus.

In some embodiments, disclosed herein is a monovalent vaccine comprising the viral antigen trimer disclosed herein. In some embodiments, disclosed herein is a bivalent vaccine comprising the viral antigen trimer disclosed herein. In some embodiments, disclosed herein is a trivalent vaccine comprising the viral antigen trimer disclosed herein. In some embodiments, disclosed herein is a quadrivalent vaccine comprising the viral antigen trimer disclosed herein.

In some embodiments, disclosed herein is a monovalent vaccine comprising the F-trimer disclosed herein. In some embodiments, disclosed herein is a bivalent vaccine comprising the F-trimer disclosed herein. In some embodiments, disclosed herein is a bivalent vaccine comprising at least one F-trimer comprising a first F protein antigen and at least one F-trimer comprising a second F protein antigen. In some embodiments, the first and second F protein antigens are derived from the same F protein of one or more virus species or strains/subtypes, or from two or more different F proteins of one or more virus species or one or more strains/subtypes of the same virus species. In some embodiments, disclosed herein is a trivalent vaccine comprising the F-trimer disclosed herein. In some embodiments, disclosed herein is a trivalent vaccine comprising at least one F-trimer comprising a first F protein antigen, at least one F-trimer comprising a second F protein antigen, and at least one F-trimer comprising a third F protein antigen. In some embodiments, the first, second, and third F protein antigens are derived from the same F protein of one or more virus species or strains/subtypes, or from two, three, or more different F proteins of one or more virus species or one or more strains/subtypes of the same virus species. In some embodiments, disclosed herein is a quadrivalent vaccine comprising the F-trimer disclosed herein. In some embodiments, disclosed herein is a quadrivalent vaccine comprising at least one F-trimer comprising a first F protein antigen, at least one F-trimer comprising a second F protein antigen, at least one F-trimer comprising a third F protein antigen, and at least one F-trimer comprising a fourth F protein antigen. In some embodiments, the first, second, third, and fourth F protein antigens are derived from the same F protein of one or more virus species or strains/subtypes, or from two, three, four, or more different F proteins of one or more virus species or one or more strains/subtypes of the same virus species.

Provided herein are an immunogenic composition, a method, and use of a fusion peptide or protein comprising an RSV viral antigen or immunogen for e.g., the prophylactic or therapeutic treatment of RSV infection. Respiratory syncytial virus (RSV) is considered to be the main cause of acute lower respiratory tract infections (ALRTIs) among infants and young children, and accounts for over 7,000 to 20,000 child deaths worldwide each year. RSV infection is the second most common cause of infant mortality in the developing countries. In addition, RSV can cause serious diseases in the elderly and immunocompromised populations. Palivizumab (SYNAGIS^{®}), a potent prophylactic humanized mAb, is only available as a passive immunization measure for those infants at high risk of RSV infection.

Despite decades of research, RSV vaccine development has been unsuccessful for numerous reasons. For example, issues with production, stability, and efficacy of RSV vaccine candidates have been difficult to overcome, and in particular, safety has been a main concern since it is recognized that formalin-inactivated RSV (FI-RSV) vaccines mediate vaccine-induced enhanced disease (VED).

The protein, including recombinant polypeptide and fusion protein, comprising an RSV viral antigen and immunogen provided herein is useful for effectively and safely treating (e.g., therapeutically or prophylactically) RSV infection. For example, the protein comprising an RSV viral antigen and immunogen provided herein treats RSV infection without regard to VED and/or antibody-dependent enhancement (ADE). In addition, the protein comprising an RSV viral antigen and immunogen provided herein is easily produced, and demonstrates stability under high stress conditions, such as high temperature, extreme pH, and high and low osmolality. Therefore, the protein and immunogenic composition provided herein circumvent and address the issues of production, stability, safety, and efficacy that have hindered RSV vaccine development.

In some aspects, the RSV viral antigen and immunogen provided herein comprise an RSV glycoprotein (F), also referred to herein as RSV F protein peptide or RSV F peptide. The RSV F protein peptide is a homotrimeric type I transmembrane protein that mediates membrane fusion and viral penetration into host cells. The RSV F protein peptide is synthesized as an F0 proprotein precursor, which is converted into disulfide-linked F1 and F2 mature forms after cleavage by furin at two sites. The RSV F protein peptide is highly conserved between RSV A and B strains. Neutralizing antibodies, such as palivizumab, target antigenic sites of F and provide protection against respiratory diseases caused by RSV infection.

In some embodiments, the protein comprising an RSV viral antigen or immunogen, e.g., an RSV F protein peptide, is capable of generating an immune response, e.g., an immune response against the RSV F peptide protein. In some embodiments, the immune response inhibits or reduces the replication of RSV in a subject, e.g., a patient. In some embodiments, the immune response comprises the production of one or more neutralizing antibodies, such as polyclonal and/or monoclonal antibodies. In some embodiments, the neutralizing antibodies inhibit or reduce the replication of RSV in a subject, e.g., a patient. In some embodiments, administration of the protein (e.g., in an immunogenic composition form) to a subject does not result in antibody-dependent enhancement (ADE) in the subject due to prior exposure to RSV. In some aspects, the protein comprising an RSV viral antigen and immunogen, e.g., an RSV F protein peptide, is used as a vaccine.

In some embodiments, the RSV viral antigen and immunogen, e.g., the RSV F protein peptide, are linked to a protein or peptide to form a fusion protein or recombinant polypeptide. In some embodiments, the protein or peptide to which the RSV viral antigen or immunogen is linked is capable of associating, e.g., covalently or non-covalently linking, with a protein or peptide, such as a protein or peptide of a fusion protein or recombinant polypeptide. Thus, in some cases, the protein or peptide to which the RSV viral antigen or immunogen is linked is a multimerization domain.

In some embodiments, the RSV viral antigen and immunogen, such as the RSV F protein peptide, are linked to a collagen propeptide, such as a collagen C-terminal propeptide, to form a fusion peptide or recombinant polypeptide. Thus, in some embodiments, the protein provided herein comprises a recombinant polypeptide comprising an RSV viral antigen and immunogen, e.g., an RSV F protein peptide, or a fragment or epitope thereof, linked to a collagen C-terminal propeptide. In some embodiments, the collagen propeptide is derived from the C-terminal propeptide of human α1 collagen and is capable of self-trimerization.

In some embodiments, linking the RSV viral antigen and immunogen, such as the RSV F protein peptide, to a collagen propeptide, such as a collagen C-terminal propeptide, contributes to the ability of the protein to generate an immune response. For example, the recombinant protein produced may preserve the tertiary and quaternary structures of the RSV F protein peptide, which may be important for the stability of the native conformation of the RSV F protein peptide, and in turn for the accessibility of antigenic sites on the surface of the protein capable of inducing an immune response, e.g., neutralizing antibodies. Additionally, linking the RSV F protein peptide to a protein or peptide capable of self-trimerization allows the aggregation of the recombinant protein, mimicking the native homotrimeric structure of the RSV F protein peptides on the viral envelope.

In some embodiments, linking the RSV F protein peptide to a collagen C-terminal propeptide results in a self-trimerizing recombinant polypeptide. In some embodiments, the protein provided herein comprises a plurality of self-trimerizing recombinant polypeptides formed from the RSV F protein peptide and the collagen propeptide, optionally wherein the plurality of recombinant proteins form, e.g., a rosette-like structure (see, e.g., Figure 2B of PCT/CN2021/099286, which is incorporated herein by reference in its entirety for all purposes). In some embodiments, the trimeric nature of the recombinant proteins contributes to the stability of the protein. In some embodiments, the macrostructure, e.g., rosette, formed from a plurality of self-trimerizing recombinant proteins contributes to the stability of the protein. In some embodiments, the trimeric nature of the recombinant proteins and the macrostructure, e.g., rosette, formed from a plurality of self-trimerizing recombinant proteins contribute to the stability of the protein. In some embodiments, the trimeric nature of the recombinant proteins contributes to the ability of the protein to generate an immune response. In some embodiments, the macrostructure, e.g., rosette, formed from a plurality of self-trimerizing recombinant proteins contributes to the ability of the protein to generate an immune response. In some embodiments, the trimeric nature of the recombinant proteins and the macrostructure formed from a plurality of self-trimerizing recombinant proteins contribute to the ability of the protein to generate an immune response.

Also provided herein are an immunogenic composition comprising the protein provided herein, a method for producing the protein provided herein, a method for treating a subject with the protein and composition provided herein, and a kit.

All publications, including patent documents, scientific articles, and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications, and other publications that are herein incorporated by reference, the definition set forth herein prevails over the definition that is incorporated herein by reference.

The section headings used herein are for organizational purposes only and are not to be construed as limiting the subject matter described.

### I. Viral Antigens and Immunogens

Respiratory syncytial virus (RSV) is the most common cause of acute lower respiratory infections in infants and young children, and a major disease burden in the elderly. Despite the fact that RSV virus was characterized half a century ago, there are currently limited vaccines against RSV, and development has been hampered by vaccine-mediated disease enhancement in children administered formalin-inactivated RSV in the 1960s. Challenges in antigen production, purity, stability, and potency of RSV vaccine candidates have also been impediments to development.

In some embodiments, the protein provided herein comprises an RSV viral antigen and/or immunogen. In some embodiments, the RSV viral antigen and/or immunogen herein are capable of promoting or stimulating a cell-mediated response and/or a humoral response. In some embodiments, the response, e.g., cell-mediated or humoral response, comprises the production of antibodies, e.g., neutralizing antibodies. In some embodiments, the neutralizing antibodies (NAbs) against the viral antigen and/or immunogen provide adaptive immune defense against RSV exposure by blocking the infection of susceptible cells. In some embodiments, the efficacy of a vaccine against several viruses is attributed to and/or correlated with its ability to induce NAbs. In some embodiments, the RSV viral antigen or immunogen is the RSV F protein peptide disclosed herein.

The RSV F protein peptide is an envelope glycoprotein of respiratory syncytial virus (RSV) (RSV as described herein includes all subtypes, such as subtypes A and B, when subtypes are not explicitly specified). The RSV F protein peptide is translated as a single precursor polypeptide (designated F0), and RSV F as described herein includes all subtypes, such as subtypes A and B, when subtypes are not explicitly specified, for example, RSV F from the RSV A2 strain (GenBank Accession No. AAC55970) and RSV F from the Australian RSV B strain. The RSV F protein mediates viral entry into cells and cell-cell fusion, is a target of neutralizing antibodies, and is highly conserved between RSV A and B strains. F0 can be cleaved at Arg109 and Arg136 by cellular furin to three fragments, wherein a shorter F2 polypeptide at the N-terminus is covalently linked by two disulfide bonds to a longer F1 polypeptide with an 18-amino acid fusion domain at the N-terminus and a hydrophobic transmembrane region near the C-terminus; and an intervening 27-amino acid fragment is released. Neutralizing monoclonal antibodies palivizumab and motavizumab bind to RSV F antigenic site II (Asn258-Val278), and have been shown to provide protection against both lower and upper respiratory RSV diseases in high-risk and term infants. The structure of the RSV F epitope peptides bound by these neutralizing antibodies is larger than that of the linear peptides, and palivizumab binds to RSV F with nanomolar affinity, whereas motavizumab binds to RSV F with picomolar affinity. Modeling predicts that the full extent of the binding of palivizumab and motavizumab requires amino acids from one or two RSV F protomers, respectively. Therefore, preserving the tertiary and quaternary structures of RSV F may be important in the development of RSV F vaccines that retain the native conformation of this important neutralizing region.

In some embodiments, the F0 precursor polypeptide is 574 amino acids in length as set forth in SEQ ID NO: 68 or 70.

In some embodiments, the F0 precursor polypeptide is 574 amino acids in length as set forth in SEQ ID NO: 67 or 69.

In some embodiments, the RSV F protein peptide herein comprises a substitution at one or more or all of residues 106, 107, 108, 109, 133, 135, and 136, and the substituting amino acids are independently selected from cysteine, alanine, threonine, tyrosine, glycine, or serine, or any combination thereof.

In some embodiments, the RSV F protein peptide herein comprises a substitution at one or more of amino acid residues 106, 107, 108, 109, 133, 135, and 136 as set forth in SEQ ID NO: 68 or 70, and the substituting amino acids are independently selected from cysteine, alanine, threonine, tyrosine, glycine, or serine, or any combination thereof.

In some embodiments, the RSV F protein peptide herein comprises a substitution, deletion, and/or insertion at and/or near residues 109, 136, 161, and/or 215. In some embodiments, the RSV F protein peptide herein comprises a substitution, deletion, and/or insertion at and/or near residues 109, 136, 161, and/or 215 of SEQ ID NO: 67 or 69. In some embodiments, the RSV F protein peptide herein comprises an alanine or proline at any one or more of residues 109, 136, 161, and/or 215 of SEQ ID NO: 67 or 69. In some embodiments, the RSV F protein peptide herein comprises an alanine at residue 109 of SEQ ID NO: 67 or 69. In some embodiments, the RSV F protein peptide herein comprises an alanine at residue 136 of SEQ ID NO: 67 or 69. In some embodiments, the RSV F protein peptide herein comprises an alanine at residues 109 and 136 of SEQ ID NO: 67 or 69. In some embodiments, the RSV F protein peptide herein comprises a proline at residue 161 of SEQ ID NO: 67 or 69. In some embodiments, the RSV F protein peptide herein comprises a proline at residue 215 of SEQ ID NO: 67 or 69. In some embodiments, the RSV F protein peptide herein comprises a proline at residues 161 and 215 of SEQ ID NO: 67 or 69.

In some embodiments, the RSV F protein peptide herein comprises an alanine at residues 109 and 136 and a proline at residues 161 and 215 of SEQ ID NO: 67 or 69.

In some embodiments, the RSV F protein peptide herein comprises a substitution, deletion, and/or insertion at and/or near any one or more of residues 131-154 of SEQ ID NO: 31 or 32. In some embodiments, the RSV F protein peptide herein comprises a deletion of 2, 3, 4, 5, 6, 7, 8, 9, 10, 11, 12, 13, 14, 15, 16, 17, 18, or more of residues 131-154 of SEQ ID NO: 31 or 32. In some embodiments, the RSV F protein peptide herein comprises a deletion of any one or more of residues 137-154 of SEQ ID NO: 31 or 32. In some embodiments, the RSV F protein peptide herein comprises a deletion of any one or more of residues 137-146 of SEQ ID NO: 31 or 32. In some embodiments, the RSV F protein peptide herein comprises a glutamine at residues 133, 135, and 136, and a deletion of residues 137-146 of SEQ ID NO: 31 or 32.

In some embodiments, the RSV F protein peptide of subtype A herein comprises amino acids 1-25 of the F0 precursor, which sequence is the signal peptide MELLILKANAITTILTAVTFCFASG (SEQ ID NO: 71). In some embodiments, the RSV F protein peptide of subtype B herein comprises amino acids 1-25 of the F0 precursor, which sequence is the signal peptide MELLIHRSSAIFLTLAINALYLTSS (SEQ ID NO: 72). In some embodiments, the precursor polypeptide F0 forms a precursor trimer. In some embodiments, the RSV F protein peptide herein is proteolytically cleaved by one or more cellular proteases, e.g., at conserved furin consensus cleavage sites, to yield a pep27 polypeptide (also referred to as p27), an F1 polypeptide, and an F2 polypeptide. In some embodiments, the pep27 polypeptide (e.g., amino acids 110-136 of the F0 precursor) is excised and in some aspects, does not form part of a mature RSV F-trimer. In some embodiments, the F2 polypeptide (which may alternatively be referred to herein as "F2" or "F2 subunit peptide") comprises amino acid residues 26-109 of the F0 precursor. In some embodiments, the F1 polypeptide (which may alternatively be referred to herein as "F1" or "F1 subunit peptide") comprises amino acid residues 137-574 of the F0 precursor, and may comprise an extracellular region (e.g., residues 137-524), a transmembrane domain (e.g., residues 525-550), and a cytoplasmic domain (e.g., residues 551-574).

In some embodiments, the RSV F protein peptide herein comprises F1 and F2 polypeptides linked by a disulfide bond to form a heterodimer, referred to as RSV F "protomer". In some embodiments, the RSV F protein peptide herein comprises three protomers that form an RSV F-trimer, and thus it is a homotrimer of the three protomers. In some embodiments, the RSV F protein peptide herein is a mature RSV F-trimer. In some embodiments, the RSV F protein peptide herein is membrane-bound. In some embodiments, the RSV F protein peptide herein is not membrane-bound. In some embodiments, the RSV F protein peptide herein is soluble and lacks the transmembrane region and the cytoplasmic region or fragments thereof. For example, conversion to a soluble form can be accomplished by truncating the RSV F protein at amino acid 513 (by removing amino acids from 514), 514, 515, 516, 517, 518, 519, 520, 521, 522, 523, or 524. In nature, the mature RSV F-trimer mediates fusion of viral and cellular membranes. The pre-fusion conformation of the mature RSV-F trimer (which may be referred to herein as "pre-F" or pre-fusion) is highly unstable (metastable). However, once RSV virus docks with the cell membrane, the RSV F protein trimer undergoes a series of conformational changes and transitions to a highly stable post-fusion ("post-F") conformation.

In some embodiments, the RSV viral antigen or immunogen comprises a signal peptide (SP) (e.g., amino acids 1-22 of SEQ ID NO: 68 or 70), or a fragment and/or mutant sequence thereof, a heptad-repeat C (HRC) (e.g., F2, which may be amino acids 23-109 of SEQ ID NO: 68 or 70), or a fragment and/or mutant sequence thereof, a furin cleavage site (FCS) (e.g., at the junction between amino acids 109/110 of SEQ ID NO: 68 or 70), or a fragment and/or mutant sequence thereof, a 27-mer fragment (pep27) (e.g., amino acids 110-136 of SEQ ID NO: 68 or 70), or a fragment and/or mutant sequence thereof, a putative fusion peptide (FP) (e.g., amino acids 137-155 of SEQ ID NO: 68 or 70), or a fragment and/or mutant sequence thereof, a heptad-repeat A (HRA) (e.g., amino acids 156-214 of SEQ ID NO: 68 or 70), or a fragment and/or mutant sequence thereof, Domains I and II (e.g., amino acids 215-476 of SEQ ID NO: 31 or 32), or fragments and/or mutant sequences thereof, a heptad-repeat B (HRB) (e.g., amino acids 477-524 of SEQ ID NO: 68 or 70), or a fragment and/or mutant sequence thereof, a transmembrane (TM) domain (e.g., amino acids 525-550 of SEQ ID NO: 68 or 70), or a fragment and/or mutant sequence thereof, and/or a cytoplasmic (CP) domain (e.g., amino acids 551-574 of SEQ ID NO: 68 or 70), or a fragment and/or mutant sequence thereof, in any suitable combination.

In some embodiments, the RSV viral antigen or immunogen is an RSV F protein peptide of RSV subtype A. In some embodiments, the RSV viral antigen or immunogen is an RSV F protein peptide of RSV subtype A2. In some embodiments, the RSV viral antigen or immunogen is an RSV F protein peptide of RSV subtype B. In some cases, the RSV F protein peptide is conserved across RSV subtypes.

In some cases, the RSV viral antigen or immunogen is a fragment of an RSV F protein peptide. In some embodiments, the RSV viral antigen or immunogen is an epitope of an RSV F protein peptide. In some embodiments, the epitope is a linear epitope. In some embodiments, the epitope is a conformational epitope. In some embodiments, the epitope is a neutralizing epitope site, for example, site I, II, or IV. In some embodiments, all neutralizing epitopes of the RSV F protein peptide or a fragment thereof are present as the RSV viral antigen or immunogen.

In some cases, for example, when the RSV viral antigen or immunogen is a fragment of an RSV F protein peptide, only a single subunit of the RSV F protein peptide is present.

In some embodiments, the RSV viral antigen or immunogen is or comprises an F1 subunit peptide. In some embodiments, the F1 subunit peptide is or comprises the amino acid sequence of 137-574 of the wild-type F protein. In some embodiments, the RSV viral antigen or immunogen is or comprises an F2 subunit peptide. In some embodiments, the RSV viral antigen or immunogen comprises an RSV F protein peptide comprising a signal peptide, a heptad-repeat C (HRC) peptide, a pep27 peptide, a fusion peptide (FP), a heptad-repeat A (HRA) peptide, a Domain I peptide, a Domain II peptide, or a heptad-repeat B (HRB) peptide, or any combination thereof. In some embodiments, the RSV viral antigen or immunogen comprises an RSV F protein peptide comprising a signal peptide. In some embodiments, the RSV viral antigen or immunogen comprises an RSV F protein peptide comprising a pep27 peptide. In some embodiments, the RSV viral antigen or immunogen comprises an RSV F protein peptide comprising a fusion peptide (FP) (also known as fusion domain (FD)). In some embodiments, the RSV viral antigen or immunogen comprises an RSV F protein peptide comprising a signal peptide, a pep27 peptide, and a fusion peptide (FP).

In some embodiments, the RSV viral antigen or immunogen comprises an RSV F protein peptide comprising an F1 subunit and an F2 subunit of the F protein. In some embodiments, the RSV viral antigen or immunogen comprises an RSV F protein peptide comprising an F1 subunit peptide and an F2 subunit peptide of the F protein without a pep27 peptide. In some embodiments, the RSV viral antigen or immunogen comprises an RSV F protein peptide comprising an F1 subunit peptide, an F2 subunit peptide, and a pep27 peptide of the F protein. In some embodiments, the RSV viral antigen or immunogen comprises an RSV F protein peptide comprising an F1 subunit peptide, an F2 subunit peptide, a pep27 peptide, and an FP of the F protein.

In some cases, for example, when the viral antigen or immunogen comprises both an F1 subunit peptide and an F2 subunit peptide of the RSV F protein peptide, the F1 and F2 subunits are linked. In some embodiments, the F1 and F2 subunits are linked by a disulfide bond. In some embodiments, the F1 and F2 subunits are linked by a pep27 peptide. For example, in some embodiments, the sequence in the direction from the N-terminus to the C-terminus is or comprises F2-pep27-F1. In some embodiments, the sequence in the direction from the N-terminus to the C-terminus is or comprises F2-pep27-FP-F1 (F2-pep27-FD-F1). In some embodiments, the FP is regarded as a structural feature of the F1 subunit peptide. In some embodiments, the F1 and F2 subunits are linked by an artificially introduced linker. For example, in some embodiments, the sequence in the direction from the N-terminus to the C-terminus is or comprises F2-artificially introduced linker-F1. The artificially introduced linker is a non-amino acid compound or one or more amino acids. In some embodiments, the F1 and F2 subunits are linked by an artificially introduced linker. In some embodiments, the one or more amino acids are CGGG (SEQ ID NO: 138). For example, in some embodiments, the sequence in the direction from the N-terminus to the C-terminus is or comprises F2-artificially introduced linker-F1, wherein F2 lacks furin site I, and/or F1 lacks furin site II, and/or pep27 peptide is absent, in particular, the artificially introduced linker replaces furin site I, the artificially introduced linker replaces furin site II, the artificially introduced linker replaces furin site I and pep27 peptide, or the artificially introduced linker replaces furin site I and pep27 peptide and one or more bases linked to furin site II. In some embodiments, the one or more bases substituted are selected from FLGFLLGV (SEQ ID NO: 126), e.g., F, FL, FLG, FLGF (SEQ ID NO: 127), FLGFL (SEQ ID NO: 128), FLGFLL (SEQ ID NO: 129), FLGFLLG (SEQ ID NO: 130), or FLGFLLGV (SEQ ID NO: 131). In some embodiments, the F1 polypeptide may comprise amino acid residues 145-574 of the F0 precursor, or may comprise an extracellular region (e.g., residues 145-520). In some embodiments, the F2 polypeptide may comprise amino acid residues 26-105 or 1-105 of the F0 precursor.

In some cases, the RSV viral antigen or immunogen is an RSV F protein peptide that does not comprise a transmembrane (TM) domain peptide. In some cases, the RSV F protein does not comprise a cytoplasmic (CP) domain peptide. In some cases, the RSV F protein does not comprise a TM domain peptide or a CP domain peptide.

In some embodiments, the RSV viral antigen or immunogen comprises an RSV F protein peptide comprising a protease cleavage site. In some embodiments, the protease cleavage site is specific for cleavage by the protease furin. In some embodiments, the protease cleavage site is specific for cleavage by the protease trypsin. In some embodiments, the protease cleavage site is specific for cleavage by the protease factor Xa. In some embodiments, the protease cleavage site is specific for cleavage by the protease cathepsin L.

In some cases, the RSV viral antigen or immunogen comprises an RSV F protein peptide that does not comprise a protease cleavage site. In some cases, the RSV viral antigen or immunogen comprises an RSV F protein peptide that does not comprise a protease cleavage site that is specific for cleavage by the protease furin. In some cases, the RSV viral antigen or immunogen comprises an RSV F protein peptide that does not comprise a protease cleavage site that is specific for cleavage by the protease trypsin. In some cases, the RSV viral antigen or immunogen comprises an RSV F protein peptide that does not comprise a protease cleavage site that is specific for cleavage by the protease factor Xa. In some cases, the RSV viral antigen or immunogen comprises an RSV F protein peptide that does not comprise a protease cleavage site that is specific for cleavage by the protease cathepsin L.

In some embodiments, the RSV viral antigen or immunogen comprises a soluble RSV F protein peptide. In some embodiments, the soluble RSV F protein peptide lacks a TM domain peptide and a CP domain peptide. In some embodiments, the soluble RSV F protein peptide does not bind to a lipid bilayer, such as a membrane or viral envelope.

In some embodiments, the RSV F protein peptide is produced from a nucleic acid sequence that has been codon-optimized. In some embodiments, the RSV F protein peptide is produced from a nucleic acid sequence that has not been codon-optimized.

In some embodiments, the RSV F protein peptide can comprise any F protein sequence known in the art, such as those disclosed in US Patent No. 10,017,543, which is incorporated herein by reference in its entirety for all purposes.

In some embodiments, the RSV viral antigen or immunogen is or comprises an RSV F protein peptide having the amino acid sequence of 1-520 of SEQ ID NO: 68 or 70. In some embodiments, the RSV viral antigen or immunogen is or comprises an RSV F protein peptide having the amino acid sequence of 26-520 of SEQ ID NO: 68 or 70.

In some embodiments, the RSV viral antigen or immunogen is or comprises the sequence of F2, the sequence of pep27, and the sequence of F1 (such as F2-pep27-F1). In some embodiments, the RSV viral antigen or immunogen comprises an exposed fusion peptide and has a post-fusion conformation. In some embodiments, the RSV viral antigen or immunogen comprises a mutation in the furin cleavage site. In some embodiments, the RSV viral antigen or immunogen comprises a mutation in the furin site I (e.g., R109A) and/or a mutation in the furin site II (e.g., R136A), and in some of these examples, the RSV viral antigen or immunogen has a post-fusion conformation, while in other examples, the RSV viral antigen or immunogen has a pre-fusion conformation. In some embodiments, the RSV viral antigen or immunogen comprises a mutation in the furin site I and a mutation in the furin site II (e.g., R109A/R136A), and in some of these examples, the RSV viral antigen or immunogen comprises a full-length F0 without an exposed fusion peptide and has a pre-fusion conformation. In some embodiments, the RSV viral antigen or immunogen comprises one or more mutations that prevent the formation of a long helix and/or stabilize the α4-α5 hinge loop. In some embodiments, the RSV viral antigen or immunogen comprises one or more mutations that retain a pre-fusion conformation. In some embodiments, the RSV viral antigen or immunogen comprises one or more mutations that improve expression. In some embodiments, substitutions at positions 161, 182 and 215 (e.g., with proline) result in higher expression levels, and E161P and S215P also increase protein stability. In some embodiments, the RSV viral antigen or immunogen comprises E161P and/or S215P and has a pre-fusion conformation. In some embodiments, the RSV viral antigen or immunogen comprises R109A, R136A, E161P, and/or S215P and has a pre-fusion conformation.

In some embodiments, the RSV viral antigen or immunogen comprises an amino acid substitution at any one, any two, any three, any four, any five, or all of amino acid positions 106, 108, 109, 133, 135, and 136 of the RSV F protein peptide. In some embodiments, the amino acids at one or more or all of amino acid positions 106, 108, 109, 133, 135, and 136 are substituted with cysteine. In some embodiments, the RSV F protein peptide herein comprises SEQ ID NO: 1 or 21 with all of residues 106, 108, 133, 135, and 136 substituted with cysteine.

In some embodiments, the viral antigen or immunogen comprises the sequence as set forth in SEQ ID NO: 11 or 31. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 11 or 31, including a sequence comprising a substitution, deletion, and/or insertion at one or more amino acid positions. In some embodiments, the RSV F protein peptide herein comprises a substitution at one or more of residues 106, 107, 108, 109, 133, 135, and 136 of SEQ ID NO: 11 or 31. In some embodiments, the RSV F protein peptide herein comprises SEQ ID NO: 11 or 31 with a substitution at one or more of residues 106, 108, 109, 133, 135, and 136. In some embodiments, the RSV F protein peptide herein comprises SEQ ID NO: 11 or 31 with one or more of residues 106, 108, 109, 133, 135, and 136 substituted with cysteine. In some embodiments, the RSV F protein peptide herein comprises SEQ ID NO: 11 or 31 with all of residues 106, 108, 133, 135, and 136 substituted with cysteine. The RSV F protein peptide herein comprises SEQ ID NO: 11 or 31 with all of residues 108, 133, 135, and 136 substituted with cysteine. The RSV F protein peptide herein comprises SEQ ID NO: 11 or 31 with all of residues 133, 135, and 136 substituted with cysteine. The RSV F protein peptide herein comprises SEQ ID NO: 11 or 31 with both of residues 135 and 136 substituted with cysteine. The RSV F protein peptide herein comprises SEQ ID NO: 11 or 31 with residue 136 substituted with cysteine. The RSV F protein peptide herein comprises SEQ ID NO: 11 or 31 with residue 135 substituted with cysteine. The RSV F protein peptide herein comprises SEQ ID NO: 11 or 31 with all of residues 106, 133, 135, and 136 substituted with cysteine. The RSV F protein peptide herein comprises SEQ ID NO: 11 or 31 with all of residues 106, 135, and 136 substituted with cysteine. The RSV F protein peptide herein comprises SEQ ID NO: 11 or 31 with all of residues 106, 133, and 136 substituted with cysteine. The RSV F protein peptide herein comprises SEQ ID NO: 11 or 31 with all of residues 106, 133, and 135 substituted with cysteine. The RSV F protein peptide herein comprises SEQ ID NO: 11 or 31 with both of residues 106 and 136 substituted with cysteine. The RSV F protein peptide herein comprises SEQ ID NO: 11 or 31 with both of residues 106 and 135 substituted with cysteine. The RSV F protein peptide herein comprises SEQ ID NO: 11 or 31 with both of residues 108 and 136 substituted with cysteine. The RSV F protein peptide herein comprises SEQ ID NO: 11 or 31 with both of residues 108 and 135 substituted with cysteine. The RSV F protein peptide herein comprises SEQ ID NO: 104 with both of residues 106 and 133 substituted with cysteine. The RSV F protein peptide herein comprises SEQ ID NO: 11 or 31 with both of residues 106 and 133 substituted with cysteine. The RSV F protein peptide herein comprises SEQ ID NO: 11 or 31 with both of residues 108 and 133 substituted with cysteine. The RSV F protein peptide herein comprises SEQ ID NO: 11 or 31 with both of residues 108 and 133 substituted with cysteine. The RSV F protein peptide herein comprises SEQ ID NO: 11 or 31 with all of residues 106, 108, 133, 135, and 136 substituted with alanine. The RSV F protein peptide herein comprises SEQ ID NO: 11 or 31 with all of residues 106, 108, 133, 135, and 136 substituted with threonine. The RSV F protein peptide herein comprises SEQ ID NO: 11 or 31 with all of residues 106, 108, 133, 135, and 136 substituted with tyrosine. The RSV F protein peptide herein comprises SEQ ID NO: 11 or 31 with all of residues 106, 108, 133, 135, and 136 substituted with histidine. The RSV F protein peptide herein comprises SEQ ID NO: 11 or 31 with all of residues 106, 108, 133, 135, and 136 substituted with serine.

In some embodiments, the viral antigen or immunogen comprises the sequence as set forth in any one of SEQ ID NOs: 11-20, 31-40, 49-53, and 62-120. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 11-20, 31-40, 49-53, and 62-120, including a sequence comprising a substitution, deletion, and/or insertion at one or more amino acid positions.

In some embodiments, the viral antigen or immunogen comprises the sequence as set forth in SEQ ID NO: 100. In some embodiments, the viral antigen or immunogen comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 100, including a sequence comprising a substitution, deletion, and/or insertion at one or more amino acid positions. In some embodiments, the RSV F protein peptide herein comprises a substitution at one or more of residues 106, 107, 108, 109, 133, 135, and 136 of SEQ ID NO: 100. In some embodiments, the RSV F protein peptide herein comprises SEQ ID NO: 100 with a substitution at one or more of residues 106, 108, 109, 133, 135, and 136. In some embodiments, the RSV F protein peptide herein comprises SEQ ID NO: 102 with one or more of residues 106, 108, 109, 133, 135, and 136 substituted with cysteine. In some embodiments, the RSV F protein peptide herein comprises SEQ ID NO: 100 with all of residues 106, 108, 109, 133, 135, and 136 substituted with cysteine. The RSV F protein peptide herein comprises SEQ ID NO: 100 with all of residues 108, 109, 133, 135, and 136 substituted with cysteine. The RSV F protein peptide herein comprises SEQ ID NO: 100 with all of residues 109, 133, 135, and 136 substituted with cysteine. The RSV F protein peptide herein comprises SEQ ID NO: 100 with all of residues 133, 135, and 136 substituted with cysteine. The RSV F protein peptide herein comprises SEQ ID NO: 100 with both of residues 135 and 136 substituted with cysteine. The RSV F protein peptide herein comprises SEQ ID NO: 100 with residue 136 substituted with cysteine. The RSV F protein peptide herein comprises SEQ ID NO: 100 with residue 135 substituted with cysteine. The RSV F protein peptide herein comprises SEQ ID NO: 100 with all of residues 106, 133, 135, and 136 substituted with cysteine. The RSV F protein peptide herein comprises SEQ ID NO: 100 with all of residues 106, 135, and 136 substituted with cysteine. The RSV F protein peptide herein comprises SEQ ID NO: 100 with all of residues 106, 133, and 136 substituted with cysteine. The RSV F protein peptide herein comprises SEQ ID NO: 100 with all of residues 106, 133, and 135 substituted with cysteine. The RSV F protein peptide herein comprises SEQ ID NO: 100 with both of residues 106 and 136 substituted with cysteine. The RSV F protein peptide herein comprises SEQ ID NO: 100 with both of residues 106 and 135 substituted with cysteine. The RSV F protein peptide herein comprises SEQ ID NO: 100 with both of residues 108 and 136 substituted with cysteine. The RSV F protein peptide herein comprises SEQ ID NO: 100 with both of residues 108 and 135 substituted with cysteine. The RSV F protein peptide herein comprises SEQ ID NO: 100 with both of residues 106 and 133 substituted with cysteine. The RSV F protein peptide herein comprises SEQ ID NO: 100 with both of residues 106 and 133 substituted with cysteine. The RSV F protein peptide herein comprises SEQ ID NO: 100 with both of residues 108 and 133 substituted with cysteine. The RSV F protein peptide herein comprises SEQ ID NO: 100 with both of residues 108 and 133 substituted with cysteine.

In some embodiments, the viral antigen or immunogen herein may comprise an RSV glycoprotein (G), or a fragment, variant, or mutant thereof; an RSV small hydrophobic protein (SH), or a fragment, variant, or mutant thereof; an RSV fusion protein (F), or a fragment, variant, or mutant thereof; an RSV matrix protein (M), or a fragment, variant, or mutant thereof; an RSV nucleoprotein (N), or a fragment, variant, or mutant thereof; an RSV phosphoprotein (P), or a fragment, variant, or mutant thereof; an RSV "large" protein (L), or a fragment, variant, or mutant thereof; an RSV M2-1 protein, or a fragment, variant, or mutant thereof; an RSV M2-2 protein, or a fragment, variant, or mutant thereof; an RSV NS-1 protein, or a fragment, variant, or mutant thereof; or an RSV NS-2 protein, or a fragment, variant, or mutant thereof; or any combination thereof.

In some embodiments, the viral antigen or immunogen is produced from a nucleic acid sequence that has been codon-optimized. In some embodiments, the viral antigen or immunogen is produced from a nucleic acid sequence that has not been codon-optimized.

In some embodiments, the RSV viral antigen or immunogen as referred to herein can include a recombinant polypeptide or fusion polypeptide comprising the viral antigen or immunogen. The term viral antigen or immunogen may be used to refer to a protein comprising an RSV viral antigen or immunogen. In certain cases, the RSV viral antigen or immunogen is an RSV protein peptide as provided herein.

### II. Recombinant Peptides and Proteins

It is contemplated that the RSV viral antigen and immunogen provided herein, e.g., the RSV F protein peptide (see Section I), can be combined, e.g., linked, with other proteins or peptides to form a recombinant polypeptide, including a fusion peptide. In some embodiments, individual recombinant polypeptides (e.g., monomers) provided herein associate to form a multimer, e.g., a trimer, of the recombinant polypeptides. In some embodiments, association of the individual recombinant polypeptide monomers occurs via covalent interactions. In some embodiments, association of the individual recombinant polypeptide monomers occurs via non-covalent interactions. In some embodiments, the interactions, e.g., covalent or non-covalent, are affected by the protein or peptide to which the RSV viral antigen or immunogen, e.g., the RSV F protein peptide, is linked. In some embodiments, for example, when the RSV viral antigen or immunogen is an RSV F protein peptide as described herein, the protein or peptide to which it is to be linked can be selected such that the native homotrimeric structure of the glycoprotein is preserved. This may be advantageous for eliciting a strong and effective immunogenic response to the RSV F protein peptide. For example, retaining and/or maintaining the native conformation of the RSV viral antigen or immunogen (e.g., the RSV F protein peptide) may improve or allow access to antigenic sites capable of generating an immune response. In some cases, the recombinant polypeptide comprising an RSV F protein peptide described herein, e.g., see Section I, is referred to herein alternatively as recombinant RSV F antigen, recombinant RSV F immunogen, or recombinant RSV F protein.

It is further contemplated that in some cases, the recombinant polypeptides or multimerized recombinant polypeptides thereof aggregate or can aggregate to form a protein comprising a plurality of RSV viral antigen and/or immunogen recombinant polypeptides. Formation of such proteins may be advantageous for generating a strong and effective immunogenic response against the RSV viral antigen and/or immunogen. For instance, the formed protein comprising a plurality of recombinant polypeptides, and thus a plurality of formed RSV viral antigens (e.g., RSV F protein peptides) may preserve the tertiary and/or quaternary structures of the viral antigens, allowing for an enhanced immune response against the native structure. In some cases, the aggregation may confer structural stability to the RSV viral antigen or immunogen, which in turn can afford access to potential antigenic sites capable of promoting an immune response.

### 1. Fusion peptides and recombinant polypeptides

In some embodiments, the RSV viral antigen or immunogen can be linked at its C-terminus (C-terminal linkage) to a trimerization domain to promote monomer trimerization. In some embodiments, the trimerization stabilizes the membrane-proximal structure of the RSV viral antigen or immunogen, e.g., the RSV F protein peptide, in a trimeric configuration.

Non-limiting examples of exogenous multimerization domains that promote the formation of stable trimers in soluble recombinant proteins include: the GCN4 leucine zipper (Harbury et al., 1993 Science 262: 1401-1407), the trimerization motif from the lung surfactant protein (Hoppe et al., 1994 FEBS Lett 344: 191-195), collagen (McAlinden et al., 2003 J Biol Chem 278: 42200-42207), and the phage T4 fibritin foldon (Miroshnikov et al., 1998 Protein Eng 11: 329-414), any one of which can be linked to the recombinant RSV viral antigen or immunogen described herein (e.g., by linking to the C-terminus of the RSV F peptide) to promote the trimerization of the recombinant viral antigen or immunogen. See also US Patent Nos. 7,268,116, 7,666,837, 7,691,815, 10,618,949, 10,906,944, and 10,960,070, and US 2020/0009244, which are incorporated herein by reference in their entireties for all purposes.

In some embodiments, one or more peptide linkers (such as a gly-ser linker, for example, a 10-amino acid glycine-serine peptide linker) can be used to link the recombinant viral antigen or immunogen to the multimerization domain. The trimer can comprise any one of the stabilizing mutations described herein (or a combination thereof), as long as the recombinant viral antigen or immunogen trimer retains the desired properties (e.g., the pre-fusion conformation).

To be therapeutically feasible, a desired trimerizing protein moiety for biologic drug designs should satisfy the following criteria. Ideally, it should be part of a naturally secreted protein, like immunoglobulin Fc, also abundant (non-toxic) in the circulation, derived from humans (lack of immunogenicity), relatively stable (long half-life), and capable of efficient self-trimerization which is strengthened by inter-chain covalent disulfide bonds, so the trimerized RSV viral antigen or immunogen is structurally stable.

Collagen belongs to the fibrous protein family and is a major component of the extracellular matrix. It is the most abundant protein in mammals, accounting for nearly 25% of the total protein in the body. Collagen plays a major structural role in the formation of bone, tendon, skin, cornea, cartilage, blood vessels, and teeth. Fibrillar collagens of types I, II, III, IV, V, and XI are all synthesized as larger trimeric precursors, called procollagens, in which the central uninterrupted triple-helical domain consisting of hundreds of "G-X-Y" repeats (or glycine repeats) is flanked by non-collagenous domains (NCs), namely the N-terminal propeptide and the C-terminal propeptide. Both the C- and N-terminal extensions are processed proteolytically upon secretion of the procollagen, an event that triggers the assembly of the mature protein into collagen fibrils which form an insoluble extracellular matrix. BMP-1 is a protease that recognizes a specific peptide sequence of procollagen near the junction between the glycine repeats and the collagen C-terminal prodomain and is responsible for the removal of the propeptide. The cleaved trimeric C-terminal propeptide of type I collagen is found in the sera of normal adults at a concentration in the range of 50-300 ng/mL, with much higher levels in children, which is indicative of active bone formation. In humans with familial high serum concentrations of C-terminal propeptide of type I collagen, the levels can reach as high as 1-6 µg/mL with no apparent abnormalities, indicating that the C-terminal propeptide is not toxic. Structural studies of the collagen trimeric C-terminal propeptide indicate that it is a trefoil structure in which all three subunits associate with one another in a junction region near their N-termini and link to the rest of the procollagen molecule. The geometry of the protein to be fused projecting in one direction is similar to that of the Fc dimer.

Type I, IV, V, and XI collagens mainly assemble into heterotrimeric forms consisting of either two α-1 chains and one α-2 chain (for type I, IV, and V), or three different chains which are highly homologous in sequence (for type XI). Both type II and III collagens are homotrimers of α-1 chains. For type I collagen, the most abundant form of collagen, a stable α(I) homotrimer is also formed and is present at variable levels in different tissues. Most of these collagen C-terminal propeptide chains can self-assemble into homotrimers, when over-expressed alone in cells. Although the N-terminal propeptide domains are synthesized first, molecular assembly into trimeric collagen begins with the aligned association of the C-terminal propeptides. It is believed that the C-terminal propeptide complex is stabilized by the formation of inter-chain disulfide bonds, but the necessity of disulfide bond formation for proper chain alignment is not clear. The triple helix formed by glycine repeats and then propagates in a zipper-like manner from the associated C-terminus to the N-terminus. This knowledge has led to the production of non-natural types of collagen matrix by swapping the C-terminal propeptides of different collagen chains using recombinant DNA technology. Noncollagenous proteins, such as cytokines and growth factors, also have been fused to the N-terminus of either procollagen or mature collagen to allow new collagen matrix formation, which is intended to allow slow release of the noncollagenous proteins from the extracellular matrix. However, in both cases, the C-terminal propeptides are required to be cleaved before the recombinant collagen fibrils assemble into an insoluble extracellular matrix.

Although other protein trimerization domains, such as GCN4 from yeast, fibritin from phage T4, and aspartate transcarbamoylase from *Escherichia coli*, have been described previously to allow the trimerization of heterologous proteins, none of these trimerizing proteins are native human proteins, nor are they naturally secreted proteins. Therefore, any trimeric fusion protein must be produced intracellularly, which may not only cause incorrect folding of naturally secreted proteins (such as soluble receptors), but also make it difficult to purify the fusion protein from thousands of other intracellular proteins. Moreover, the critical drawback of using such non-human protein trimerization domains (e.g., from yeast, phage, and bacteria) for trimeric biologic drug design is their putative immunogenicity in humans, rendering such fusion proteins ineffective shortly after being injected into the body.

The use of collagen in a recombinant polypeptide as described herein thus has many advantages, including: (1) collagen is the most abundant protein secreted in mammals, accounting for nearly 25% of the total protein in the body; (2) the major forms of collagen naturally occur as trimeric helixes, and their globular C-terminal propeptides are responsible for initiating trimerization; (3) the collagen trimeric C-terminal propeptide proteolytically released from the mature collagen is found to be naturally present at submicrogram/mL level in the blood of mammals, and is known to be non-toxic to the body; (4) the linear triple-helical region of collagen can be included as a linker with predicted spacing of 2.9 Å per residue, or excluded as part of the fusion protein, so the distance between a protein to be trimerized and the collagen C-terminal propeptide can be precisely adjusted to achieve an optimal biological activity; (5) the recognition site of BMP1 which cleaves the C-terminal propeptide from the procollagen can be mutated or deleted to prevent the disruption of a trimeric fusion protein; and (6) the C-terminal propeptide domain self-trimerizes via disulfide bonds and it provides a universal affinity tag, which can be used for purification of any secreted fusion protein produced. In some embodiments, the collagen C-terminal propeptide to which the RSV viral antigen and immunogen, e.g., the RSV F protein peptide, are linked enables the recombinant production of soluble, covalently-linked homotrimeric fusion proteins.

In some embodiments, the RSV viral antigen or immunogen is linked to a collagen C-terminal propeptide to form a recombinant polypeptide. In some embodiments, the C-terminal propeptides of the recombinant polypeptides form inter-polypeptide disulfide bonds. In some embodiments, the recombinant proteins form a trimer. In some embodiments, the RSV viral antigen or immunogen is an RSV F protein peptide as described in Section I.

In some embodiments, the C-terminal propeptide is of human collagen. In some embodiments, the C-terminal propeptide comprises a C-terminal polypeptide of proα1(I), proα1(II), proα1(III), proα1(V), proα1(XI), proα2(I), proα2(V), proα2(XI), or proα3(XI), or a fragment thereof. In some embodiments, the C-terminal propeptide is or comprises a C-terminal polypeptide of proα1(I).

In some embodiments, the C-terminal propeptide is or comprises the amino acid sequence as set forth in SEQ ID NO: 103. In some embodiments, the C-terminal propeptide is an amino acid sequence having at least or about 85%, 90%, 92%, 95%, or 97% sequence identity to the sequence of SEQ ID NO: 103. In some embodiments, the C-terminal propeptide is or comprises the amino acid sequence as set forth in SEQ ID NO: 104. In some embodiments, the C-terminal propeptide is an amino acid sequence having at least or about 85%, 90%, 92%, 95%, or 97% sequence identity to the sequence of SEQ ID NO: 104. In some embodiments, the C-terminal propeptide is or comprises the amino acid sequence as set forth in SEQ ID NO: 105. In some embodiments, the C-terminal propeptide is an amino acid sequence having at least or about 85%, 90%, 92%, 95%, or 97% sequence identity to the sequence of SEQ ID NO: 105. In some embodiments, the C-terminal propeptide is or comprises the amino acid sequence as set forth in SEQ ID NO: 106. In some embodiments, the C-terminal propeptide is an amino acid sequence having at least or about 85%, 90%, 92%, 95%, or 97% sequence identity to the sequence of SEQ ID NO: 106. In some embodiments, the C-terminal propeptide is or comprises the amino acid sequence as set forth in SEQ ID NO: 107. In some embodiments, the C-terminal propeptide is an amino acid sequence having at least or about 85%, 90%, 92%, 95%, or 97% sequence identity to the sequence of SEQ ID NO: 107. In some embodiments, the C-terminal propeptide is or comprises the amino acid sequence as set forth in SEQ ID NO: 108. In some embodiments, the C-terminal propeptide is an amino acid sequence having at least or about 85%, 90%, 92%, 95%, or 97% sequence identity to the sequence of SEQ ID NO: 108.

In some embodiments, the C-terminal propeptide is or comprises the amino acid sequence as set forth in SEQ ID NO: 109. In some embodiments, the C-terminal propeptide is an amino acid sequence having at least or about 85%, 90%, 92%, 95%, or 97% sequence identity to the sequence of SEQ ID NO: 109. In some embodiments, the C-terminal propeptide is or comprises the amino acid sequence as set forth in SEQ ID NO: 110. In some embodiments, the C-terminal propeptide is an amino acid sequence having at least or about 85%, 90%, 92%, 95%, or 97% sequence identity to the sequence of SEQ ID NO: 110. In some embodiments, the C-terminal propeptide is or comprises the amino acid sequence as set forth in SEQ ID NO: 111. In some embodiments, the C-terminal propeptide is an amino acid sequence having at least or about 85%, 90%, 92%, 95%, or 97% sequence identity to the sequence of SEQ ID NO: 111. In some embodiments, the C-terminal propeptide is or comprises the amino acid sequence as set forth in SEQ ID NO: 112. In some embodiments, the C-terminal propeptide is an amino acid sequence having at least or about 85%, 90%, 92%, 95%, or 97% sequence identity to the sequence of SEQ ID NO: 112. In some embodiments, the C-terminal propeptide is or comprises the amino acid sequence as set forth in SEQ ID NO: 115. In some embodiments, the C-terminal propeptide is an amino acid sequence having at least or about 85%, 90%, 92%, 95%, or 97% sequence identity to the sequence of SEQ ID NO: 113. In some embodiments, the C-terminal propeptide is or comprises the amino acid sequence as set forth in SEQ ID NO: 114. In some embodiments, the C-terminal propeptide is an amino acid sequence having at least or about 85%, 90%, 92%, 95%, or 97% sequence identity to the sequence of SEQ ID NO: 114.

In some embodiments, the C-terminal propeptide is or comprises the amino acid sequence as set forth in SEQ ID NO: 115. In some embodiments, the C-terminal propeptide is an amino acid sequence having at least or about 85%, 90%, 92%, 95%, or 97% sequence identity to the sequence of SEQ ID NO: 115. In some embodiments, the C-terminal propeptide is or comprises the amino acid sequence as set forth in SEQ ID NO: 116. In some embodiments, the C-terminal propeptide is an amino acid sequence having at least or about 85%, 90%, 92%, 95%, or 97% sequence identity to the sequence of SEQ ID NO: 116. In some embodiments, the C-terminal propeptide is or comprises the amino acid sequence as set forth in SEQ ID NO: 117. In some embodiments, the C-terminal propeptide is an amino acid sequence having at least or about 85%, 90%, 92%, 95%, or 97% sequence identity to the sequence of SEQ ID NO: 117. In some embodiments, the C-terminal propeptide is or comprises the amino acid sequence as set forth in SEQ ID NO: 118. In some embodiments, the C-terminal propeptide is an amino acid sequence having at least or about 85%, 90%, 92%, 95%, or 97% sequence identity to the sequence of SEQ ID NO: 118.

In some embodiments, the C-terminal propeptide is or comprises the amino acid sequence of a collagen trimerization domain (e.g., the C-terminal propeptide of human α1(I) collagen) with an aspartic acid (D) to asparagine (N) substitution in the BMP-1 site, for instance where RAD is mutated to RAN. In some embodiments, the C-terminal propeptide is or comprises the amino acid sequence of a collagen trimerization domain (e.g., the C-terminal propeptide of human α1(I) collagen) with an alanine (A) to asparagine (N) substitution in the BMP-1 site, for instance where RAD is mutated to RND. In some embodiments, the C-terminal propeptide herein may comprise a mutated BMP-1 site, e.g., RSAN (SEQ ID NO: 121) instead of DDAN (SEQ ID NO: 122). In some embodiments, the C-terminal propeptide herein may comprise a BMP-1 site, for example, a sequence comprising the RAD (e.g., RADDAN (SEQ ID NO: 123)) sequence instead of RAN (e.g., RANDAN (SEQ ID NO: 124)) or RND (e.g., RNDDAN (SEQ ID NO: 125)) may be used in the fusion polypeptide disclosed herein.

In some embodiments, the C-terminal propeptide is or comprises an amino acid sequence that is a fragment of any one of SEQ ID NOs: 103-118.

In some embodiments, the C-terminal propeptide can comprise a sequence comprising glycine-X-Y repeats, wherein X and Y are independently any amino acid, or an amino acid sequence having at least 85%, 90%, 92%, 95%, or 97% identity thereto, capable of forming inter-polypeptide disulfide bonds and trimerizing the recombinant polypeptides. In some embodiments, X and Y are independently proline or hydroxyproline.

In some cases where an RSV F peptide protein (e.g., RSV viral antigen or immunogen, e.g., see Section I) is linked to the C-terminal propeptide to form a recombinant polypeptide, the recombinant polypeptides form a trimer, resulting in a homotrimer of RSV F protein peptides. In some embodiments, the trimerized recombinant polypeptide comprises an F protein peptide trimer as a crutch-shaped rod. In some embodiments, the RSV F protein peptide of the trimerized recombinant polypeptide is in a pre-fusion conformation. In some embodiments, the RSV F protein peptide of the trimerized recombinant polypeptide is in a post-fusion conformation. In some embodiments, the conformation state allows for access to different antigenic sites on the F protein peptide. In some embodiments, the antigenic site is an epitope, such as a linear epitope or a conformational epitope. An advantage of having the trimerized recombinant polypeptide is that an immune response can be enhanced against a variety of potential and diverse antigenic sites.

In some embodiments, the trimerized recombinant polypeptide comprises individual recombinant polypeptides comprising the same viral antigen or immunogen. In some embodiments, the trimerized recombinant polypeptide comprises individual recombinant polypeptides each comprising a different viral antigen or immunogen from other recombinant polypeptides. In some embodiments, the trimerized recombinant polypeptide comprises individual recombinant polypeptides, wherein one of the individual recombinant polypeptides comprises a viral antigen or immunogen different from other recombinant polypeptides. In some embodiments, the trimerized recombinant polypeptide comprises individual recombinant polypeptides, wherein two of the individual recombinant polypeptides comprise the same viral antigen or immunogen, and the viral antigen or immunogen is different from that comprised in the remaining recombinant polypeptide.

In some embodiments, the recombinant polypeptide comprises any RSV viral antigen or immunogen described in Section I. In some embodiments, the recombinant polypeptide comprises any RSV viral antigen or immunogen described in Section I linked, as described herein, to the collagen C-terminal propeptide as described herein.

In some embodiments, the recombinant polypeptide or the fusion protein comprises a first sequence as set forth in any one of SEQ ID NOs: 11-20, 31-40, 49-53, 62-102, and 119-120 linked to a second sequence as set forth in any one of SEQ ID NOs: 103-118, wherein the C-terminus of the first sequence is linked directly to the N-terminus of the second sequence.

In some embodiments, the recombinant polypeptide or the fusion protein comprises a first sequence as set forth in any one of SEQ ID NOs: 11-20, 31-40, 49-53, 62-102, and 119-120 linked to a second sequence as set forth in any one of SEQ ID NOs: 103-118, wherein the C-terminus of the first sequence is linked indirectly to the N-terminus of the second sequence, e.g., via a linker. In some embodiments, the linker comprises a sequence comprising glycine-X-Y repeats. In some embodiments, the recombinant polypeptide or the fusion protein comprises a first sequence as set forth in any one of SEQ ID NOs: 11-20, 31-40, and 119-120 linked to a second sequence as set forth in any one of SEQ ID NOs: 103-118, wherein the C-terminus of the first sequence is linked indirectly to the N-terminus of the second sequence, e.g., via a linker. In some embodiments, the linker comprises a sequence comprising glycine-X-Y repeats. In some embodiments, the recombinant polypeptide or the fusion protein comprises a first sequence as set forth in any one of SEQ ID NOs: 49-53 and 62-102 linked to a second sequence as set forth in any one of SEQ ID NOs: 103-118, wherein the C-terminus of the first sequence is linked indirectly to the N-terminus of the second sequence, e.g., via a linker. In some embodiments, the linker comprises a sequence comprising glycine-X-Y repeats.

In some embodiments, the recombinant polypeptide is or comprises a sequence independently selected from the sequences as set forth in SEQ ID NOs: 11-20, 31-40, 49-53, 62-102, and 119-120. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to a sequence independently selected from SEQ ID NOs: 64-71, 80-89, 98-99, and 102-103.

In some embodiments, the recombinant polypeptide or the fusion protein comprises a first sequence as set forth in any one of SEQ ID NOs: 11-20, 31-40, 49-53, and 62-66 linked to a second sequence as set forth in any one of SEQ ID NOs: 103-118, wherein the C-terminus of the first sequence is linked indirectly to the N-terminus of the second sequence, e.g., via a linker. In some embodiments, the linker comprises a sequence comprising glycine-X-Y repeats.

In some embodiments, the recombinant polypeptide is or comprises the sequence as set forth in SEQ ID NO: 1 or 21. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 1 or 21, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 1 or 21 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 1 or 21 comprising any one, two, three, four, five, or more mutations selected from R106A, R108A, R133A, R135A, and R136A, or any combination thereof. In some embodiments, the recombinant polypeptide is or comprises a variant of SEQ ID NO: 1 or 21 comprising any one, two, three, four, five, or more mutations selected from R106T, R108T, R133T, R135T, and R136T, or any combination thereof. In some embodiments, the recombinant polypeptide is or comprises a variant of SEQ ID NO: 1 or 21 comprising any one, two, three, four, five, or more mutations selected from R106Y, R108Y, R133Y, R135Y, and R136Y, or any combination thereof. In some embodiments, the recombinant polypeptide is or comprises a variant of SEQ ID NO: 1 or 21 comprising any one, two, three, four, five, or more mutations selected from R106G, R108G, R133G, R135G, and R136G, or any combination thereof. In some embodiments, the recombinant polypeptide is or comprises a variant of SEQ ID NO: 1 or 21 comprising any one, two, three, four, five, or more mutations selected from R106S, R108S, R133S, R135S, and R136S, or any combination thereof.

In some embodiments, the recombinant polypeptide is or comprises the sequence as set forth in SEQ ID NO: 6 or 26. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 6 or 26, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 6 or 26 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 6 or 26 comprising any one, two, three, four, or five mutations selected from R106A, R108A, R133A, R135A, and R136A, or any combination thereof. In some embodiments, the recombinant polypeptide is or comprises a variant of SEQ ID NO: 6 or 261 comprising any one, two, three, four, or five mutations selected from R106T, R108T, R133T, R135T, and R136T, or any combination thereof. In some embodiments, the recombinant polypeptide is or comprises a variant of SEQ ID NO: 6 or 26 comprising any one, two, three, four, or five mutations selected from R106Y, R108Y, R133Y, R135Y, and R136Y, or any combination thereof. In some embodiments, the recombinant polypeptide is or comprises a variant of SEQ ID NO: 6 or 26 comprising any one, two, three, four, or five mutations selected from R106G, R108G, R133G, R135G, and R136, or any combination thereof. In some embodiments, the recombinant polypeptide is or comprises a variant of SEQ ID NO: 6 or 26 comprising any one, two, three, four, or five mutations selected from R106S, R108S, R133S, R135S, and R136S, or any combination thereof.

In some embodiments, the recombinant polypeptide is or comprises the sequence as set forth in SEQ ID NO: 2 or 22. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 2 or 22, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 2 or 22 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 2 or 22 comprising any one, two, three, four, or five mutations selected from R106C, R108C, R133C, R135C, and R136C, or any combination thereof.

In some embodiments, the recombinant polypeptide is or comprises the sequence as set forth in SEQ ID NO: 3 or 23. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 3 or 23, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 3 or 23 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 3 or 23 comprising any one, two, three, four, or more mutations selected from R108C, R133C, R135C, and R136C, or any combination thereof.

In some embodiments, the recombinant polypeptide is or comprises the sequence as set forth in SEQ ID NO: 4 or 24. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 4, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 4 or 24 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 4 or 24 comprising any one, two, or three mutations selected from R133C, R135C, and R136C, or any combination thereof.

In some embodiments, the recombinant polypeptide is or comprises the sequence as set forth in SEQ ID NO: 5 or 25. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 5 or 25, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 5 or 25 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 5 or 25 comprising any one or two mutations selected from R135C and R136C.

In some embodiments, the recombinant polypeptide is or comprises the sequence as set forth in SEQ ID NO: 7 or 27. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 6, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 7 or 27 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 7 or 27 comprising any one, two, three, four, or five mutations selected from R106C, R108C, R133C, R135C, and R136C, or any combination thereof.

In some embodiments, the recombinant polypeptide is or comprises the sequence as set forth in SEQ ID NO: 8 or 28. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 8 or 28, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 8 or 28 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 8 or 28 comprising any one, two, three, or four mutations selected from R108C, R133C, R135C, and R136C, or any combination thereof.

In some embodiments, the recombinant polypeptide is or comprises the sequence as set forth in SEQ ID NO: 9 or 29. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 8, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 9 or 29 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 9 or 29 comprising any one, two, or three mutations selected from R133C, R135C, and R136C, or any combination thereof.

In some embodiments, the recombinant polypeptide is or comprises the sequence as set forth in SEQ ID NO: 10 or 30. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 9, including a sequence comprising a substitution, deletion, and/or insertion at one or more amino acid positions such as 102, 106, 107, 108, 109, 131, 132, 133, 134, 135, 136, 137, 138, 139, 140, 141, 142, 143, 144, 145, 146, 147, 148, 149, 150, 151, 152, 153, 154, 155, 161, 215, 218, 379, or 447 (amino acid positions relative to SEQ ID NO: 67, 68, 69, or 70), or any combination thereof. In some embodiments, the recombinant polypeptide is or comprises a variant of SEQ ID NO: 10 or 30 comprising any one, two, three, four, five, or more mutations selected from P102A, R109A, R136A, E161P, E218A, S215P, I379A, M447V, R106C, R108C, R133C, R135C, R136C, R106A, R108A, R133A, R135A, R136A, R106T, R108T, R133T, R135T, R136T, R106Y, R108Y, R133Y, R135Y, R136Y, R106G, R108G, R133G, R135G, R136G, R106S, R108S, R133S, R135S, and R136S, or any combination thereof. In some embodiments, the recombinant polypeptide is or comprises a variant of SEQ ID NO: 10 or 30 comprising any one or two mutations selected from R135C and R136C.

In some embodiments, the recombinant polypeptide is or comprises the sequence as set forth in any one of SEQ ID NOs: 1-10, 21-30, 41-48, and 54-61. In some embodiments, the recombinant polypeptide is or comprises an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 1-10, 21-30, 41-48, and 54-61.

As indicated above, in some embodiments, the recombinant polypeptides provided herein not only associate to form a trimer, but can also aggregate or be aggregated to produce a protein comprising a plurality of recombinant polypeptides. In some embodiments, the protein formed has a macrostructure. In some cases, the macrostructure may confer structural stability to the RSV viral antigen or immunogen recombinant polypeptide, which in turn can afford access to potential antigenic sites capable of promoting an immune response.

In some embodiments, the trimerized recombinant polypeptides aggregate to form a protein comprising a plurality of trimerized recombinant polypeptides. In some embodiments, the plurality of trimerized recombinant polypeptides forms a protein having a macrostructure. In some embodiments, the protein comprises a rosette-like oligomer comprising an F protein peptide trimer as a crutch-shaped rod.

In some embodiments, provided herein is a complex comprising a recombinant polypeptide selected from SEQ ID NOs: 1-10, 21-30, 41-48, and 54-61, or a fragment, variant, or mutant thereof, in any suitable combination. In some embodiments, provided herein is a complex comprising a trimer of a recombinant polypeptide selected from SEQ ID NOs: 1-10, 21-30, 41-48, and 54-61, or a fragment, variant, or mutant thereof, wherein the recombinant polypeptides trimerize via inter-polypeptide disulfide bonds to form the trimer.

In some embodiments, the protein described herein comprising a plurality of recombinant polypeptides is an immunogen. In some embodiments, the protein described herein comprising a plurality of recombinant polypeptides is comprised in a nanoparticle. For example, in some embodiments, the protein is linked directly to a nanoparticle, e.g., a protein nanoparticle. In some embodiments, the protein is linked indirectly to a nanoparticle. In some embodiments, the protein described herein comprising a plurality of recombinant polypeptides is comprised in a virus-like particle (VLP).

### 2. Polynucleotides and vectors

Also provided are polynucleotides (nucleic acid molecules) encoding the RSV antigens or immunogens and recombinant polypeptides provided herein, and vectors for genetically engineering cells to express such RSV antigens or immunogens and recombinant polypeptides.

In some embodiments, provided is a polynucleotide encoding the recombinant polypeptide provided herein. In some aspects, the polynucleotide comprises a single nucleic acid sequence, such as a nucleic acid sequence encoding a recombinant polypeptide. In other cases, the polynucleotide comprises a first nucleic acid sequence encoding a recombinant polypeptide comprising a particular RSV viral antigen or immunogen and a second nucleic acid sequence encoding a recombinant polypeptide comprising a different RSV viral antigen or immunogen.

In some embodiments, the polynucleotide encoding the recombinant polypeptide comprises at least one promoter operably linked to control the expression of the recombinant polypeptide. In some embodiments, the polynucleotide comprises two, three, or more promoters operably linked to control the expression of the recombinant polypeptide.

In some embodiments, for example, when the polynucleotide comprises two or more nucleic acid coding sequences, such as sequences encoding recombinant polypeptides comprising different RSV viral antigens or immunogens, at least one promoter is operably linked to control the expression of the two or more nucleic acid sequences. In some embodiments, the polynucleotide comprises two, three, or more promoters operably linked to control the expression of the recombinant polypeptide.

In some embodiments, the expression of the recombinant polypeptide is inducible or conditional. Thus, in some aspects, the polynucleotide encoding the recombinant polypeptide comprises a conditional promoter, enhancer, or transactivator. In some such aspects, the conditional promoter, enhancer, or transactivator is an inducible promoter, enhancer, or transactivator, or a repressible promoter, enhancer, or transactivator. For example, in some embodiments, an inducible or conditional promoter can be used to restrict expression of the recombinant polypeptide to a specific microenvironment. In some embodiments, the expression driven by the inducible or conditional promoter is regulated by exposure to an exogenous agent, such as heat, radiation, or drug.

In cases where the polynucleotide comprises more than one nucleic acid sequence encoding a recombinant polypeptide, the polynucleotide may further comprise a nucleic acid sequence encoding a peptide between the one or more nucleic acid sequences. In some cases, the nucleic acid positioned between the nucleic acid sequences encodes a peptide that separates the translation products of the nucleic acid sequences during or after translation. In some embodiments, the peptide comprises an internal ribosome entry site (IRES), a self-cleaving peptide, or a peptide that causes ribosome skipping, such as a T2A peptide.

In some embodiments, the polynucleotide encoding the recombinant polypeptide is introduced into a composition comprising cultured cells (e.g., host cells), such as by retroviral transduction, transfection, or transformation. In some embodiments, this can allow for the expression (e.g., production) of the recombinant polypeptide. In some embodiments, the expressed recombinant polypeptide is purified.

In some embodiments, the polynucleotide (nucleic acid molecule) provided herein encodes an RSV viral antigen or immunogen as described herein. In some embodiments, the polynucleotide (nucleic acid molecule) provided herein encodes a recombinant polypeptide comprising an RSV viral antigen or immunogen, e.g., an RSV F peptide protein described herein.

Also provided is a vector or construct comprising the nucleic acid molecule as described herein. In some embodiments, the vector or construct comprises one or more promoters operably linked to the nucleic acid molecule encoding the recombinant polypeptide to drive its expression. In some embodiments, the promoter is operably linked to one or more than one nucleic acid molecule, e.g., nucleic acid molecules encoding recombinant polypeptides comprising different RSV viral antigens or immunogens.

In some embodiments, the vector is a viral vector. In some embodiments, the viral vector is a retroviral vector. In some embodiments, the retroviral vector is a lentiviral vector. In some embodiments, the retroviral vector is a gammaretroviral vector.

In some embodiments, the vector or construct comprises a single promoter that drives the expression of one or more nucleic acid molecules of the polynucleotide. In some embodiments, such promoters can be multicistronic (bicistronic or tricistronic, see e.g., U.S. Patent No. 6,060,273). For example, in some embodiments, the transcription unit can be engineered as a bicistronic unit comprising an IRES (internal ribosome entry site), which allows for the co-expression of gene products (e.g., those encoding different recombinant polypeptides) by a message from a single promoter. In some embodiments, the vector provided herein is bicistronic, allowing the vector to comprise and express two nucleic acid sequences. In some embodiments, the vector provided herein is tricistronic, allowing the vector to comprise and express three nucleic acid sequences.

In some embodiments, a single promoter directs the expression of an RNA that comprises, in a single open reading frame (ORF), two or three genes (e.g., those encoding a chimeric signaling receptor and encoding a recombinant receptor) separated from one another by sequences encoding a self-cleavage peptide (e.g., a 2A sequence) or a protease recognition site (e.g., furin). The ORF thus encodes a single polypeptide, which, either during (in the case of 2A) or after translation, is processed into individual proteins. In some cases, the peptide, such as T2A, may cause the ribosome to skip (ribosome skipping) the synthesis of a peptide bond at the C-terminus of a 2A element, leading to the separation of the end of the 2A sequence from the next peptide downstream (see, e.g., de Felipe. Genetic Vaccines and Ther. 2: 13 (2004); and de Felipe et al., Traffic 5: 616-626 (2004)). Many 2A elements are known in the art. Examples of 2A sequences that can be used in the methods and nucleic acids disclosed herein include, but are not limited to, 2A sequences from the foot-and-mouth disease virus (F2A), equine rhinitis A virus (E2A), Thosea asigna virus (T2A), and porcine teschovirus-1 (P2A) as described in U.S. Patent Publication No. 20070116690.

In some embodiments, the vector is comprised in a virus. In some embodiments, the virus is a pseudovirus. In some embodiments, the virus is a viral-like particle. In some embodiments, the vector is comprised in a cell. In some embodiments, the virus or cell in which the vector is comprised comprises a recombinant genome.

### III. Immunogenic Compositions and Formulations

In some embodiments, provided herein is an immunogenic composition comprising a trimer of recombinant polypeptides comprising a sequence selected from SEQ ID NOs: 1-10, 21-30, 41-48, and 54-61, or a combination of any two or more of the trimers. In some embodiments, a unit dose of the immunogenic composition may comprise from about 10 µg to about 100 µg of the RSV F antigen, preferably from about 25 µg to about 75 µg of the RSV F antigen, preferably from about 40 µg to about 60 µg of the RSV F antigen, or about 50 µg of the RSV F antigen. In some embodiments, the dose comprises 3 µg of the RSV F antigen. In other embodiments, the dose comprises 9 µg of the RSV F antigen. In other embodiments, the dose comprises 30 µg of the RSV F antigen.

In some cases, it may be desirable to combine the disclosed immunogen with other pharmaceutical products (e.g., vaccines) which induce a protective response to other agents. For example, a composition comprising the recombinant RSV F antigen described herein, e.g., a trimer or protein, can be administered simultaneously (typically separately) or sequentially with other vaccines recommended by the Advisory Committee on Immunization Practices (ACIP; cdc.gov/vaccines/acip/index.html) for the targeted age group (e.g., infants from approximately one to six months of age), such as an influenza vaccine or a varicella zoster vaccine. Thus, the disclosed immunogen including the recombinant RSV F antigen described herein may be administered simultaneously or sequentially with vaccines against, for example, hepatitis B (HepB), diphtheria, tetanus and pertussis (DTaP), pneumococci (PCV), Haemophilus influenzae type b (Hib), polio, influenza, and rotavirus.

Multivalent or combined vaccines provide protection against multiple pathogens. In certain cases, multivalent vaccines can provide protection against multiple strains of the same pathogen. In certain cases, multivalent vaccines provide protection against multiple pathogens, such as the combined vaccine Tdap, which provides protection against strains of tetanus, pertussis, and diphtheria. Multivalent vaccines are highly desirable to minimize the number of immunizations required to provide protection against multiple pathogens or pathogenic strains, to reduce administration costs, and to increase coverage rates. This may be particularly useful, for example, when vaccinating babies or children.

In some embodiments, the vaccine comprising the immunogenic composition described herein, for example, is a multivalent vaccine (e.g., a bivalent vaccine). In some embodiments, the antigenic materials for incorporation into the multivalent vaccine composition of the present invention are derived from RSV type A or type B, or a combination thereof. The antigens for incorporation into the multivalent vaccine composition of the present invention may be derived from one strain of RSV or multiple strains, for example, two to five strains, in order to provide a broader spectrum of protection. In one embodiment, the antigens for incorporation into the multivalent vaccine composition of the present invention are derived from multiple strains of RSV virus. Other useful antigens include live viruses, attenuated viruses, and inactivated viruses, such as inactivated poliovirus (Jiang et al., J. Biol. Stand., (1986) 14: 103-9), attenuated strains of hepatitis A virus (Bradley et al., J. Med. Virol., (1984) 14: 373-86), attenuated measles virus (James et al., N. Engl. J. Med., (1995) 332: 1262-6), and epitopes of pertussis virus (e.g., ACEL-IMUNErM acellular DTP, Wyeth-Lederle Vaccines and Pediatrics).

In some aspects, the vaccine provided herein is a universal vaccine. In some embodiments, a universal vaccine is a vaccine which provides protection against multiple strains of the same virus, such as multiple strains of RSV. The development of an effective universal RSV vaccine will reduce costs and labor, e.g., with seasonal vaccine formulation, and allow for more robust pandemic preparedness.

In some aspects, a universal vaccine is a vaccine comprising multiple epitopes derived from distinct viral strains. In some aspects, a universal vaccine comprises a single epitope that is conserved across distinct viral strains. For example, a universal vaccine can be based on the relatively conserved domain of the RSV F protein.

In some embodiments, the antigenic materials for incorporation into the multivalent vaccine composition of the present invention are derived from RSV type A and type B, with a weight ratio of 0.1-20, such as 2 : 1, 1.5 : 1, 1 : 1, 1 : 1.5, or 1 : 2, in the combination. In some embodiments, the antigenic material derived from RSV type A is a recombinant polypeptide or trimer thereof, which is formed by linking any one of the RSV A F protein peptides described herein (e.g., an RSV A virus antigen or immunogen, e.g., see Section I) to a C-terminal propeptide. In some embodiments, the RSV A F protein peptide comprises an F1 subunit and an F2 subunit of the RSV A F protein, or fragments thereof, optionally wherein the F1 subunit and the F2 subunit are linked by a disulfide bond or an artificially introduced linker. In some embodiments, the antigenic material derived from RSV type B is a recombinant polypeptide or trimer thereof, which is formed by linking any one of the RSV B F protein peptides described herein (e.g., an RSV virus antigen or immunogen, e.g., see Section I) to a C-terminal propeptide. In some embodiments, the RSV B F protein peptide comprises an F1 subunit and an F2 subunit of the RSV B F protein, or fragments thereof, optionally wherein the F1 subunit and the F2 subunit are linked by a disulfide bond or an artificially introduced linker. In some embodiments, the RSV type A is the RSV A2 strain. In some embodiments, the RSV type B is the Australian RSV B strain. In some embodiments, the artificially introduced linker is a non-amino acid compound or one or more amino acids. In some embodiments, the one or more amino acids are independently selected from CGGG (SEQ ID NO: 138). In some embodiments, the artificially introduced linker replaces any one, two, or three of furin site I, furin site II, and pep27 peptide. In some embodiments, the artificially introduced linker replaces furin site I and pep27 peptide and one or more bases linked to furin site II. In some embodiments, the one or more bases are selected from FLGFLLGV (SEQ ID NO: 126), e.g., F, FL, FLG, FLGF (SEQ ID NO: 127), FLGFL (SEQ ID NO: 128), FLGFLL (SEQ ID NO: 129), FLGFLLG (SEQ ID NO: 130), or FLGFLLGV (SEQ ID NO: 131). In some embodiments, the antigenic material derived from RSV type A is or comprises the sequence as set forth in any one of SEQ ID NOs: 1-20, or an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to any one of SEQ ID NOs: 1-20. In some embodiments, the antigenic material derived from RSV type A is or comprises the sequence as set forth in any one of SEQ ID NOs: 41-50, or an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to any one of SEQ ID NOs: 41-50. In some embodiments, the antigenic material derived from RSV type B is or comprises the sequence as set forth in any one of SEQ ID NOs: 21-40, or an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to any one of SEQ ID NOs: 21-40. In some embodiments, the antigenic material derived from RSV type A is or comprises the sequence as set forth in any one of SEQ ID NOs: 54-63, or an amino acid sequence having at least or about 80%, 81%, 82%, 83%, 84%, 85%, 86%, 87%, 88%, 89%, 90%, 91%, 92%, 93%, 94%, 95%, 96%, 97%, 98%, or 99% sequence identity to any one of SEQ ID NOs: 54-63. Also provided is an immunogenic composition comprising the disclosed immunogen (e.g., the disclosed recombinant RSV F antigen or a nucleic acid molecule encoding a protomer of the disclosed recombinant RSV F antigen) and a pharmaceutically acceptable carrier. In some embodiments, the immunogenic composition comprises the trimerized recombinant polypeptide provided herein and optionally a pharmaceutically acceptable carrier. In some embodiments, the immunogenic composition comprises a protein comprising a plurality of trimerized recombinant polypeptides provided herein and optionally a pharmaceutically acceptable carrier. In some embodiments, the immunogenic composition comprises the protein nanoparticle provided herein and optionally a pharmaceutically acceptable carrier. In some embodiments, the immunogenic composition comprises the VLP provided herein and optionally a pharmaceutically acceptable carrier. In some embodiments, the immunogenic composition comprises the isolated nucleic acid provided herein and optionally a pharmaceutically acceptable carrier. In some embodiments, the immunogenic composition comprises the vector provided herein and optionally a pharmaceutically acceptable carrier. In some embodiments, the immunogenic composition comprises the virus provided herein and optionally a pharmaceutically acceptable carrier. In some embodiments, the immunogenic composition comprises the pseudovirus provided herein and optionally a pharmaceutically acceptable carrier. In some embodiments, the immunogenic composition comprises the cell provided herein and optionally a pharmaceutically acceptable carrier. In some embodiments, the immunogenic composition, such as the immunogenic composition described herein, is a vaccine. In some embodiments, the vaccine is a prophylactic vaccine. In some embodiments, the vaccine is a therapeutic vaccine. In some embodiments, the vaccine is both a prophylactic vaccine and a therapeutic vaccine. Such pharmaceutical compositions can be administered to a subject by a variety of administration modes known to the person of ordinary skill in the art, for example, intramuscular, intradermal, subcutaneous, intravenous, intraarterial, intraarticular, intraperitoneal, intranasal, sublingual, tonsillar, oropharyngeal, or other parenteral and mucosal routes. In several embodiments, the pharmaceutical composition comprising one or more of the disclosed immunogens is an immunogenic composition. Actual methods for preparing administrable compositions will be known or apparent to those skilled in the art, and are described in more detail in such publications as Remingtons Pharmaceutical Sciences, 19th Ed., Mack Publishing Company, Easton, Pa., 1995.

Thus, an immunogen, e.g., a recombinant RSV F antigen, e.g., a trimer or protein described herein can be formulated with a pharmaceutically acceptable carrier to help retain the biological activity while also helping to increase stability during storage within an acceptable temperature range. Potential carriers include, but are not limited to, physiologically balanced culture medium, phosphate buffer saline solution, water, emulsions (e.g., oil/water or water/oil emulsions), various types of wetting agents, cryoprotective additives or stabilizers such as proteins, peptides or hydrolysates (e.g., albumin and gelatin), sugars (e.g., sucrose, lactose, and sorbitol), amino acids (e.g., sodium glutamate), or other protective agents. The resulting aqueous solution may be packaged for use as is or lyophilized. The lyophilized formulation is combined with a sterile solution prior to administration for either single or multiple administrations.

A formulated composition, especially a liquid formulation, may comprise a bacteriostat to prevent or minimize degradation during storage, including but not limited to effective concentrations (typically 1% w/v) of benzyl alcohol, phenol, m-cresol, chlorobutanol, methylparaben, and/or propylparaben. A bacteriostat may be contraindicated for some patients; therefore, a lyophilized formulation may be reconstituted in a solution with or without such a component.

The immunogenic composition of the present invention can comprise a pharmaceutically acceptable vehicle substance as required to approximate physiological conditions, such as pH adjusting and buffering agents, tonicity adjusting agents, wetting agents, and the like, for example, sodium acetate, sodium lactate, sodium chloride, potassium chloride, calcium chloride, sorbitan monolaurate, and triethanolamine oleate. The immunogenic composition may optionally comprise an adjuvant to enhance the immune response of the host. Suitable adjuvants are, for example, toll-like receptor agonists, alum, AlPO₄, alhydrogel, lipid A and derivatives or variants thereof, oil emulsions, saponins, neutral liposomes, liposomes containing the vaccine and cytokines, non-ionic block copolymers, and chemokines. Non-ionic block polymers containing polyoxyethylene (POE) and polyoxypropylene (POP), such as POE-POP-POE block copolymers, MPL^{™} (3-O-deacylated monophosphoryl lipid A; Corixa, Hamilton, Ind.) and IL-12 (Genetics Institute, Cambridge, Mass.), and many other suitable adjuvants well known in the art, may be used as an adjuvant (Newman et al., 1998, Critical Reviews in Therapeutic Drug Carrier Systems 15: 89-142). These adjuvants have the advantage in that they help to stimulate the immune system in a non-specific way, thus enhancing the immune response against a pharmaceutical product. In some embodiments, the immunogenic composition of the present invention may comprise or be administered with more than one adjuvant. In some embodiments, the immunogenic composition of the present invention may comprise or be administered with two adjuvants. In some embodiments, the immunogenic composition of the present invention may comprise or be administered with a plurality of adjuvants. For example, in some cases, a vaccine, e.g., a vaccine comprising the immunogenic composition provided herein, may comprise or be administered in combination with a plurality of adjuvants.

For vaccine compositions, examples of suitable adjuvants include, for example, aluminum hydroxide, lecithin, Freund's adjuvant, MPL^{™}, and IL-12. In some embodiments, the vaccine composition (e.g., the RSV vaccine composition) or nanoparticle immunogen disclosed herein can be formulated as a controlled-release or time-release formulation. This can be achieved in a composition that comprises a slow-release polymer or via a microencapsulated delivery system or bioadhesive gel. Various pharmaceutical compositions can be prepared in accordance with standard procedures well known in the art.

In some embodiments, the immunogenic composition of the present invention can comprise an adjuvant formulation comprising a metabolizable oil (e.g., squalene) and α-tocopherol and polyoxyethylene sorbitan monooleate (Tween-80) in the form of an oil-in-water emulsion. In some embodiments, the adjuvant formulation can comprise from about 2% to about 10% squalene, from about 2% to about 10% α-tocopherol (e.g., D-α-tocopherol), and from about 0.3% to about 3% polyoxyethylene sorbitan monooleate. In some embodiments, the adjuvant formulation can comprise about 5% squalene, about 5% tocopherol, and about 0.4% polyoxyethylene sorbitan monooleate. In some embodiments, the immunogenic composition of the present invention can comprise 3-O-deacylated monophosphoryl lipid A (3D-MPL), and an adjuvant in the form of an oil-in-water emulsion, which adjuvant comprises a metabolizable oil, α-tocopherol, and polyoxyethylene sorbitan monooleate. In some embodiments, the immunogenic composition of the present invention can comprise QS21 (extract of Quillaja saponaria Molina: fraction 21), 3D-MPL, and an oil-in-water emulsion, wherein the oil-in-water emulsion comprises a metabolizable oil, α-tocopherol, and polyoxyethylene sorbitan monooleate. In some embodiments, the immunogenic composition of the present invention can comprise QS21, 3D-MPL, and an oil-in-water emulsion, wherein the oil-in-water emulsion has the following composition: a metabolizable oil, such as squalene, α-tocopherol, and Tween-80. In some embodiments, the immunogenic composition of the present invention can comprise an adjuvant in the form of a liposome composition.

In some embodiments, the immunogenic composition of the present invention can comprise an adjuvant formulation comprising a metabolizable oil (e.g., squalene), polyoxyethylene sorbitan monooleate (Tween-80), and Span 85. In some embodiments, the adjuvant formulation can comprise about 5% (w/v) squalene, about 0.5% (w/v) polyoxyethylene sorbitan monooleate, and about 0.5% (w/v) Span 85.

In some embodiments, the immunogenic composition of the present invention can comprise an adjuvant formulation comprising quillaja saponin, cholesterol, and phospholipid, e.g., in the form of a nanoparticle composition. In some embodiments, the immunogenic composition of the present invention can comprise a mixture of separately purified fractions of *Quillaja saponaria Molina*, which are subsequently formulated with cholesterol and phospholipid.

In some embodiments, the immunogenic composition of the present invention can comprise an adjuvant selected from MF59^{™}, Matrix-A^{™}, Matrix-C^{™}, Matrix-M^{™}, AS01, AS02, AS03, and AS04.

In some embodiments, the immunogenic composition of the present invention can comprise a toll-like receptor 9 (TLR9) agonist, wherein the TLR9 agonist is an oligonucleotide of from 8 to 35 nucleotides in length comprising an unmethylated cytidine-phospho-guanosine (also referred to as CpG or cytosine-phosphate-guanosine) motif, and the RSV antigen and the oligonucleotide are present in the immunogenic composition in amounts effective to stimulate an immune response against the RSV antigen in a mammalian subject, such as a human subject in need thereof. TLR9 (CD289) recognizes unmethylated cytidine-phospho-guanosine (CpG) motifs found in microbial DNA, which can be mimicked using synthetic CpG-containing oligodeoxynucleotides (CpG-ODNs). CpG-ODNs are known to enhance antibody production and to stimulate T helper 1 (Th1) cell responses (Coffman et al., Immunity, 33: 492-503, 2010). Optimal oligonucleotide TLR9 agonists often comprise a palindromic sequence following the general formula of 5'-purine-purine-CG-pyrimidine-pyrimidine-3', or 5'-purine-purine-CG-pyrimidine-pyrimidine-CG-3'. See U.S. Patent No. 6,589,940, which is incorporated herein by reference in its entirety. In some embodiments, the CpG oligonucleotide is linear. In other embodiments, the CpG oligonucleotide is circular or comprises a hairpin loop. The CpG oligonucleotide may be single-stranded or double-stranded. In some embodiments, the CpG oligonucleotide may comprise a modification. Modifications include, but are not limited to, modifications of the 3' OH or 5' OH group, modifications of the nucleotide bases, modifications of the sugar component, and modifications of the phosphate group. Modified bases may be comprised in the palindromic sequence of the CpG oligonucleotide as long as the modified bases maintain the same specificity for its natural complement through Watson-Crick base pairing (e.g., the palindromic portion is still self-complementary). In some embodiments, the CpG oligonucleotide comprises a non-canonical base. In some embodiments, the CpG oligonucleotide comprises a modified nucleoside. In some embodiments, the modified nucleoside is selected from 2'-deoxy-7-deazaguanosine, 2'-deoxy-6-thioguanosine, arabinoguanosine, 2'-deoxy-2' substituted-arabinoguanosine, and 2'-O-substituted-arabinoguanosine. The CpG oligonucleotide may comprise a modification of the phosphate group. For example, in addition to phosphodiester linkages, phosphate modifications include, but are not limited to, methyl phosphonate, phosphorothioate, phosphoramidate (bridging or non-bridging), phosphotriester, and phosphorodithioate, and may be used in any combination. Other non-phosphate linkages may also be used. In some embodiments, the oligonucleotide comprises only a phosphorothioate backbone. In some embodiments, the oligonucleotide comprises only a phosphodiester backbone. In some embodiments, the oligonucleotide comprises a combination of phosphate linkages in the phosphate backbone, such as a combination of phosphodiester and phosphorothioate linkages. Oligonucleotides with phosphorothioate backbones may be more immunogenic than those with phosphodiester backbones and appear to be more resistant to degradation after injection into the host (Braun et al., J Immunol, 141: 2084-2089, 1988; and Latimer et al., Mol Immunol, 32: 1057-1064, 1995). The CpG oligonucleotide of the present invention comprises at least one, two, or three inter-nucleotide phosphorothioate linkages. In some embodiments, when a plurality of CpG oligonucleotide molecules are present in a pharmaceutical composition comprising at least one excipient, both stereoisomers of the phosphorothioate linkage are present in the plurality of CpG oligonucleotide molecules. In some embodiments, all of the inter-nucleotide linkages of the CpG oligonucleotide are phosphorothioate linkages, or in other words, the CpG oligonucleotide has a phosphorothioate backbone.

Any suitable CpG oligodeoxynucleotide (ODN) or a combination thereof can be used as an adjuvant in the present invention. For instance, K-type ODNs (also referred to as B type) encode multiple CpG motifs on a phosphorothioate backbone. K-type ODNs may be based on the following sequence: TCC**ATGGA**CG**TTCCT**GAGCGTT (SEQ ID NO: 132). The use of phosphorothioate nucleotides enhances resistance to nuclease digestion when compared with native phosphodiester nucleotides, resulting in a substantially longer in vivo half-life. K-type ODNs trigger pDCs to differentiate and produce TNF-α, and B cells to proliferate and secrete IgM. D-type ODNs (also referred to as A type) are constructed from a mixed phosphodiester/phosphorothioate backbone, comprise a single CpG motif flanked by palindromic sequences, and have polyG tails at the 3' and 5' ends (a structural motif that facilitates the formation of concatamers). D-type ODNs may be based on the following sequence: GGTGCATCGATGCAGGGGGG (SEQ ID NO: 133). D-type ODNs trigger pDCs to mature and secrete IFN-α, but have no effect on B cells. C-type ODNs resemble K-type in being composed entirely of phosphorothioate nucleotides, but resemble D-type in containing palindromic CpG motifs. C-type ODNs may be based on the following sequence: TCG**T**CG**TT**CG**AA**CG**A**CGTTGAT (SEQ ID NO: 134). Such ODNs stimulate B cells to secrete IL-6 and pDCs to produce IFN-α. P-type ODNs comprise two palindromic sequences, enabling them to form a higher ordered structure. P-type ODNs may be based on the following sequence: **T**CG**T**CG**A**CG**AT**C**GG**CGCGCG**C**CG (SEQ ID NO: 135). P-type ODNs activate B cells and pDCs, and induce substantially greater IFN-α production when compared with C-type ODNs. In this paragraph, bold letters in ODN sequences indicate self-complementary palindromic sequences, and CpG motifs are underlined.

Exemplary CpG ODNs, e.g., CpG 7909 (5'-TCGTCGTTTTGTCGTTTTGTCGTT-3') (SEQ ID NO: 136) and CpG 1018 (5'-TGACTGTGAACGTTCGAGATGA-3') (SEQ ID NO: 137), are known and disclosed in US Patent Nos. 7,255,868, 7,491,706, 7,479,285, 7,745,598, 7,785,610, 8,003,115, 8,133,874, 8,114,418, 8,222,398, 8,333,980, 8,597,665, 8,669,237, 9,028,845, and 10,052,378; application publication US 2020/0002704; and Bode et al., "CpG DNA as a vaccine adjuvant", Expert Rev Vaccines (2011), 10(4): 499-511, all of which are incorporated herein by reference in their entireties for all purposes.

One or more adjuvants may be used in combination, and may include, but are not limited to, aluminum hydroxide (aluminum salt), oil-in-water emulsions, water-in-oil emulsions, liposomes, and microparticles, such as poly(lactide-co-glycolide) microparticles (Shah et al., Methods Mol, 1494: 1-14, 2017). In some embodiments, the immunogenic composition further comprises an aluminum salt adjuvant to which the RSV antigen is adsorbed. In some embodiments, the aluminum salt adjuvant comprises one or more of amorphous aluminum hydroxyphosphate sulfate, aluminum hydroxide, aluminum phosphate, and potassium aluminum sulfate. In some embodiments, the aluminum salt adjuvant comprises one or both of aluminum hydroxide and aluminum phosphate. In some embodiments, the aluminum salt adjuvant comprises aluminum hydroxide. In some embodiments, a unit dose of the immunogenic composition comprises from about 0.25 to about 0.50 mg Al³⁺, or about 0.35 mg Al³⁺. In some embodiments, the immunogenic composition further comprises other adjuvants. Other suitable adjuvants include, but are not limited to, squalene-in-water emulsions (e.g., MF59 or AS03/CAS-1), TLR3 agonists (e.g., poly-IC or poly-ICLC), TLR4 agonists (e.g., bacterial lipopolysaccharide derivatives such as monophosphoryl lipid A (MPL), and/or saponins such as Quil A or QS-21, as in AS01 or AS02), TLR5 agonists (bacterial flagellin), and TLR7, TLR8, and/or TLR9 agonists (imidazoquinoline derivatives such as imiquimod and resiquimod) (Coffman et al., Immunity, 33: 492-503, 2010). In some embodiments, other adjuvants comprise MPL and alum (e.g., AS04). For veterinary use and for the production of antibodies in non-human animals, mitogenic components of Freund's adjuvant (both complete and incomplete) can be used. CAS-1 is α-tocopherol, squalene, and polysorbate 80 in an oil-in-water emulsion.

In some embodiments, the immunogenic composition comprises a pharmaceutically acceptable excipient, including, for example, solvents, bulking agents, buffering agents, tonicity adjusting agents, and preservatives (Pramanick et al., Pharma Times, 45: 65-77, 2013). In some embodiments, the immunogenic composition may comprise an excipient that functions as one or more of a solvent, a bulking agent, a buffering agent, and a tonicity adjusting agent (e.g., sodium chloride in saline may serve as both an aqueous vehicle and a tonicity adjusting agent).

In some embodiments, the immunogenic composition comprises an aqueous vehicle as a solvent. Suitable vehicles include, for example, sterile water, saline solution, phosphate buffered saline, and Ringer's solution. In some embodiments, the composition is isotonic.

The immunogenic composition may comprise a buffering agent. Buffering agents control pH to inhibit degradation of the active agent during processing, storage, and optionally reconstitution. Suitable buffering agents include, for example, salts, including acetate, citrate, phosphate, or sulfate. Other suitable buffering agents include, for example, amino acids such as arginine, glycine, histidine, and lysine. The buffering agent may further comprise hydrochloric acid or sodium hydroxide. In some embodiments, the buffering agent maintains the pH of the composition within a range of 6 to 9. In some embodiments, the pH is greater than (lower limit) 6, 7, or 8. In some embodiments, the pH is less than (upper limit) 9, 8, or 7. That is, the pH is in the range of from about 6 to 9 in which the lower limit is less than the upper limit.

The immunogenic composition may comprise a tonicity adjusting agent. Suitable tonicity adjusting agents include, for example, dextrose, glycerol, sodium chloride, glycerin, and mannitol.

The immunogenic composition may comprise a bulking agent. A bulking agent is particularly useful when the pharmaceutical composition is to be lyophilized before administration. In some embodiments, the bulking agent is a protectant that aids in the stabilization and prevention of degradation of the active agent during freeze or spray drying and/or during storage. Suitable bulking agents are sugars (mono-, di- and polysaccharides), such as sucrose, lactose, trehalose, mannitol, sorbital, glucose, and raffinose.

The immunogenic composition may comprise a preservative. Suitable preservatives include, for example, antioxidants and antimicrobial agents. However, in preferred embodiments, the immunogenic composition is prepared under sterile conditions and is in a single-use container, and thus does not necessitate inclusion of a preservative.

In some embodiments, the composition can be provided as a sterile composition. The pharmaceutical composition typically comprises an effective amount of the disclosed immunogen, and can be prepared by conventional methods. Typically, the amount of the immunogen in each dose of the immunogenic composition is selected as an amount which induces an immune response without significant, adverse side effects. In some embodiments, the composition can be provided in unit dosage form for use to induce an immune response in a subject. A unit dosage form comprises a suitable single preselected dose for administration to a subject, or suitable marked or measured multiples of two or more preselected unit doses, and/or a metering mechanism for administering the unit dose or multiples thereof. In other embodiments, the composition further comprises an adjuvant.

### IV. Methods of Inducing an Immune Response

In some embodiments, provided herein is a method for generating an immune response against an RSV surface antigen in a subject, comprising administering to the subject an effective amount of any one of the recombinant polypeptides or trimers thereof, nucleic acids, vectors, host cells, immunogenic compositions, or vaccine compositions described herein. In some embodiments, provided herein is a method for generating an immune response against an RSV surface antigen in a subject, comprising administering to the subject an effective amount of a complex comprising a recombinant polypeptide selected from SEQ ID NOs: 1-10, 21-30, 41-48, and 54-61. In some embodiments, provided herein is a method for generating an immune response against an RSV surface antigen in a subject, wherein the surface antigen comprises an F protein or an antigenic fragment thereof, and the method comprises administering to the subject an effective amount of a complex comprising a recombinant polypeptide selected from SEQ ID NOs: 1-10, 21-30, 41-48, and 54-61. In some embodiments, provided herein is a method for generating an immune response against an RSV surface antigen in a subject, wherein the surface antigen comprises a sequence selected from SEQ ID NOs: 11-20, 31-40, 49-53, and 62-102, and the method comprises administering to the subject an effective amount of a complex comprising a recombinant polypeptide selected from SEQ ID NOs: 1-10, 21-30, 41-48, and 54-61. In some embodiments, provided herein is a method for generating an immune response against an RSV surface antigen in a subject, wherein the surface antigen comprises an RSV F protein or an antigenic fragment thereof and optionally the surface antigen comprises a sequence of any one or more of SEQ ID NOs: 11-20, 31-40, 49-53, and 62-102, or an antigenic fragment thereof, and the method comprises administering to the subject an effective amount of a complex comprising a recombinant polypeptide comprising the sequence as set forth in any one of SEQ ID NOs: 1-10, 21-30, 41-48, and 54-61.

In some embodiments, provided herein is a method for generating an immune response against an RSV surface antigen in a subject, wherein the surface antigen comprises an F protein or an antigenic fragment thereof, and the method comprises administering to the subject an effective amount of a complex comprising a recombinant polypeptide comprising a sequence selected from SEQ ID NOs: 1-10, 21-30, 41-48, and 54-61, or a combination of any two or more of the complexes.

The disclosed immunogen (e.g., a recombinant RSV F antigen, e.g., a trimer or protein described herein, a nucleic acid molecule (such as an RNA molecule) or vector encoding a protomer of the disclosed recombinant RSV F antigen, or a protein nanoparticle or virus-like particle comprising the disclosed recombinant RSV F antigen) can be administered to a subject to induce an immune response against the corresponding RSV F antigen in the subject. In a particular example, the subject is a human. The immune response can be a protective immune response, e.g., a response that inhibits subsequent infection with the corresponding RSV. Induction of the immune response can also be used to treat or inhibit an infection and disease associated with the corresponding RSV.

In some embodiments, a subject can be selected for treatment who has already been infected with RSV, or is at risk of infection with RSV, for example because of exposure or potential exposure to RSV. Following administration of the disclosed immunogen, the subject can be monitored for infection or a symptom associated with RSV, or both.

Typical subjects intended for treatment with the therapeutic agent and method of the present invention include humans, as well as non-human primates and other animals. To identify a subject for prophylaxis or treatment according to the method of the present invention, accepted screening methods are employed to determine risk factors associated with a targeted or suspected disease or condition, or to determine the status of an existing disease or condition in a subject. These screening methods include, for example, conventional work-ups to determine environmental, familial, occupational, and other such risk factors that may be associated with the targeted or suspected disease or condition, as well as diagnostic methods, such as various ELISAs and other immunoassay methods to detect and/or characterize RSV infection. These and other conventional methods allow the clinician to select a patient in need of therapy using the method and pharmaceutical composition of the present invention. In accordance with these methods and principles, the composition can be administered according to the teachings herein, or other conventional methods, as an independent prophylaxis or treatment regimen, or as a follow-up, adjunct, or coordinate treatment regimen to other treatments.

Administration of the disclosed immunogen, e.g., the RSV F antigen, e.g., a trimer or protein, can be for prophylactic or therapeutic purpose. When provided prophylactically, the disclosed therapeutic agent is provided prior to any symptom, e.g., prior to infection. The prophylactic administration of the disclosed therapeutic agent serves to prevent or ameliorate any subsequent infection. When provided therapeutically, the disclosed therapeutic agent is provided at or after the onset of a symptom of a disease or infection, e.g., after development of a symptom of RSV infection corresponding to the RSV F antigen, or after diagnosis with RSV infection. The therapeutic agent can thus be provided prior to the anticipated exposure to RSV so as to attenuate the anticipated severity, duration, or extent of infection and/or an associated disease symptom, after exposure or suspected exposure to the virus, or after the actual initiation of infection.

The immunogen and immunogenic composition thereof described herein are provided to a subject, preferably a human, in an amount effective to induce or enhance an immune response against the RSV F antigen in the subject. The actual dose of the disclosed immunogen will vary depending on factors such as the disease indication and particular status of the subject (e.g., the subject's age, size, fitness, extent of symptoms, susceptibility factors, and the like), time and route of administration, other drugs or treatments being administered concurrently, as well as the specific pharmacology of the composition for eliciting the desired activity or biological response in the subject. Dosage regimens can be adjusted to provide an optimum prophylactic or therapeutic response.

An immunogenic composition comprising one or more of the disclosed immunogens can be used in a coordinate (or prime-boost) vaccination protocol or combinatorial formulation. In certain embodiments, novel combinatorial immunogenic compositions and coordinate immunization protocols employ separate immunogens or formulations, each directed toward eliciting an anti-viral immune response, such as an immune response against the RSV F antigen. Separate immunogenic compositions that elicit the anti-viral immune response can be combined in a polyvalent immunogenic composition administered to a subject in a single immunization step, or they can be administered separately (in monovalent immunogenic compositions) in a coordinate (or prime-boost) immunization protocol.

There can be several boosts, and each boost can be a different disclosed immunogen. In certain examples, the boost may be the same immunogen as another boost, or the prime. The prime and boost can be administered as a single dose or multiple doses, for example, two doses, three doses, four doses, five doses, six doses, or more can be administered to a subject over days, weeks, or months. There can also be multiple boosts, e.g., 1 to 5 (e.g., 1, 2, 3, 4, or 5 boosts), or more. Different doses can be used in a series of sequential immunizations. For example, a relatively large dose is used in a primary immunization, and then a relatively small dose is used in the boost.

In certain embodiments, the boost can be administered about 2 weeks, about 3 to 8 weeks, or about 4 weeks following the prime, or about several months after the prime. In certain embodiments, the boost can be administered about 5, about 6, about 7, about 8, about 10, about 12, about 18, or about 24 months following the prime, or more or less time after the prime. Periodic additional boosts can also be used at appropriate time points to enhance the subject's "immune memory". The adequacy of the vaccination parameters chosen, e.g., formulation, dose, regimen, and the like, can be determined by taking aliquots of serum from the subject and assaying antibody titers during the immunization program. In addition, the clinical condition of the subject can be monitored for the desired effect, e.g., prevention of infection or improvement in disease state (e.g., reduction in viral load). If such monitoring indicates that vaccination is suboptimal, the subject can be boosted with an additional dose of the immunogenic composition, and the vaccination parameters can be modified in a fashion expected to enhance the immune response.

In certain embodiments, the prime-boost method can comprise providing a DNA-primer and protein-boost vaccination protocol to a subject. The method can comprise two or more administrations of the nucleic acid molecule or protein.

For protein therapeutics, typically, each human dose comprises 1-1000 µg of protein, such as from about 1 µg to about 100 µg, for example, from about 1 µg to about 50 µg, such as about 1 µg, about 2 µg, about 5 µg, about 10 µg, about 15 µg, about 20 µg, about 25 µg, about 30 µg, about 40 µg, or about 50 µg.

The amount utilized in an immunogenic composition is selected based on the subject population (e.g., infants or elderly). The optimal amount for a particular composition can be determined by standard studies involving observation of antibody titers and other responses in a subject. It is understood that a therapeutically effective amount of the disclosed immunogen, such as the disclosed recombinant RSV F antigen, e.g., a trimer or protein, viral vector, or nucleic acid molecule in an immunogenic composition can include an amount that is ineffective at eliciting an immune response by administration of a single dose, but that is effective upon administration of multiple doses, for example in a prime-boost administration protocol.

Upon administration of the disclosed immunogen of the present invention, the immune system of the subject typically responds to the immunogenic composition by producing antibodies specific for the RSV F protein peptide comprised in the immunogen. Such a response indicates that an immunologically effective dose is delivered to the subject.

In some embodiments, the subject's antibody response is determined in the context of evaluating an effective dose/immunization protocol. In most cases, it is sufficient to evaluate the antibody titers in serum or plasma obtained from the subject. Decisions as to whether to administer booster vaccinations and/or to change the amount of the therapeutic agent administered to the individual may be at least partially based on the antibody titer level. The antibody titer level can be based on, for example, an immunobinding assay which measures the concentration of antibodies in the serum which bind to an antigen including, for example, the recombinant RSV F antigen, e.g., a trimer or protein.

The method is considered effective even if it does not completely eliminate or reduce or prevent RSV infection. For example, elicitation of an immune response against RSV with one or more of the disclosed immunogens can reduce or inhibit RSV infection by a desired amount, for example, by at least 10%, at least 20%, at least 50%, at least 60%, at least 70%, at least 80%, at least 90%, at least 95%, at least 98%, or even at least 100% (elimination or prevention of detectable infected cells), as compared to RSV infection in the absence of the immunogen. In other embodiments, RSV replication can be reduced or inhibited by the disclosed method. The method is considered effective even if it does not completely eliminate RSV replication. For example, the immune response elicited using one or more of the disclosed immunogens can reduce replication of the corresponding RSV by a desired amount, for example, by at least 10%, at least 20%, at least 50%, at least 60%, at least 70%, at least 80%, at least 90%, at least 95%, at least 98%, or even at least 100% (elimination or prevention of detectable RSV replication), as compared to RSV replication in the absence of the immunogen.

In some embodiments, the disclosed immunogen is administered to the subject simultaneously with administration of the adjuvant. In other embodiments, the disclosed immunogen is administered to the subject after administration of the adjuvant and within a sufficient amount of time to induce the immune response.

One approach to administration of nucleic acids is direct immunization with plasmid DNA, such as with a mammalian expression plasmid. Immunization with nucleic acid constructs is well known in the art and taught, for example, in U.S. Pat. No. 5,643,578 (which describes methods for immunizing vertebrates by introducing DNA encoding a desired antigen to elicit a cell-mediated or a humoral response), and U.S. Pat. Nos. 5,593,972 and 5,817,637 (which describe operably linking a nucleic acid sequence encoding an antigen to regulatory sequences enabling its expression). U.S. Pat. No. 5,880,103 describes several methods for delivering nucleic acids encoding immunogenic peptides or other antigens to an organism. The methods include liposomal delivery of the nucleic acids (or of the synthetic peptides themselves), and immunostimulatory constructs, or ISCOMS^{™}, a negatively charged cage-like structure of 30-40 nm in size formed spontaneously after mixing cholesterol and Quil A^{™} (saponin). Protective immunity has been generated in a variety of experimental models of infection, including toxoplasmosis and Epstein-Barr virus-induced tumors, using ISCOMS^{™} as the delivery vehicle for antigens (Mowat and Donachie, Immunol. Today 12: 383, 1991). Doses of antigen as low as 1 µg encapsulated in ISCOMS^{™} have been found to produce Class I mediated CTL responses (Takahashi et al., Nature 344: 873, 1990).

In some embodiments, a plasmid DNA vaccine is used to express the disclosed immunogen in a subject. For example, a nucleic acid molecule encoding the disclosed immunogen can be administered to a subject to induce an immune response against the RSV F antigen. In some embodiments, the nucleic acid molecule can be comprised on a plasmid vector for DNA immunization, such as the pVRC8400 vector, as described in Barouch et al., J. Virol, 79, 8828-8834, 2005, which is incorporated by reference herein.

In another approach of using nucleic acids for immunization, the disclosed recombinant RSV F antigen, e.g., a trimer or protein, can be expressed by attenuated viral hosts or vectors or bacterial vectors. Recombinant vaccinia virus, adeno-associated virus (AAV), herpes virus, retrovirus, cytomegalovirus, or other viral vectors can be used to express the peptide or protein, inducing a CTL response. Vaccinia vectors and methods useful in immunization protocols are described in, e.g., U.S. Pat. No. 4,722,848. BCG (Bacillus Calmette Guerin) provides another vector for expression of peptides (see Stover, Nature 351: 456-460, 1991).

In one embodiment, a nucleic acid encoding the disclosed recombinant RSV F antigen is introduced directly into the cell. For example, the nucleic acid can be loaded onto gold microspheres by standard methods and introduced into the skin by a device such as Bio-Rad's HELIOS^{™} Gene Gun. The nucleic acids can be "naked", consisting of plasmids under control of a strong promoter. Typically, the DNA is injected into muscle, although it can also be injected directly into other sites. The injected dose is usually from about 0.5 µg/kg to about 50 mg/kg, and typically is about 0.005 mg/kg to about 5 mg/kg (see, e.g., U.S. Pat. No. 5,589,466).

For example, the nucleic acid can be loaded onto gold microspheres by standard methods and introduced into the skin by a device such as Bio-Rad's HELIOS^{™} Gene Gun. The nucleic acids can be "naked", consisting of plasmids under control of a strong promoter. Typically, the DNA is injected into muscle, although it can also be injected directly into other sites. The injected dose is usually from about 0.5 µg/kg to about 50 mg/kg, and typically is about 0.005 mg/kg to about 5 mg/kg (see, e.g., U.S. Pat. No. 5,589,466).

In another embodiment, an mRNA-based immunization protocol can be used to deliver a nucleic acid encoding the disclosed recombinant RSV F antigen directly into the cell. In some embodiments, nucleic acid-based vaccines based on mRNA may provide a potent alternative to the previously mentioned approaches. mRNA vaccines eliminate safety concerns about DNA integration into the host genome, and can be directly translated in the host cell cytoplasm. Moreover, the simple cell-free, in vitro synthesis of RNA avoids the manufacturing complications associated with viral vectors. Two exemplary forms of RNA-based vaccination that can be used to deliver a nucleic acid encoding the disclosed recombinant RSV F antigen include conventional non-amplifying mRNA immunization (see, e.g., Petsch et al., "Protective efficacy of in vitro synthesized, specific mRNA vaccines against influenza A virus infection", Nature biotechnology, 30(12): 1210-6, 2012) and self-amplifying mRNA immunization (see, e.g., Geall et al., "Nonviral delivery of self-amplifying RNA vaccines", PNAS, 109(36): 14604-14609, 2012; Magini et al., "Self-Amplifying mRNA Vaccines Expressing Multiple Conserved Influenza Antigens Confer Protection against Homologous and Heterosubtypic Viral Challenge", PLoS One, 11(8): e0161193, 2016; and Brito et al., "Self-amplifying mRNA vaccines", Adv Genet., 89: 179-233, 2015).

In some embodiments, administration of a therapeutically effective amount of one or more of the disclosed immunogens to a subject induces a neutralizing immune response in the subject. To evaluate neutralization activity, following immunization of a subject, serum can be collected from the subject at appropriate time points, frozen, and stored for neutralization testing. Methods for assaying neutralization activity are known to the person of ordinary skill in the art and are further described herein, including, but not limited to, plaque reduction neutralization (PRNT) assays, microneutralization assays, flow cytometry-based assays, and single-cycle infection assays. In some embodiments, the serum neutralization activity can be assayed using a panel of RSV pseudoviruses.

In some embodiments, administration of a therapeutically effective amount of one or more of the disclosed immunogens to a subject induces a neutralizing immune response in the subject. To evaluate neutralization activity, following immunization of a subject, serum can be collected from the subject at appropriate time points, frozen, and stored for neutralization testing. Methods for assaying neutralization activity are known to the person of ordinary skill in the art and are further described herein, including, but not limited to, plaque reduction neutralization (PRNT) assays, microneutralization assays, flow cytometry-based assays, and single-cycle infection assays. In some embodiments, the serum neutralization activity can be assayed using a panel of RSV pseudoviruses.

In some embodiments, a neutralizing immune response induced by the disclosed immunogen herein produces neutralizing antibodies against RSV. In some embodiments, the neutralizing antibodies herein bind to a cellular receptor or co-receptor of RSV or a component thereof. Nucleolin is an entry co-receptor for RSV and also mediates the cellular entry of influenza viruses, parainfluenza viruses, some enteroviruses, and bacteria that cause tularaemia. Binding of the pre-fusion RSV-F glycoprotein to the insulin-like growth factor-1 receptor (IGF1R) may also trigger the activation of protein kinase Cζ (PKCζ), recruiting nucleolin from the nuclei of cells to the plasma membrane to bind to RSV-F on virions. In some embodiments, the viral receptor or co-receptor is a paramyxovirus receptor or co-receptor, preferably a pneumonia virus receptor or co-receptor, more preferably a human RSV receptor or co-receptor. For example, CCR1, CCR2, CCR3, CCR4, CCR5, and/or CCR8 receptors may be involved in human RSV infection. RhoA is another example of a host cell RSV receptor or co-receptor. In some embodiments, the neutralizing antibodies herein modulate, decrease, antagonize, mitigate, block, inhibit, abrogate, and/or interfere with at least one RSV activity or binding, or with RSV receptor activity or binding, in vitro, in situ, and/or in vivo, such as RSV release, RSV receptor signaling, membrane RSV cleavage, RSV activity, RSV production, and/or synthesis. In some embodiments, the immunogen disclosed herein induces neutralizing antibodies against RSV that modulate, decrease, antagonize, mitigate, block, inhibit, abrogate, and/or interfere with the binding of RSV to an RSV receptor or co-receptor, such as nucleolin, IGF1R, CCR1, CCR2, CCR3, CCR4, CCR5, CCR8, and/or RhoA.

### V. Articles of Manufacture or Kits

Also provided is an article of manufacture or kit comprising the provided recombinant polypeptide, protein, and immunogenic composition. The article of manufacture may comprise a container and a label or package insert on or associated with the container. Suitable containers include, for example, bottles, vials, syringes, test tubes, IV solution bags, etc. The container may be formed from a variety of materials such as glass or plastic. In some embodiments, the container has a sterile access port. Exemplary containers include intravenous solution bags and vials, including those with a stopper pierceable by a needle for injection. The article of manufacture or kit may further comprise a package insert indicating that the compositions can be used to treat a particular condition such as a condition described herein (e.g., RSV infection). Alternatively, or additionally, the article of manufacture or kit may further comprise another or the same container comprising a pharmaceutically acceptable buffer. It may further comprise other materials such as other buffers, diluents, filters, needles, and/or syringes.

The label or package insert may indicate that the composition is used for treating RSV infection in an individual. The label or package insert, which is on or associated with the container, may indicate instructions for reconstitution and/or use of the formulation. The label or package insert may further indicate that the formulation is useful or intended for subcutaneous, intravenous, or other modes of administration for treating or preventing RSV infection in an individual.

In some embodiments, the container contains the composition, alone or in combination with another composition effective for treating, preventing, and/or diagnosing the condition. The article of manufacture or kit may comprise (a) a first container with a composition contained therein (i.e., a first medicament), wherein the composition comprises the immunogenic composition or protein or recombinant polypeptide thereof; and (b) a second container with a composition contained therein (i.e., a second medicament), wherein the composition comprises a further agent, such as an adjuvant or another therapeutic agent, and the article of manufacture or kit further comprises instructions on the label or package insert for treating the subject with the second medicament in an effective amount.

### Terminology

Unless defined otherwise, all terms of art, notations, and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polypeptide" and "protein" are used interchangeably to refer to a polymer of amino acid residues, and are not limited to a minimum length. Polypeptides, including the provided receptors and other polypeptides, e.g., linkers or peptides, may comprise amino acid residues including natural and/or non-natural amino acid residues. The terms also include post-translational modifications of the polypeptide, for example, glycosylation, sialylation, acetylation, and phosphorylation. In some aspects, the polypeptide may comprise modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

The term "F0" refers to the precursor polypeptide of the RSV F protein, which is a single polypeptide consisting of a signal polypeptide sequence, an F1 polypeptide sequence, a pep27 polypeptide sequence, and an F2 polypeptide sequence. Typically, the F0 polypeptide of RSV consists of 574 amino acids.

The term "F1" refers to the polypeptide chain of the mature RSV F protein. The native F1 comprises approximately residues 137-574 of the RSV F0 precursor and consists of (from the N-terminus to the C-terminus) an extracellular region (approximately residues 137-524), a transmembrane domain (approximately residues 525-550), and a cytoplasmic domain (approximately residues 551-574). The term encompasses both the native F1 polypeptide and F1 polypeptides from the native sequence that comprise modifications (e.g., amino acid substitutions, insertions, or deletions), such as modifications designed to stabilize or enhance the immunogenicity of F mutants.

The term "F2" refers to the polypeptide chain of the mature RSV F protein. The native F2 comprises approximately residues 26-109 of the RSV F0 precursor. The term encompasses both the native F2 polypeptide and F2 polypeptides from the native sequence that comprise modifications (e.g., amino acid substitutions, insertions, or deletions), such as modifications designed to stabilize or enhance the immunogenicity of F mutants. In the native RSV F protein, the F2 polypeptide is linked to the F1 polypeptide by two disulfide bonds to form an F2-F1 heterodimer.

The term "F protein" or "F peptide" refers to a polypeptide or protein having all or part of the amino acid sequence of an RSV F protein (e.g., F0, F1, and/or F2), including the native F protein or variants thereof. The term "recombinant F protein" or "recombinant F peptide" refers to a polypeptide or protein that has one or more amino acid insertions, substitutions, or deletions relative to the native RSV F protein (e.g., F0, F1, and/or F2). In this context, a fragment of an F protein or F peptide may comprise one or more or all epitopes of the native RSV F protein.

The term "multimerization domain" refers to an amino acid sequence that is capable of forming a multimer, e.g., a trimer. An example of such multimerization domains is the collagen C-terminal propeptide.

The term "pep27" refers to a 27-amino acid polypeptide that is cleaved from the F0 precursor during the maturation of the RSV F protein. The sequence of pep27 is flanked by two furin cleavage sites that are cleaved by cellular proteases during F protein maturation to produce F1 and F2 polypeptides.

The term "immunogenicity" refers to the ability of a substance to provoke, elicit, stimulate, or induce an immune response against a specific antigen in an animal in the presence or absence of an adjuvant.

The term "mutation" refers to the deletion, addition, or substitution of amino acid residues in the amino acid sequence of a protein or polypeptide as compared to the amino acid sequence of a reference protein or polypeptide.

The term "soluble" refers to a protein that is soluble in an aqueous liquid and remains soluble.

The term "sequence identity" is used to describe the relatedness between two amino acid sequences or between two nucleotide sequences. "Sequence identity" refers to the value of percent sequence identity determined by optimally aligning two sequences (i.e., maximizing sequence identity) over a comparison window, where the two sequences being aligned can be optimally aligned by the addition or deletion (i.e., gaps) of residues. Percent sequence identity can be calculated by determining the number of positions at which the identical nucleic acid base or amino acid residue occurs in both sequences in the optimal alignment mode to yield the number of matched positions, dividing the number of matched positions by the total number of positions compared, and multiplying the result by 100 to yield the percent sequence identity. Sequence identity between two amino acid sequences can be calculated using available local alignment tools (e.g., BLAST) or global alignment tools (e.g., those implementing the Needleman-Wunsch algorithm).

In this context, reference to an amino acid position refers to a position in a query amino acid sequence that corresponds to a particular position in a reference amino acid sequence when the query amino acid sequence is optimally aligned with the reference amino acid sequence. In the present invention, the positions of amino acids in the described RSV A F protein peptides are determined based on the amino acid sequences as set forth in the reference amino acid sequence SEQ ID NO: 67 or 69, and the positions of amino acids in the described RSV B F protein peptides are determined based on the amino acid sequences as set forth in the reference amino acid sequence SEQ ID NO: 68 or 70.

As used herein, a "subject" is a mammal, such as a human or other animal, and typically is human. In some embodiments, the subject, e.g., patient, to which one or more agents, cells, cell populations, or compositions are administered, is a mammal, typically a primate, such as a human. In some embodiments, the primate is a monkey or an ape. The subject can be male or female, and can be any suitable age, including infant, juvenile, adolescent, adult, and geriatric subjects. In some embodiments, the subject is a non-primate mammal, such as a rodent.

As used herein, "treatment" (and grammatical variations thereof) refers to complete or partial amelioration or reduction of a disease or condition or disorder, or a symptom, adverse effect or outcome, or phenotype associated therewith. Desirable effects of treatment include, but are not limited to, preventing occurrence or recurrence of the disease, alleviation of symptoms, diminishment of any direct or indirect pathological consequences of the disease, preventing metastasis, decreasing the rate of disease progression, amelioration or palliation of the disease state, and remission or improved prognosis. The term does not imply a complete cure of a disease or the complete elimination of any symptom, or effectiveness for all symptoms or consequences.

As used herein, "delaying development of a disease" means to defer, hinder, slow, retard, stabilize, inhibit, and/or postpone development of the disease (such as cancer). This delay may be of varying lengths of time, depending on the history of the disease and/or individual being treated. In some embodiments, a sufficient or significant delay can, in effect, encompass prevention, in that the individual does not develop the disease. For example, a late-stage cancer, such as development of metastasis, may be delayed.

As used herein, "preventing" includes providing prophylaxis with respect to the occurrence or recurrence of a disease in a subject that may be predisposed to the disease but has not yet been diagnosed with the disease. In some embodiments, the provided cell and composition are used to delay development of a disease or to slow the progression of a disease.

As used herein, to "inhibit" a function or activity is to reduce the function or activity when compared to otherwise same conditions except for a condition or parameter of interest, or alternatively, as compared to another condition. For example, cells that inhibit tumor growth reduce the rate of tumor growth as compared to that in the absence of the cells.

An "effective amount" of an agent, e.g., a pharmaceutical formulation, cell, or composition, in the context of administration, refers to an amount effective, at doses/amounts and for periods of time necessary, to achieve a desired result, such as a therapeutic or prophylactic result.

A "therapeutically effective amount" of an agent, e.g., a pharmaceutical formulation, cell, or composition, refers to an amount effective, at doses and for periods of time necessary, to achieve a desired therapeutic result, such as the treatment of a disease, condition, or disorder, and/or the pharmacokinetic or pharmacodynamic effect of the treatment. The therapeutically effective amount may vary depending on factors such as the disease state, age, sex, and weight of the subject, and the population of cells administered. In some embodiments, the provided method comprises administering the cell and/or composition at an effective amount, e.g., a therapeutically effective amount.

A "prophylactically effective amount" refers to an amount effective, at doses and for periods of time necessary, to achieve a desired prophylactic result. Typically but not necessarily, since a prophylactic dose is used in the subject prior to or at an earlier stage of disease, the prophylactically effective amount is less than the therapeutically effective amount. In the context of lower tumor burden, the prophylactically effective amount in some aspects may be higher than the therapeutically effective amount.

As used herein, the term "about" refers to the usual error range for the respective value readily known to the skilled person in this technical field. Reference to "about" a value or parameter herein includes (and describes) embodiments that are directed to that value or parameter per se.

As used herein, the singular forms "a", "an", and "the" include plural referents unless the context clearly dictates otherwise. For example, "a" or "an" means "at least one" or "one or more".

Throughout the present invention, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it should be understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, non-aqueous, or any combination thereof.

As used herein, the term "vector" refers to a nucleic acid molecule capable of propagating another nucleic acid to which it has been linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### Exemplary Embodiments

Embodiment 1. A protein comprising a plurality of recombinant polypeptides, each recombinant polypeptide comprising a respiratory syncytial virus (RSV) F protein peptide, or a fragment or epitope thereof linked to a collagen C-terminal propeptide, wherein the C-terminal propeptides of the recombinant polypeptides form inter-polypeptide disulfide bonds.

Embodiment 2. The protein according to embodiment 1, wherein the RSV is of subtype A and/or subtype B.

Embodiment 3. The protein according to embodiment 1 or 2, wherein the epitope is a linear epitope or a conformational epitope.

Embodiment 4. The protein according to any one of embodiments 1 to 3, wherein the F protein peptide comprises an F1 subunit peptide and an F2 subunit peptide, and the protein comprises three recombinant polypeptides.

Embodiment 5. The protein according to any one of embodiments 1 to 4, wherein the F protein peptide comprises a signal peptide, a heptad-repeat C (HRC) peptide, a pep27 peptide, a fusion peptide (FP), a heptad-repeat A (HRA) peptide, a Domain I peptide, a Domain II peptide, or a heptad-repeat B (HRB) peptide, or any combination thereof.

Embodiment 6. The protein according to any one of embodiments 1 to 5, wherein the F protein peptide comprises an F1 subunit but not an F2 subunit of the F protein, or vice versa.

Embodiment 7. The protein according to any one of embodiments 1 to 6, wherein the F protein peptide comprises an F1 subunit and an F2 subunit of the F protein, optionally without pep27, optionally wherein the F1 subunit and the F2 subunit are linked by a disulfide bond or an artificially introduced linker.

Embodiment 8. The protein according to any one of embodiments 1 to 7, wherein the F protein peptide does not comprise a transmembrane (TM) domain peptide and/or a cytoplasmic (CP) domain peptide.

Embodiment 9. The protein according to any one of embodiments 1 to 8, wherein the F protein peptide comprises a protease cleavage site, wherein the protease is optionally furin, trypsin, factor Xa, or cathepsin L.

Embodiment 10. The protein according to any one of embodiments 1 to 8, wherein the F protein peptide does not comprise a protease cleavage site, wherein the protease is optionally furin, trypsin, factor Xa, or cathepsin L.

Embodiment 11. The protein according to any one of embodiments 1 to 10, wherein the F protein peptide is soluble, or does not bind directly to a lipid bilayer, e.g., a membrane or viral envelope.

Embodiment 12. The protein according to any one of embodiments 1 to 11, wherein the F protein peptides are the same or different among the recombinant polypeptides of the protein.

Embodiment 13. The protein according to any one of embodiments 1 to 12, wherein the F protein peptide is fused directly to the C-terminal propeptide, or is linked to the C-terminal propeptide via a linker, such as a linker comprising glycine-X-Y repeats, wherein X and Y are independently any amino acid, optionally proline or hydroxyproline.

Embodiment 14. The protein according to any one of embodiments 1 to 13, which is soluble, or does not bind directly to a lipid bilayer, e.g., a membrane or viral envelope.

Embodiment 15. The protein according to any one of embodiments 1 to 14, wherein the protein is capable of forming a rosette-like oligomer comprising an F protein peptide trimer.

Embodiment 16. The protein according to any one of embodiments 1 to 15, wherein the protein is capable of binding to a cell surface attachment factor or receptor of a subject, optionally wherein the subject is a mammal, such as a primate, e.g., human.

Embodiment 17. The protein according to any one of embodiments 1 to 16, wherein the C-terminal propeptide is of human collagen.

Embodiment 18. The protein according to any one of embodiments 1 to 17, wherein the C-terminal propeptide comprises a C-terminal polypeptide of proα1(I), proα1(II), proα1(III), proα1(V), proα1(XI), proα2(I), proα2(V), proα2(XI), or proα3(XI), or a fragment thereof.

Embodiment 19. The protein according to any one of embodiments 1 to 18, wherein the C-terminal propeptides are the same or different among the recombinant polypeptides.

Embodiment 20. The protein according to any one of embodiments 1 to 19, wherein the C-terminal propeptide comprises SEQ ID NO: 103, or an amino acid sequence having at least 90% identity thereto, capable of forming inter-polypeptide disulfide bonds and trimerizing the recombinant polypeptides.

Embodiment 21. The protein according to any one of embodiments 1 to 19, wherein the C-terminal propeptide comprises SEQ ID NO: 104, or an amino acid sequence having at least 90% identity thereto, capable of forming inter-polypeptide disulfide bonds and trimerizing the recombinant polypeptides.

Embodiment 22. The protein according to any one of embodiments 1 to 19, wherein the C-terminal propeptide comprises SEQ ID NO: 105, or an amino acid sequence having at least 90% identity thereto, capable of forming inter-polypeptide disulfide bonds and trimerizing the recombinant polypeptides.

Embodiment 23. The protein according to any one of embodiments 1 to 19, wherein the C-terminal propeptide comprises SEQ ID NO: 106, or an amino acid sequence having at least 90% identity thereto, capable of forming inter-polypeptide disulfide bonds and trimerizing the recombinant polypeptides.

Embodiment 24. The protein according to any one of embodiments 1 to 19, wherein the C-terminal propeptide comprises SEQ ID NO: 107, or an amino acid sequence having at least 90% identity thereto, capable of forming inter-polypeptide disulfide bonds and trimerizing the recombinant polypeptides.

Embodiment 25. The protein according to any one of embodiments 1 to 19, wherein the C-terminal propeptide comprises SEQ ID NO: 108, or an amino acid sequence having at least 90% identity thereto, capable of forming inter-polypeptide disulfide bonds and trimerizing the recombinant polypeptides.

Embodiment 26. The protein according to any one of embodiments 1 to 19, wherein the C-terminal propeptide comprises SEQ ID NO: 109, or an amino acid sequence having at least 90% identity thereto, capable of forming inter-polypeptide disulfide bonds and trimerizing the recombinant polypeptides.

Embodiment 27. The protein according to any one of embodiments 1 to 19, wherein the C-terminal propeptide comprises any one of SEQ ID NOs: 110-114, or an amino acid sequence having at least 90% identity thereto, capable of forming inter-polypeptide disulfide bonds and trimerizing the recombinant polypeptides.

Embodiment 28. The protein according to any one of embodiments 1 to 19, wherein the C-terminal propeptide comprises SEQ ID NO: 115, or an amino acid sequence having at least 90% identity thereto, capable of forming inter-polypeptide disulfide bonds and trimerizing the recombinant polypeptides.

Embodiment 29. The protein according to any one of embodiments 1 to 19, wherein the C-terminal propeptide comprises any one of SEQ ID NOs: 116-118, or an amino acid sequence having at least 90% identity thereto, capable of forming inter-polypeptide disulfide bonds and trimerizing the recombinant polypeptides.

Embodiment 30. The protein according to any one of embodiments 1 to 29, wherein the C-terminal propeptide comprises an amino acid sequence comprising glycine-X-Y repeats linked to the N-terminus of any one of SEQ ID NOs: 103-118, wherein X and Y are independently any amino acid, optionally proline or hydroxyproline, or an amino acid sequence having at least 90% identity thereto, capable of forming inter-polypeptide disulfide bonds and trimerizing the recombinant polypeptides.

Embodiment 31. The protein according to any one of embodiments 1 to 30, wherein the F protein peptide in each recombinant polypeptide is in a pre-fusion conformation or a post-fusion conformation, optionally wherein the protein comprises a rosette-like oligomer comprising an F protein peptide trimer as a crutch-shaped rod.

Embodiment 32. The protein according to any one of embodiments 1 to 31, wherein the F protein peptide in each recombinant polypeptide comprises any one of SEQ ID NOs: 11-20, 21-40, 49-53, and 62-66, or an amino acid sequence having at least 80% identity thereto.

Embodiment 33. The protein according to any one of embodiments 1 to 31, wherein the recombinant polypeptide comprises any one of SEQ ID NOs: 16-20, 26-30, 50, 52, 53, 63, 65, and 66, or an amino acid sequence having at least 80% identity thereto.

Embodiment 34. An immunogen comprising the protein according to any one of embodiments 1 to 33.

Embodiment 35. A protein nanoparticle comprising the protein according to any one of embodiments 1 to 33 linked directly or indirectly to the nanoparticle.

Embodiment 36. A virus-like particle (VLP) comprising the protein according to any one of embodiments 1 to 33.

Embodiment 37. An isolated nucleic acid encoding one, two, three, or more recombinant polypeptides of the protein according to any one of embodiments 1 to 33.

Embodiment 38. The isolated nucleic acid according to embodiment 37, wherein a polypeptide encoding the F protein peptide is fused in-frame to a polypeptide encoding the collagen C-terminal propeptide.

Embodiment 39. The isolated nucleic acid according to embodiment 37 or 38, which is operably linked to a promoter.

Embodiment 40. The isolated nucleic acid according to any one of embodiments 37 to 39, which is a DNA molecule.

Embodiment 41. The isolated nucleic acid according to any one of embodiments 37 to 39, which is an RNA molecule, optionally an mRNA molecule, such as a nucleoside-modified mRNA, a non-amplifying mRNA, a self-amplifying mRNA, or a trans-amplifying mRNA.

Embodiment 42. A vector comprising the isolated nucleic acid according to any one of embodiments 37 to 41.

Embodiment 43. The vector according to embodiment 42, which is a viral vector.

Embodiment 44. A virus, pseudovirus, or cell comprising the vector according to embodiment 42 or 43, optionally wherein the virus or cell has a recombinant genome.

Embodiment 45. An immunogenic composition comprising the protein, immunogen, protein nanoparticle, VLP, isolated nucleic acid, vector, virus, pseudovirus, or cell according to any one of embodiments 1 to 44, and a pharmaceutically acceptable carrier.

Embodiment 46. A vaccine comprising the immunogenic composition according to embodiment 45 and optionally an adjuvant, wherein the vaccine is optionally a subunit vaccine, and/or optionally wherein the vaccine is a prophylactic and/or therapeutic vaccine.

Embodiment 47. The vaccine according to embodiment 46, wherein the vaccine comprises a plurality of different adjuvants.

Embodiment 48. A method for producing a protein, comprising: expressing the isolated nucleic acid or vector according to any one of embodiments 37 to 43 in a host cell to produce the protein according to any one of embodiments 1 to 33; and purifying the protein.

Embodiment 49. A protein produced by the method according to embodiment 48.

Embodiment 50. A method for generating an immune response against an RSV F protein peptide, or a fragment or epitope thereof in a subject, comprising administering to the subject an effective amount of the protein, immunogen, protein nanoparticle, VLP, isolated nucleic acid, vector, virus, pseudovirus, cell, immunogenic composition, or vaccine according to any one of embodiments 1 to 47 and 49 to generate the immune response.

Embodiment 51. The method according to embodiment 50, which is used for treating or preventing RSV infection.

Embodiment 52. The method according to embodiment 50 or 51, wherein the immune response generated inhibits or reduces the replication of RSV in the subject.

Embodiment 53. The method according to any one of embodiments 50 to 52, wherein the immune response comprises a cell-mediated response and/or a humoral response, and optionally comprises the production of one or more neutralizing antibodies, such as polyclonal antibodies or monoclonal antibodies.

Embodiment 54. The method according to any one of embodiments 50 to 53, wherein the immune response is directed against the RSV F protein peptide, or a fragment or epitope thereof, but not against the C-terminal propeptide.

Embodiment 55. The method according to any one of embodiments 50 to 54, wherein the administration does not result in antibody-dependent enhancement (ADE) in the subject due to prior exposure to one or more strains of RSV.

Embodiment 56. The method according to any one of embodiments 50 to 55, wherein the administration does not result in antibody-dependent enhancement (ADE) in the subject when subsequently exposed to one or more strains of RSV.

Embodiment 57. The method according to any one of embodiments 50 to 56, further comprising a priming step and/or a boosting step.

Embodiment 58. The method according to any one of embodiments 50 to 57, wherein the administering step is performed via topical, transdermal, subcutaneous, intradermal, oral, intranasal (e.g., intranasal spray), intratracheal, sublingual, buccal, rectal, vaginal, inhaled, intravenous (e.g., intravenous injection), intraarterial, intramuscular (e.g., intramuscular injection), intracardiac, intraosseous, intraperitoneal, transmucosal, intravitreal, subretinal, intraarticular, periarticular, local, or transepidermal administration.

Embodiment 59. The method according to any one of embodiments 50 to 58, wherein the effective amount is administered in a single dose or in a series of doses separated by one or more intervals.

Embodiment 60. The method according to any one of embodiments 50 to 59, wherein the effective amount is administered without an adjuvant.

Embodiment 61. The method according to any one of embodiments 50 to 59, wherein the effective amount is administered with an adjuvant.

Embodiment 62. A method comprising administering to a subject an effective amount of the protein according to any one of embodiments 1 to 33 to produce in the subject neutralizing antibodies or neutralizing antisera against RSV.

Embodiment 63. The method according to embodiment 62, wherein the subject is a mammal, optionally a human or a non-human primate.

Embodiment 64. The method according to embodiment 62 or 63, further comprising isolating the neutralizing antibodies or neutralizing antisera from the subject.

Embodiment 65. The method according to embodiment 64, further comprising administering to a human subject an effective amount of the isolated neutralizing antibodies or neutralizing antisera via passive immunization to prevent or treat RSV infection.

Embodiment 66. The method according to any one of embodiments 62 to 65, wherein the neutralizing antibodies or neutralizing antisera against RSV comprise polyclonal antibodies against the RSV F protein peptide, or a fragment or epitope thereof, optionally wherein the neutralizing antibodies or neutralizing antisera are free or substantially free of antibodies against the collagen C-terminal propeptide.

Embodiment 67. The method according to any one of embodiments 62 to 65, wherein the neutralizing antibodies comprise monoclonal antibodies against the RSV F protein peptide, or a fragment or epitope thereof, optionally wherein the neutralizing antibodies are free or substantially free of antibodies against the collagen C-terminal propeptide.

Embodiment 68. The protein, immunogen, protein nanoparticle, VLP, isolated nucleic acid, vector, virus, pseudovirus, cell, immunogenic composition, or vaccine according to any one of embodiments 1 to 47 and 49, for use in inducing an immune response against RSV in a subject, and/or for use in treating or preventing RSV infection.

Embodiment 69. Use of the protein, immunogen, protein nanoparticle, VLP, isolated nucleic acid, vector, virus, pseudovirus, cell, immunogenic composition, or vaccine according to any one of embodiments 1 to 47 and 49 for inducing an immune response against RSV in a subject, and/or for treating or preventing RSV infection.

Embodiment 70. Use of the protein, immunogen, protein nanoparticle, VLP, isolated nucleic acid, vector, virus, pseudovirus, cell, immunogenic composition, or vaccine according to any one of embodiments 1 to 47 and 49 in the manufacture of a medicament or a prophylactic agent for inducing an immune response against RSV in a subject, and/or for treating or preventing RSV infection.

Embodiment 71. A method for analyzing a sample, comprising: contacting the sample with the protein according to any one of embodiments 1 to 33, and detecting binding between the protein and an analyte capable of specific binding to the RSV F protein peptide, or a fragment or epitope thereof.

Embodiment 72. The method according to embodiment 71, wherein the analyte is an antibody, receptor, or cell recognizing the F protein peptide, or a fragment or epitope thereof.

Embodiment 73. The method according to embodiment 71 or 72, wherein the binding indicates that the analyte is present in the sample, and/or that the subject from which the sample is derived has been infected with the RSV.

Embodiment 74. A kit comprising the protein according to any one of embodiments 1 to 33 and a substrate, pad, or vial containing or immobilizing the protein, optionally wherein the kit is a kit for ELISA or lateral flow assay.

Embodiment 75. The protein according to embodiment 1, which is a protein comprising two recombinant polypeptides corresponding to subtype A and subtype B, each recombinant polypeptide comprising a respiratory syncytial virus (RSV) F protein peptide, or a fragment or epitope thereof linked to a collagen C-terminal propeptide, wherein the C-terminal propeptides of the recombinant polypeptides form inter-polypeptide disulfide bonds.

Embodiment 76. The protein according to embodiment 75, wherein the weight ratio of subtype A to subtype B is 0.1-20, e.g., 1 : 1.

Embodiment 77. The protein according to any one of embodiments 1 to 9, wherein the F protein peptide comprises an F1 subunit and an F2 subunit of the F protein, or fragments thereof, optionally with pep27, optionally wherein the F1 subunit and the F2 subunit are linked by a disulfide bond or an artificially introduced linker.

Embodiment 78. The protein according to any one of embodiments 1 to 6 and 77, wherein the pep27 peptide, or a fragment or epitope thereof comprises one or more mutation sites.

Embodiment 79. The protein according to any one of embodiments 1 to 6, 77, and 78, wherein the pep27 peptide, or a fragment or epitope thereof comprises a sulfur-containing amino acid at the one or more mutation sites.

Embodiment 80. The protein according to any one of embodiments 1 to 6 and 77 to 79, wherein the one or more mutation sites of the pep27 peptide, or a fragment or epitope thereof are independently selected from R133C, R135C, R136C, or any combination thereof.

Embodiment 81. The protein or method according to any one of embodiments 1 to 19 and 31 to 80, wherein the fusion protein comprises a sequence independently selected from those as set forth in SEQ ID NOs: 1-102 and 119-120.

Embodiment 82. The protein or method according to any one of embodiments 1 to 19 and 31 to 81, wherein the fusion protein comprises a sequence independently selected from those as set forth in SEQ ID NOs: 16-20, 36-40, 50, 52, 53, 63, 64, and 66, optionally a sequence independently selected from those as set forth in one or more of SEQ ID NOs: 17, 37, 50, and 63.

Embodiment 83. The protein according to any one of embodiments 1 to 9 and 77, wherein the F protein peptide comprises an F1 subunit and an F2 subunit of the F protein, or fragments thereof, optionally wherein the F1 subunit and the F2 subunit are linked by a disulfide bond or an artificially introduced linker.

Embodiment 84. The protein according to any one of embodiments 1 to 9, 77, and 83, wherein the F1 subunit and the F2 subunit are linked by the artificially introduced linker.

Embodiment 85. The protein according to any one of embodiments 1 to 9, 77, and 83 to 84, wherein the artificially introduced linker is a non-amino acid compound or one or more amino acids.

Embodiment 86. The protein according to any one of embodiments 1 to 9, 77, and 83 to 84, wherein the artificially introduced linker is one or more amino acids.

Embodiment 87. The protein according to any one of embodiments 1 to 9, 77, and 83 to 86, wherein the one or more amino acids are independently selected from CGGG (SEQ ID NO: 138).

Embodiment 88. The protein according to any one of embodiments 1 to 9, 77, and 83 to 87, wherein the artificially introduced linker replaces furin site I.

Embodiment 89. The protein according to any one of embodiments 1 to 9, 77, and 83 to 88, wherein the artificially introduced linker replaces furin site II.

Embodiment 90. The protein according to any one of embodiments 1 to 9, 77, and 83 to 89, wherein the artificially introduced linker replaces furin site I and pep27 peptide.

Embodiment 91. The protein according to any one of embodiments 1 to 9, 77, and 83 to 90, wherein the artificially introduced linker replaces furin site I and pep27 peptide and one or more bases linked to furin site II.

Embodiment 92. The protein according to any one of embodiments 1 to 9, 77, and 83 to 91, wherein the one or more bases are selected from FLGFLLGV (SEQ ID NO: 126), e.g., F, FL, FLG, FLGF (SEQ ID NO: 127), FLGFL (SEQ ID NO: 128), FLGFLL (SEQ ID NO: 129), FLGFLLG (SEQ ID NO: 130), or FLGFLLGV (SEQ ID NO: 131).

### Additional Exemplary Schemes

Scheme 1. Use of a recombinant subunit vaccine comprising a soluble RSV viral surface antigen in the manufacture of a medicament for preventing respiratory syncytial virus (RSV) infection in a mammal, wherein the soluble RSV viral surface antigen comprises an F peptide, or a fragment or epitope thereof having one or more mutation sites, and the F peptide, or a fragment or epitope thereof comprises a sulfur-containing amino acid or a sulfur-containing amino acid derivative at the one or more mutation sites, and the soluble RSV viral surface antigen is linked to collagen by in-frame fusion to form a disulfide-linked trimeric fusion protein.

Scheme 2. The use according to scheme 1, wherein the RSV is of subtype A or/and subtype B.

Scheme 3. The use according to any one of schemes 1 and 2, wherein the RSV viral surface antigen comprises an F2 peptide, or a fragment or epitope thereof.

Scheme 4. The use according to any one of schemes 1 to 3, wherein the RSV viral surface antigen comprises a mutant F1 peptide, or a fragment or epitope thereof.

Scheme 5. The use according to any one of schemes 1 to 4, wherein one or more mutation sites of the F2 peptide, or a fragment or epitope thereof are R106C or/and R108C.

Scheme 6. The use according to any one of schemes 1 to 5, wherein the RSV viral surface antigen comprises a pep27 peptide, or a fragment or epitope thereof.

Scheme 7. The use according to scheme 6, wherein the pep27 peptide, or a fragment or epitope thereof comprises one or more mutation sites.

Scheme 8. The use according to scheme 6 or 7, wherein the pep27 peptide, or a fragment or epitope thereof comprises a sulfur-containing amino acid at the one or more mutation sites.

Scheme 9. The use according to any one of schemes 6 to 8, wherein the one or more mutation sites of the pep27 peptide, or a fragment or epitope thereof are independently selected from R133C, R135C, R136C, or any combination thereof.

Scheme 10. The use according to any one of schemes 1 to 9, wherein the fusion protein comprises a sequence independently selected from those as set forth in SEQ ID NOs: 1-102, or a fragment thereof.

Scheme 11. The use according to any one of schemes 1 to 10, wherein the fusion protein comprises a sequence independently selected from those as set forth in SEQ ID NOs: 6-10, 26-30, 45-48, and 58-61.

Scheme 12. The use according to any one of schemes 1 to 11, wherein the recombinant subunit vaccine is administered by intramuscular injection.

Scheme 13. The use according to any one of schemes 1 to 11, wherein the recombinant subunit vaccine is administered by intranasal spray.

Scheme 14. The use according to any one of schemes 1 to 12, wherein the recombinant subunit vaccine is administered in a single dose or in a series of doses separated by intervals of weeks or months.

Scheme 15. The use according to any one of schemes 1 to 13, wherein the recombinant subunit vaccine is administered without adjuvant.

Scheme 16. The use according to any one of schemes 1 to 13, wherein the recombinant subunit vaccine is administered with one or more adjuvants.

Scheme 17. The use according to any one of schemes 1 to 16, wherein the recombinant subunit vaccine comprises a pharmaceutically acceptable excipient selected from one or more of buffers, osmotic pressure regulators, stabilizers, and bacteriostatic agents.

Scheme 18. Use of a soluble respiratory syncytial virus (RSV) surface antigen in the preparation of a reagent for use in a method for detecting antibodies against RSV from mammalian serum, the method comprising the step of contacting the serum with the soluble RSV surface antigen, wherein the soluble RSV viral surface antigen comprises an F1 peptide, or a fragment or epitope thereof having one or more mutation sites and there is a sulfur-containing amino acid at the one or more mutation sites, and the soluble RSV surface antigen is linked to collagen by in-frame fusion to form a disulfide-linked trimeric fusion protein.

Scheme 19. Use of a recombinant subunit vaccine comprising a soluble surface antigen from respiratory syncytial virus (RSV) in the preparation of neutralizing antibodies for treating a patient infected with the RSV virus via passive immunization, the preparation comprising: immunizing a mammal, and purifying the neutralizing antibodies produced, wherein the soluble RSV viral surface antigen comprises an F peptide, or a fragment or epitope thereof having one or more mutation sites and there is a sulfur-containing amino acid at the one or more mutation sites, and the soluble surface antigen is linked to collagen by in-frame fusion to form a disulfide-linked trimeric fusion protein.

Scheme 20. The use according to scheme 19, wherein the neutralizing antibodies comprise polyclonal antibodies and/or monoclonal antibodies, wherein the neutralizing antibodies are monoclonal antibodies against an F protein or peptide.

Scheme 21. One or more polynucleotides encoding a soluble RSV viral surface antigen comprising a sequence independently selected from those as set forth in SEQ ID NOs: 1-102, or a fragment thereof.

Scheme 22. One or more vectors comprising the one or more polynucleotides according to scheme 21.

Scheme 23. A host cell comprising the one or more polynucleotides according to scheme 21, or the one or more vectors according to claim 22.

Scheme 24. The host cell according to scheme 23, which is a GH-CHO cell.

Scheme 25. The host cell according to scheme 23, wherein the vector is pTPRIMER-T0D.

### Examples

The following examples are included for illustrative purposes only and are not intended to limit the scope of the present invention. In the following examples, unless otherwise stated, when reference is made to an adjuvant such as Alum, alum, etc., it refers to an aluminum adjuvant comprising aluminum hydroxide; and when reference is made to a Trimer, e.g., a DS-CAV1-Trimer or a WT RSV F-Trimer or similar expressions, the Trimer refers to a trimer formed after fusion to a collagen C-terminal propeptide, and the collagen C-terminal propeptide used is the same as that used in other fusion proteins herein, e.g., SCB-N25C_A and SCB-N25C_A.

### Example 1: Recombinant polypeptides comprising RSV F protein peptide

RSV F glycoprotein constructs were derived from the RSV A2 strain (GenBank Accession No. AAC55970) and the Australian RSV B strain. The coding sequence for residues 1-520 of the F protein peptide was codon-optimized, synthesized, and subcloned into the mammalian expression vector pTPRIMER-T0D, which encodes the *Hind*III and *Bgl*II sites of the C-terminal propeptide of human α1 collagen. Figure 1 shows a schematic diagram of exemplary recombinant polypeptides of the RSV A2 strain. A: SCB-N25_A fusion protein (SEQ ID NO: 1); B: SCB-N25C_A (SEQ ID NO: 2); C: SCB-N25A_A (SEQ ID NO: 139); D: SCB-N25T_A (SEQ ID NO: 140); E: SCB-N25Y_A (SEQ ID NO: 141); F: SCB-N25G_A (SEQ ID NO: 142); G: SCB-N25S_A (SEQ ID NO: 143); and K: 3.1 SCB-N25C_A (SEQ ID NO: 41). Figure 10 shows a schematic diagram of exemplary recombinant polypeptides of the Australian RSV B strain. A: SCB-N25_B fusion protein (SEQ ID NO: 21); B: SCB-N25C_B (SEQ ID NO: 22); C: SCB-N25A_B (SEQ ID NO: 144); D: SCB-N25T_B (SEQ ID NO: 145); E: SCB-N25Y_B (SEQ ID NO: 146); F: SCB-N25G_B (SEQ ID NO: 147); G: SCB-N25S_B (SEQ ID NO: 148); and K: 3.1 SCB-N25C_B (SEQ ID NO: 54).

Each of the recombinant polypeptides described in this example was used in the experiments described in the following examples.

### Example 2: Pre-fusion F protein expression of recombinant polypeptides comprising RSV F protein peptides derived from RSV A2 strain

Expression levels of pre-fusion F protein in HEK-293T cell supernatants cultured for 3 days were determined by measuring the optical density using an ELISA kit (Figure 2). HEK-293T cells were transiently transfected with plasmids carrying the variants listed on the horizontal axis, and after three days, culture supernatants were harvested and centrifuged to remove cells and cell debris.

293T cells stably passaged in DMEM (with 10% FBS) were diluted to 0.75 million/mL, 0.5 mL of cells and 1.5 mL of DMEM were plated in 6-well plates, and the plates were incubated overnight in a 37°C CO2 incubator. 300 µL of DMEM (without FBS) was placed in a 1.5 mL centrifuge tube, followed by the addition of 9 µL of FuGene 6, and the mixture was mixed thoroughly and then allowed to stand at room temperature for 5 min. 100 µL of the DMEM-FuGene6 mixture was placed in each of three 1.5 mL centrifuge tubes, followed by the respective addition of 1 µg of the corresponding recombinant plasmid, and the mixture was mixed thoroughly and then allowed to stand at room temperature for 20 min. 1 mL of the medium was removed from each well of the 6-well plates with cultured 293T cells, and the entire volume of the above plasmid-FuGene 6 mixture was then added to each corresponding well, respectively. The plates were swirled gently for homogenization and then incubated in an incubator, and the cell supernatants were collected for protein expression detection after three days. The centrifuged supernatants were then analyzed at OD280 using Synagis and the AM22 antibody to determine the OD values. Panel A: shows comparison of the high optical density (OD) values among the variants; and Panel B: shows comparison of the medium OD values among the variants. SCB-N25C_A showed higher expression levels of pre-fusion F protein than other variants. SCB-N20, which is disclosed in US 10,899,800 B2 and comprises the E161P/S215P mutations, was used as the control.

Expression levels of pre-fusion F protein in Day-3 CHO cell supernatants were determined by measuring the optical density using an ELISA kit (Figure 3). Panel A: shows comparison of the high optical density (OD) values among the variants; and Panel B: shows comparison of the medium OD values among the variants. SCB-N25C_A showed high expression levels of pre-fusion F protein among the variants.

Expression levels of pre-fusion F protein in Day-7 CHO cell supernatants were determined by measuring the optical density using an ELISA kit (Figure 4). Panel A: shows comparison of the high OD values among the variants; and Panel B: shows comparison of the medium OD values among the variants. SCB-N25C_A showed high expression levels of pre-fusion F protein among variants.

### Example 3: Production of recombinant polypeptides comprising RSV F protein peptide

Secreted forms of recombinant polypeptides comprising the RSV F protein peptide were provided herein as vaccine candidates.

RSV F glycoprotein constructs were derived from the RSV A2 strain (GenBank Accession No. AAC55970) and the Australian RSV B strain. The sequence encoding residues 1 to 520 of the F protein peptide was codon-optimized, synthesized, and subcloned between the *Hind*III and *Bgl*II sites of a mammalian expression vector encoding the C-terminal propeptide of human α1 collagen. Figures 1 and 10 show schematic diagrams of exemplary recombinant polypeptides.

The recombinant plasmids were transfected into GH-CHO (dfhr⁻) cells. Selection was performed in medium without hypoxanthine-thymidine (HT) (Invitrogen), and gene amplification was conducted stepwise with increasing concentrations of MTX (Sigma) to achieve high-titer expression of the fusion proteins under serum-free culture conditions using CD007-4 TM1 medium (Jianshun Biosciences). The exemplary recombinant polypeptides were subjected to primary purification via affinity binding to Endo180 with salt gradient elution, followed by further purification on a Superdex 200 gel filtration column (GE Healthcare). The purity of the exemplary recombinant polypeptides comprising the RSV F peptide was determined by size exclusion-high-performance liquid chromatography (SEC-HPLC) according to the manufacturer's instructions (Sepax Technologies) (Figure 6). The main peak area of the SCB-N25_A fusion protein was 92.8%, and the main peak area of the SCB-N25C_A protein was 81.7%. The main peak area of the 3.1SCB-N25C_A protein was 88.58%.

Figure 5 shows the expression levels of the peptides expressed from the exemplary fusion peptides as analyzed by 8% SDS-PAGE in serum-free fed-batch cell cultures. SCB-N25C_A: Cell-free conditioned media from Day 1 to Day 13 were separated under non-reducing and reducing conditions, followed by Coomassie brilliant blue staining. The theoretical molecular weight of the fusion protein is 91 KD for the monomer and 273 KD for the trimer. 3.1SCB-N25_A: Cell-free conditioned media from Day 1 to Day 8 were separated under non-reducing and reducing conditions, followed by Coomassie brilliant blue staining. The experimental results showed that high expression levels were achieved on Day 3.

Figure 7 shows affinity kinetic assays. Panels A, B, and E of Figure 7 show binding studies by biolayer interferometry between Synagis and exemplary fusion peptides comprising the RSV F protein peptide.

For the affinity kinetic assays, a Fortebio Octet molecular interaction analyzer was used as the detection instrument, and 5 Protein A sensors were used as the sensors, which were soaked in PBS prior to use.

Reagent formulation: Regeneration solution (0.01 M glycine solution): 0.03 g of glycine was accurately weighed, dissolved in water to 40 mL, and mixed thoroughly. The pH was adjusted to approximately 1.5 with 6 M hydrochloric acid, and the solution was stored at room temperature.

Loading order: A clean 96-well assay plate was used, and loading was performed at 200 µL per well in the following order:

| 1 | 2 | 3 | 4 | 5 | 6 | 7 | 8 | 9 | 10 | 11 | 12 |
|---|---|---|---|---|---|---|---|---|---|---|---|
| B | L1 | B | S1 | | B | L2 | B | S1 | | R | N |
| B | L1 | B | S2 | | B | L2 | B | S2 | | R | N |
| B | L1 | B | S3 | | B | L2 | B | S3 | | R | N |
| B | L1 | B | S4 | | B | L2 | B | S4 | | R | N |
| B | L1 | B | S5 | | B | L2 | B | S5 | | R | N |

| | | | | | | | | | | | |
|---|---|---|---|---|---|---|---|---|---|---|---|
| Notes: B: PBS; L1: 10 µg/mL Synagis; L2: 10 µg/mL D25; S1 = 40 µg/mL protein sample to be tested; S2 = 20 µg/mL protein sample to be tested; S3 = 10 µg/mL protein sample to be tested; S4 = 5 µg/mL protein sample to be tested; S5 = PBS; R: regeneration solution (0.01 M glycine solution); and N: PBS. | | | | | | | | | | | |

After the above reagents and samples were loaded, the data acquisition software was opened, and the information of the samples and reagents used was completed before the program was run. The assays were performed in two groups: the affinity between the protein samples to be tested and Synagis was tested in the first group; and the affinity between the protein samples to be tested and the D25 antibody was tested in the second group. After the run was completed, analysis was performed using the Octet data analysis software, and the Kₒₙ, K_{dis}, and K_{D} results were obtained.

The binding affinities between Synagis and the purified exemplary SCB-N25_A and SCB-N25C_A recombinant polypeptides are shown in Figure 7 and the labels in Figure 7. Panels C, D, and F of Figure 7 show affinity kinetic studies by binding between D25 and exemplary fusion peptides comprising the RSV F protein peptide. The corresponding K_{D}, Kₒₙ, and K_{dis} values from the antibody D25 affinity assays are shown in the labels in Figure 7. SCB-N25C_A exhibited high affinity for both palivizumab and antibody D25.

### Example 4: Functional characterization of recombinant polypeptides comprising RSV F protein peptide

To evaluate the immunogenicity and protective efficacy of the exemplary recombinant polypeptides produced as described in Example 1, randomly divided BALB/c mice were immunized twice intramuscularly on Day 0 and Day 21 with one of three doses (1 µg, 6 µg, and 30 µg) of the exemplary fusion polypeptides with different adjuvants. Another group immunized with PBS served as the control group.

Figures 8A-8E show the results of immunization assays using exemplary fusion peptides comprising the RSV F protein peptide (as shown in Figure 8A).

Figure 8A shows a schematic diagram of the immunization experimental method: mice were immunized on Day 0 and Day 21, and sera were collected on Day 0 and Day 35, respectively. 6- to 8-week-old SPF female BALB/c mice were randomly divided into 16 groups of 10 mice each; the mice in each group were immunized intramuscularly with 50 µL of PBS or the corresponding antigen, and boosted once after 3 weeks; and blood samples were collected by retroorbital venipuncture at Week 2 after the second immunization, and sera were obtained by centrifugation. Spleens were harvested from 4 mice in each group for ELISpot assays.

Figure 8B shows the serum anti-F IgG ELISA titer values against purified exemplary fusion peptides comprising the RSV F protein peptide and the adjuvant Alum, Alum + CpG 1018, or CAS-1. Sera from animals immunized with the hRSV vaccine candidate were mixed with live hRSV A2 virus, and the serum-virus mixtures were incubated with Hep2 cells. RSV A2 virus can infect Hep2 cells. After 3 to 4 days of incubation (determined by the Cytopathic Effect (CPE), when CPE in the virus control well reached 60% or above), the newly proliferated viruses were captured by biotin-labeled Synagis, followed by color development with SA-HRP secondary antibody. The level of virus proliferation can be indirectly reflected by the absorbance value at OD450 nm. If the added serum sample to be tested comprises neutralizing antibodies that can neutralize hRSV virus, the neutralized viruses will lose the ability to infect cells, which will in turn affect the absorbance value. The lower the OD450 nm value, the higher the neutralizing antibody titer in the serum. The fusion proteins adjuvanted with Alum + CpG 1018 showed higher titer values than the corresponding fusion proteins adjuvanted with Alum or CAS-1. SCB-N25C_A showed higher titer values than other antigens.

Figure 8C shows the titers of D25 and palivizumab competitive IgG that provide 50% inhibition of the binding of D25 and palivizumab to heat-inactivated RSV (HI-RSV) particles, as determined by serum sample dilutions. Values are expressed as log2 and mean ± SEM. DCA: D25 was added as the coating antibody to high-binding ELISA 96-well plates, and the plates were coated overnight at 2-8°C. Subsequently, a mixture of serially diluted serum samples and SCB-N20-biotin (N20-biotin) antigen label (1 : 1) was added. DCA in the immune serum competed with D25 for binding to the Site 0 on the F protein of the N20-biotin antigen label. Then, secondary antibody (SA-HRP) was added to bind to N20-biotin. Unbound fractions were removed by elution in each step. Finally, TMB was added for color development, and the reaction was terminated with H₂SO₄. The lower the OD450 nm value, the higher the competitive antibody DCA titer in the serum. PCA: Synagis was added as the coating antibody to high-binding ELISA 96-well plates, and the plates were coated overnight at 2-8°C. Subsequently, a mixture of serially diluted serum samples and SCB-N20-biotin (N20-biotin) antigen label (1 : 1) was added. PCA in the immune serum competed with Synagis for binding to the Site II on the F protein of the N20-biotin antigen label. Then, secondary antibody (SA-HRP) was added to bind to N20-biotin. Unbound fractions were removed by elution in each step. Finally, TMB was added for color development, and the reaction was terminated with H₂SO₄. The lower the OD450 nm value, the higher the competitive antibody PCA titer in the serum. All antigens adjuvanted with Alum showed lower antibody (DCA and PCA) responses than those with the other two adjuvants. When used in combination with the Alum + CpG1018 adjuvant, both DS-CAV1-Trimer and SCB-N20_A showed high DCA and PCA titers, with no significant difference between them. SCB-N25_Aperformed the worst. None of the four antigen candidates used in combination with the CAS-1 adjuvant had effective DCA titers. SCB-N20_A and SCB-N25_A had high PCA titers and the DS-CAV1-Trimer had the lowest titers.

Figure 8D shows the results of the ELISpot assay. Splenocytes from vaccine-immunized mice secreted cytokines locally upon stimulation, which were captured by specific monoclonal antibodies. The captured cytokines were bound to a biotin-labeled secondary antibody, followed by binding to alkaline phosphatase-labeled avidin. After incubation with the BCIP/NBT substrate, purple-blue spots appeared on the PVDF plate, indicating that the cells secreted specific cytokines. The results were obtained by analyzing the spots with an ELISpot enzyme-linked spot analysis system. N25C_A antigen in combination with the Alum adjuvant generated a strong Th1-type cellular immune response. All antigens in combination with the Alum + CpG1018 adjuvant generated a low Th2-type cellular immune response. All antigens in combination with the CAS-1 adjuvant generated high Th1-type and Th2-type cellular immune responses.

Sera were evaluated by enzyme-linked immunosorbent assay (ELISA). Briefly, 96-well plates were coated overnight at 4°C with 2 µg/mL of purified exemplary fusion peptides in PBS and blocked with 1 mg/mL of BSA. The plates were washed with PBST, followed by incubation with serial 2-fold serum dilutions (from 1 : 64 to 1 : 262,144) for 2 hours at room temperature. Bound antibodies were detected with HRP-conjugated goat anti-mouse IgG (SouthernBiotech) for 1 hour at room temperature. Enzymatic reaction was performed with TMB (Thermo) and terminated by the addition of 2 M HCl, and the absorbance at 450 nm was recorded. Sera from PBS-immunized mice at the same dilutions were used as the negative control. The antibody titer is defined as the serum dilution that resulted in a ratio of OD for RSV F-trimer to OD for PBS of 2.0.

RSV microneutralization assays were performed using HeLa cells and the RSV A2 strain. Sera were heat-inactivated at 56°C for 30 minutes, and serially diluted in serum-free DMEM in 96-well cell culture plates (50 µL per well). An equal volume of virus (1,000 pfu/mL, prepared in serum-free DMEM) was added to the plates, and the serum-virus mixtures were incubated at 37°C for 1 hour. 100 µL of DMEM supplemented with 10% FBS containing approximately 5 × 10⁴ HeLa cells was added to each well of the plates, and the plates were incubated at 37°C until the positive control (virus only) wells showed 100% CPE. The plates were washed with PBST, and fixed with PBS containing 80% pre-chilled acetone for 10 minutes. Appropriate amount of palivizumab was added to the wells. The plates were blocked with 1 mg/mL of BSA for 1 hour, and then incubated at room temperature for 2 hours. After three washes, HRP-conjugated goat anti-human IgG (SouthernBiotech) was added, the enzymatic reaction was performed, and the OD at 450 nm was recorded. The dilution that causes 50% inhibition of CPE formation is determined as the neutralizing antibody titer.

Since Antigenic Site II is exposed on the exemplary recombinant polypeptides, palivizumab and D25 mAb competitive ELISA assays were performed to determine whether the antibodies induced by the exemplary recombinant polypeptides were directed against this site.

Palivizumab and D25 mAb competitive ELISA assays were performed using 96-well ELISA plates coated with 5 × 10⁶ pfu/mL heat-inactivated RSV (HI-RSV) in 50 mM carbonate-bicarbonate buffer (pH 9.2), with incubation overnight at 4°C. Uncoated surfaces were blocked with 1 mg/mL of BSA. Two-fold dilutions of the serum mixture (from 1 : 32 to 1 : 4,096) were added to the wells together with an appropriate amount of palivizumab or D25 mAb, and the plates were incubated at room temperature for 2 hours. Bound palivizumab was detected using HRP-conjugated goat anti-human IgG (SouthernBiotech) and the TMB substrate. The wells containing sera from PBS-immunized mice represent the non-competitive positive control group, and the percent inhibition is calculated as ((OD PBS - OD RSV F-trimer)/OD PBS) × 100%. Competitive binding titers are expressed as dilutions that cause 50% inhibition.

Figures 9A and 9B show the immunogenicity assays of the vaccine candidate based on 3.1 SCB-N25C_A in RSV-infected mice. According to the detection results shown in Figure 9B, the RSV virus vaccine candidates (SCB-N25C_A and 3.1 SCB-N25C_A) used in combination with an aluminum adjuvant significantly produced neutralizing antibodies against the hRSV A2 and hRSV B18537 strains. For 3.1 SCB-N25C_A, the neutralizing antibody titers against the hRSV A2 and hRSV B18537 strains increased in a dose-dependent manner. When administered at the same molar dose (SCB-N25C_A and 3.1 SCB-N25C_A administered at 0.36 µg, corresponding to 0.24 µg of Abrysvo^{®} and Arexvy^{®}), the neutralizing antibody titers against the hRSV A2 strain in the SCB-N25C_A and 3.1 SCB-N25C_A groups were significantly higher than those in the comparator vaccine groups. The detection method for neutralizing antibodies is shown in Figure 12, which shows the titer detection of neutralizing antibodies against the RSV A2 and B18537 strains.

Figure 11 shows the ELISA-based affinity assays of antibodies against RSV vaccine candidate antigens.

### Affinity assay:

First, the antibodies were diluted to 1 µg/mL with PBS and coated onto ELISA plates at a volume of 100 µl per well, and the plates were incubated overnight at 2°C-8°C. After incubation, the coating solution was discarded, and the plates were washed 3 times with PBST at a volume of 300 µl per well, followed by blocking. Then, the proteins to be tested were 3-fold serially diluted from an initial concentration of 5 µg/mL to the 11th well, and added to the ELISA plates at a volume of 100 µl per well. Diluent was added to the 12th well, and the plates were incubated at 37°C for 90 min. After incubation, the liquid in the wells was discarded, and the plates were washed 3 times with PBST at a volume of 300 µl per well. Subsequently, Trimer-Tag rabbit polyclonal antibody was diluted to 1 µg/mL with diluent and added to the ELISA plates at a volume of 100 µl per well, and the plates were incubated at 37°C for 60 min. After incubation, the liquid in the wells was discarded, and the plates were washed 3 times with PBST at a volume of 300 µl per well. Then, the Goat Anti-Rabbit-IgG-HRP was diluted with diluent at a ratio of 1:10000 and added to the ELISA plates at a volume of 100 µl per well, and the plates were incubated at 37°C for 60 min. After incubation, the liquid in the wells was discarded, and the plates were washed 3 times with PBST at a volume of 300 µl per well. Finally, the chromogenic substrate was added to the ELISA plates at a volume of 100 µl per well, and the plates were incubated in the dark. After incubation, the reaction was terminated with 100 µl of sulfuric acid standard solution per well, and the OD450 value of each well was read with a microplate reader. After the assays were completed, the data were exported and processed with software to obtain the results.

Figure 11 shows the detection results using four antibodies recognizing different epitopes: A: Clone 4D7 antibody (AntibodySystem); B: Clone AM 22 antibody (Creative Biolabs); C: Clone D26 antibody; and D: Clone RSB1 antibody (AntibodySystem). Clone 4D7 antibody has stronger recognition for the post-fusion conformation. The stronger the signal recognized by this antibody, the closer the antigen is to the post-fusion conformation (as shown for WT-RSV-F-Trimer in Panel A, Figure 11). The other three antibodies have stronger specific recognition for the pre-fusion conformation (as shown in Panels B, C, and D, Figure 9). According to the detection results in Figure 11, compared with the wild-type RSV-F protein, the antigen proteins of the RSV virus vaccine candidates (SCB-N25C_A and SCB-N25C_B) showed stronger binding affinity for pre-fusion conformation-specific antibodies of the RSV F protein, and weaker binding affinity for post-fusion conformation-specific antibodies of the RSV F protein, indicating that the antigen proteins of the RSV virus vaccine candidates (SCB-N25C_A and SCB-N25C_B) contain a higher proportion of RSV F protein in the pre-fusion conformation.

Figures 12A-12B show the titer detection of neutralizing antibodies against the RSV A2 and B18537 strains. The assays were performed in RSV-naive mice to verify the immunogenicity.

### Immunization procedure:

6- to 8-week-old SPF female BALB/c mice were randomly divided into 4 groups of 10 mice each;
the mice in each group were immunized intramuscularly with 50 µL of the RSV virus vaccine candidate, following a 3-dose immunization program on Days 0, 21, and 42; and blood samples were collected by retroorbital venipuncture 2 weeks after the second immunization (Day 35) and the third immunization (Day 56), and sera were obtained by centrifugation.

Titer detection of neutralizing antibodies against the RSV A2 and B18537 strains:
Sera from animals immunized with the hRSV vaccine candidate were mixed with live hRSV A2 and hRSV B18537 viruses, respectively, and the serum-virus mixtures were incubated with Hep2 cells. The hRSV A2 and hRSV B18537 viruses can infect Hep2 cells. After 3 to 4 days of incubation (determined by the Cytopathic Effect (CPE), when CPE in the virus control well reached 60% or above), the newly proliferated viruses were captured by biotin-labeled Synagis, followed by color development with SA-HRP secondary antibody. The level of virus proliferation is indirectly measured by the absorbance value at OD450 nm. If the added serum sample to be tested comprises neutralizing antibodies that can neutralize hRSV virus, the neutralized viruses will lose the ability to infect cells, which will in turn affect the absorbance value. The lower the OD450 nm value, the higher the neutralizing antibody titer in the serum.

According to the detection results shown in Figure 12B, the RSV vaccine candidate (SCB-N25C bivalent vaccine) used in combination with an aluminum adjuvant significantly enhanced the production of neutralizing antibodies against the hRSV A2 and hRSV B18537 strains. For the SCB-N25C bivalent vaccine, the neutralizing antibody titers against the hRSV A2 and hRSV B18537 strains increased in a dose-dependent manner.

Figures 13A-13C show the immunogenicity assays of an exemplary trimerized fusion peptide bivalent vaccine in primed mice.

### Detection of neutralizing antibodies:

The detection results shown in Figure 13B strongly indicated that the SCB-N25C bivalent PreF-trimer vaccine candidate elicited effective immune responses in the primed mouse model, which were comparable to those of the recently approved and marketed RSV vaccine antigens. Furthermore, both the SCB-N25C bivalent candidates without and with an adjuvant elicited similar immune responses in the mouse model studied. The detection method for neutralizing antibodies is shown in Figures 12A-12B, which show the titer detection of neutralizing antibodies against the RSV A2 and B18537 strains.

### ELISpot assay:

Splenocytes from RSV-immunized mice secreted cytokines such as IFN-γ, IL-2, IL-4, and IL-5 locally upon stimulation with the peptide pools of RSV A or B virus, which were captured by specific monoclonal antibodies. The captured cytokines were bound to a biotin-labeled secondary antibody, followed by binding to alkaline phosphatase-labeled avidin. After incubation with the BCIP/NBT substrate, purple-blue spots appeared on the PVDF plate, indicating that the cells secreted specific cytokines. The immune responses of mice in different groups were compared by analyzing the spots with an ELISpot enzyme-linked spot analysis system.

Detection evaluation for Figure 13C: Splenocytes were collected on Day 28 post-vaccination to evaluate antigen-specific T cell responses. Th1 (IL-2 and IFN-γ) and Th2 (IL-4 and IL-5) cytokines were detected by ELISpot upon stimulation with the peptide pools of RSV A and RSV B F proteins. The results indicated that the overall cell-mediated immunity (CMI) responses were comparable between the non-adjuvanted group and the aluminum adjuvant group, with a bias towards a Th1 phenotype in this animal model. This also reflects that Th1 cells play an active role in resisting RSV infection.

Figures 14A-14B show the immunogenicity assays of an exemplary trimerized fusion peptide bivalent vaccine in non-human primates.

Figures 15A-15D show mouse challenge assays.

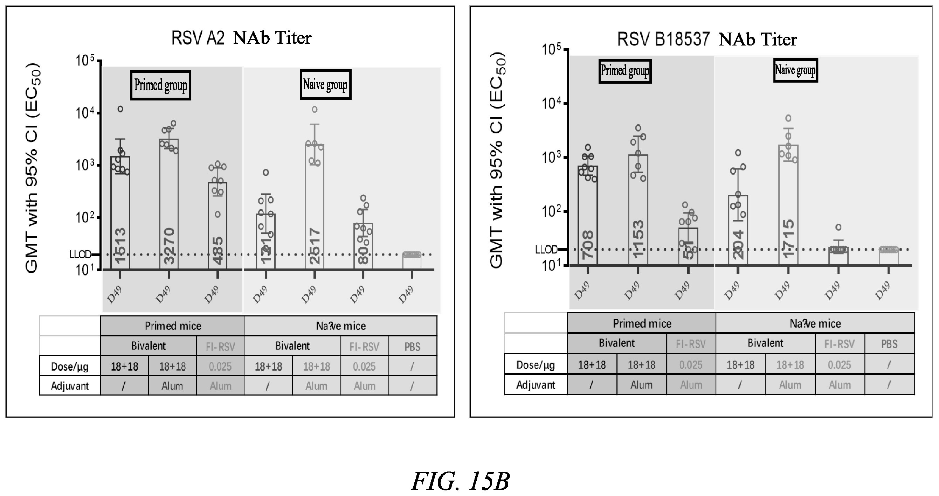

The detection method for neutralizing antibodies is shown in Figure 12**,** which shows the titer detection of neutralizing antibodies against the RSV A2 and B18537 strains. The detection results in Figure 15B indicated that, compared with the normal saline (PBS) group, high levels of neutralizing antibodies were induced by three doses of the bivalent SCB-N25C vaccine with an aluminum adjuvant in naive animals, as well as by a single dose of the bivalent SCB-N25C_A and SCB-N25C_B vaccine without an adjuvant or with an aluminum adjuvant in primed animals. In contrast, the formalin-inactivated (FI) vaccine FI-RSV induced low levels of neutralizing antibodies. In addition, the bivalent vaccines showed superior broad-spectrum immunity to the monovalent vaccines. The neutralizing antibody titers (IU/ml) against the bivalent vaccines were approximately 3-fold higher than those against the monovalent SCB-N25C_B vaccine; and the neutralizing antibody titers (IU/ml) against the bivalent vaccines were approximately 2-fold higher than those against the monovalent SCB-N25C_A vaccine (data not shown).

### Detection of viral load:

1 mL of Hep2 cells at a density of 3.0 × 10⁵/mL was added to each well of a 12-well cell plate, and the plate was placed in a CO2 cell incubator overnight to obtain a monolayer of cells. The samples to be tested were taken out from a -80°C freezer and thawed in a 37°C water bath. Tissues were homogenized twice in a TissueLyser II (QIAGEN) at 30 Hz, for 1 min 20 s each time. The homogenized tissues were added to a 15 mL centrifuge tube, and the remaining HBSS solution was transferred to the centrifuge tube. After mixing thoroughly, the mixture was centrifuged at 4,000 rpm and 4°C for 10 min. The centrifuged supernatant was transferred to a new centrifuge tube. The centrifuged supernatant (final tissue concentration of 100 mg/mL in the supernatant) was serially diluted 6-fold for 3 gradients with assay medium, with the dilution method as shown in Figure 1. After 0.5 mL of the medium was discarded from each well of the 12-well cell plate, 0.05 mL of the tissue homogenate supernatant or its dilutions was added to each well of the 12-well plate pre-plated with the cells. The plate was incubated on a shaker for 10 min, followed by incubation in a 37°C cell incubator for 4 h to allow sufficient virus adsorption. The liquid in the 12-well cell plate was replaced with medium containing agarose, and the plate was left at room temperature for 40 min. After the agarose solidified, the plate was placed in a 37°C cell incubator and incubated for 7 days. After 7 days, the cells were fixed with 3 mL of paraformaldehyde per well at room temperature for 4 h, then the fixative was removed, and the plate was rinsed with water. Then, 0.25 mL of 0.5% crystal violet solution was added to each well, and the plate was shaken on a shaker for 15 min. The crystal violet was rinsed off with water, and the plate was dried. Images were scanned, the number of plaques in each well was counted, and the virus titer in the sample was calculated. The virus titer is expressed as Log10 (number of plaques per gram of lung tissue homogenate).

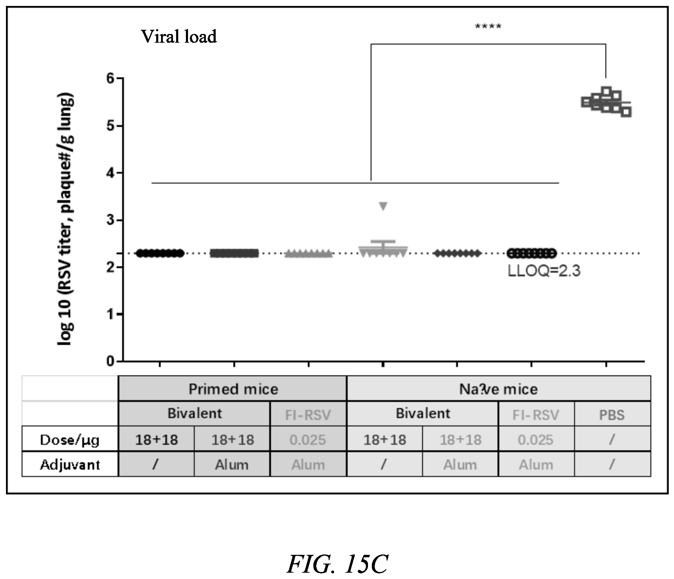

The results in Figure 15C indicated that, compared with the PBS group, the formalin-inactivated (FI) vaccine FI-RSV reduced the RSV virus titer in lung tissues, while both the non-adjuvanted and adjuvanted SCB-N25C bivalent vaccines significantly reduced the RSV viral load in the lung tissues of primed animals, indicating a strong in vivo anti-RSV immune effect induced by the vaccines.

### Pathological evaluation

### HE staining and analysis method:

After perfusion, the lung tissues were fixed in 4% paraformaldehyde for more than 24 hours, dehydrated, and then sectioned at a thickness of 4 µm. The staining rack with the mounted sections was placed in an oven at 60°C-65°C for heating for 1 hour until the paraffin melted to a transparent liquid state. The staining rack was left at room temperature for 10 minutes for cooling. After cooling, HE staining was performed, and the sections were finally mounted. Finally, manual semi-quantitative scoring was performed.

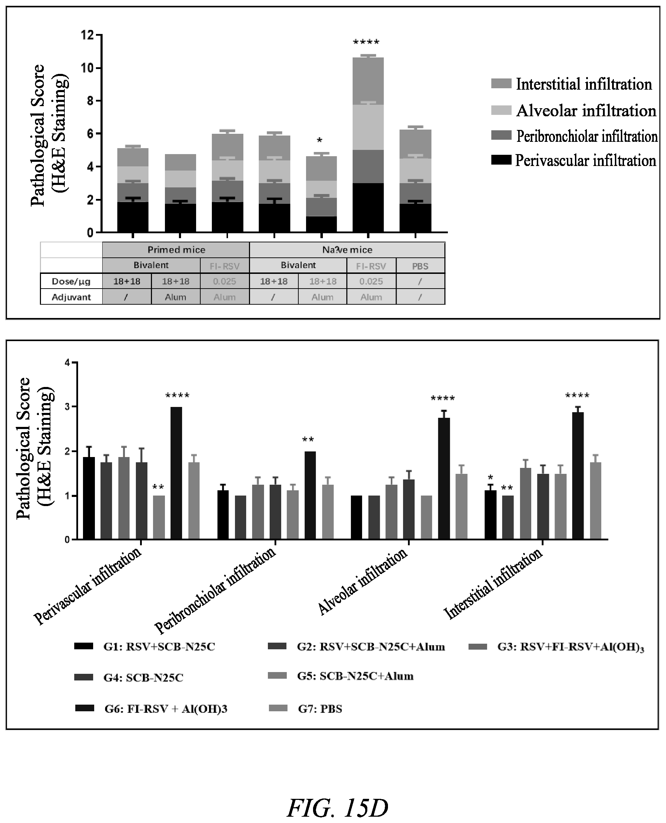

The results in Figure 15D indicated that, compared with the PBS group, the formalin-inactivated (FI) vaccine FI-RSV aggravated lung pathological damage as expected, showing vaccine-enhanced disease. In contrast, both the non-adjuvanted and adjuvanted bivalent vaccines SCB-N25C_A and SCB-N25C_B significantly reduced the RSV viral load in the lung tissues of primed animals, with no significant increases in lung pathology.

Figures 16A-16C show head-to-head immunogenicity comparison assays of an exemplary trimerized fusion peptide vaccine versus commercially available RSV vaccines.

The titers of neutralizing antibodies against the hRSV A2 and hRSV B18537 strains in the SCB-N25C_A group were higher than those in the Arexvy^{®} and Abrysvo^{®} groups (Figure 16B). The ratio of neutralizing antibody titer to binding antibody titer was significantly superior to that in the Arexvy^{®} and Abrysvo^{®} groups. It is demonstrated that SCB-N25C_A may serve as a potential best-in-class RSV vaccine (Figure 16C).

### Example 5: Head-to-head immunogenicity comparison of SCB-N25C_A versus commercially available RSV vaccines

The detection method for neutralizing antibodies is shown in Figures 12A-12B**,** which show the titer detection of neutralizing antibodies against the RSV A2 and B18537 strains. The titers of neutralizing antibodies against the hRSV A2 and hRSV B18537 strains in the SCB-N25C_A group were higher than those in the Arexvy^{®} and Abrysvo^{®} groups (Figure 16B). The ratio of neutralizing antibody titer to binding antibody titer in the SCB-N25C_A group was significantly superior to that in the Arexvy^{®} and Abrysvo^{®} groups (Figure 16C). It is demonstrated that SCB-N25C_A may serve as a potential best-in-class RSV vaccine.

### Example 6: Validation of predicted structure by electron microscopy

Figure 17 shows electron microscopy images of the RSV A2 and B strains.

Protein samples of SCB-N25C_A and SCB-N25C_B were added onto negatively stained grids that had been subjected to glow discharge hydrophilization treatment, and left to stand for a period of time to allow the particles to attach to the grids. After excess sample was blotted off with filter paper, the grids were rinsed with deionized water to avoid interference from salt ions in the sample buffer on subsequent experiments. The rinsed particles were then stained with staining solution at room temperature. After staining was completed, excess liquid was blotted off by touching the edge of the grids with filter paper, and the grids were left to air-dry naturally. Sample observation and electron microscopy data collection were performed on a Thermo Fisher Talos L120C 120 kV transmission electron microscope. A CETA direct electron counting detector was used as the electron detector, and data collection was completed with SerialEM software in super-resolution mode.

According to the detection results in Figure 17, the vast majority of the antigen protein particles of the SCB-N25C_A and SCB-N25C_B RSV virus vaccine candidates were present in a round shape in monomeric or multimeric forms, which was consistent with the RSV F protein in the post-fusion conformation reported in literature, and significantly different from the rod-like shape of the wild-type RSV-F protein. This indicates that the antigen proteins of the trimeric RSV virus vaccine candidates disclosed herein contain a higher proportion of RSV F protein in the pre-fusion conformation. They retain the major neutralizing antibody epitopes (Ø, V, IV, III, II, and I), and exhibit weak binding to post-fusion conformation-specific antibodies. The titers of neutralizing antibodies against both SCB-N25C_A and SCB-N25C_B were approximately 10-fold higher than those for the F-trimer in the post-fusion conformation. At Epitope Ø (D25 mAb) and Epitope II (palivizumab), the binding affinity of the SCB-N25C vaccine candidate, SCB-N25C_A or SCB-N25C_B, was approximately 20-fold higher than that of the F protein in the post-fusion conformation (data not shown).

### Example 7: Clinical trial design

The SCB-N25C vaccine comprises RSV F protein subunits from two major circulating strains, strain A (SCB-N25C_A) and strain B (SCB-N25C_B). SCB-N25C_A or SCB-N25C_B is a recombinant RSV F-trimer protein designed by fusing the extracellular domain of the RSV F protein to Trimer-Tag^{™}. Once prepared, each 0.5 mL dose will comprise 90 µg or 360 µg total antigen of SCB-N25C_A and SCB-N25C_B in a 1 : 1 ratio, which is the SCB-N25C bivalent vaccine (SCB-1019). Two dose levels of the SCB-1019 vaccine will be tested in the study, with each dose level formulated with or without alum.

| Dosage form | SCB-N25C_A | SCB-N25C_B | Total SCB-N25C (SCB-1019) | Alum |
|---|---|---|---|---|
| Low | 45 µg | 45 µg | 90 µg | - |
| Low + adjuvant | 45 µg | 45 µg | 90 µg | 750 µg |
| High | 180 µg | 180 µg | 360 µg | - |
| High + adjuvant | 180 µg | 180 µg | 360 µg | 750 µg |

Placebo: normal saline (NaCl 0.9%). Administration route: i.m.

### Example 8: Affinity of SCB-1019T(A) (3.1SCB-N25C A) and SCB-1019T(B) (3.1SCB-N25C B) for RSV F protein mAbs

The method for the affinity kinetic assay is shown in Figure 7. Binding studies of exemplary fusion peptides of the RSV F protein peptide were performed by biolayer interferometry. First, individual mAbs were immobilized on Protein A or AMC2 sensors, and the sensors were then immersed in the exemplary fusion peptides at different concentrations to measure binding kinetics. The resulting curves were fitted to a 1 : 1 binding model by subtracting buffer reference values to obtain the Kₒₙ, K_{dis}, and K_{D} values, as shown in Table 1. SCB-1019T(A) and SCB-1019T(B) were compared to the post-fusion control protein WT-F-Trimer and the commercial GSK vaccine F protein control. SCB-1019T(A) and SCB-1019T(B) showed strong binding affinity for individual pre-fusion conformation-specific mAbs, and very weak affinity for the post-fusion conformation-specific mAb 4D7.

Table 1 shows the results of the affinity kinetic assays. Binding studies of exemplary fusion peptides of the RSV F protein peptide were performed by biolayer interferometry. First, individual mAbs were immobilized on Protein A or AMC2 sensors, and the sensors were then immersed in the exemplary fusion peptides at different concentrations to measure binding kinetics. The resulting curves were fitted to a 1 : 1 binding model by subtracting buffer reference values to obtain the Kₒₙ, K_{dis}, and K_{D} values, as shown in the table. SCB-1019T(A) and SCB-1019T(B) were compared to the post-fusion control protein WT-F-Trimer and the commercial GSK vaccine F protein control. SCB-1019T(A) and SCB-1019T(B) showed strong binding affinity for individual pre-fusion conformation-specific mAbs, and very weak affinity for the post-fusion conformation-specific mAb 4D7.

**Table 1**

| Sample name | Monoclonal antibody | Epitope name | KD (M) | kon(1/Ms) | kdis(1/s) | RLU(nm) |
|---|---|---|---|---|---|---|
| SCB-1019T(A) | D25 | Φ | 1.14E-10 | 2.96E+05 | 3.38E-05 | 1.1907 |
| SCB-1019T(B) | | | 2.47E-10 | 4.53E-05 | 1.12E-04 | 1.4998 |
| WT-F-Trimer | | | <1.0E-12* | 1.21E+04 | <1.0E-07 | 0.2664 |
| GSK | | | 2.21E-10 | 4.72E+05 | 1.04E-04 | 1.1135 |
| SCB-1019T(A) | AM22 | Φ | 2.09E-10 | 1.00E-05 | 2.10E-05 | 1.1255 |
| SCB-1019T(B) | | | 4.95E-10 | 8.07E+04 | 3.99E-05 | 1.1049 |
| WT-F-Trimer | | | <1.0E-12* | 2.28E-04 | <1.0E-07 | 0.1231 |
| GSK | | | 6.44E-10 | 3.04E+05 | 1.96E-04 | 1.4232 |
| SCB-1019T(A) | hRSV90 | V | <1.0E-12 | 1.23E-05 | <1.0E-07 | 0.9284 |
| SCB-1019T(B) | | | 1.94E-09 | 7.88E+05 | 1.53E-03 | 0.068 |
| WT-F-Trimer | | | 1.27E-08" | 2.14E+04 | 2.71E-04 | 0.0884 |
| GSK | | | 7.71E-10 | 2.82E+05 | 2.18E-04 | 1.0291 |
| SCB-1019T(A) | RSB1 | V | <1.0E-12 | 1.82E-05 | <1.0E-07 | 1.3052 |
| SCB-1019T(B) | | | 2.72E-11 | 2.00E-05 | 5.43E-06 | 1.4636 |
| WT-F-Trimer | | | 2.48E-09* | 1.58E-04 | 3.93E-05 | 0.2119 |
| GSK | | | 5.36E-10 | 3.53E+05 | 1.89E-04 | 1.3428 |
| SCB-10191(A) | MPE8 | III | <1.0E-12 | 412E+04 | <1.0E-07 | 0.59 |
| SCB-1019T(B) | | | 7.71E-10 | 4.45E-04 | 3.43E-05 | 0.6721 |
| WT-F-Trimer | | | 2.21E-08* | 1.02E-04 | 2.26E-04 | 0.0968 |
| GSK | | | 2.22E-09 | 1.59E-05 | 3.53E-04 | 0.815 |
| SCB-1019T(A) | AM14 | Spatial epitope | 3.82E-10 | 1.49E-05 | 5.67E-05 | 0.6931 |
| SCB-1019T(B) | | | 2.00E-10 | 2.22E+05 | 4.43E-05 | 0.5339 |
| WT-F-Trimer | | | NA | NA | NA | 0 |
| GSK | | | 4.84E-10 | 7.52E-05 | 3.64E-04 | 1.3376 |
| SCB-1019T(A) | 101F | IV | <1.0E-12 | 4.68E-05 | <1.0E-07 | 1.303 |
| SCB-1019T(B) | | | <1.0E-12 | 4.85E+05 | <1.0E-07 | 1.4591 |
| WT-F-Trimer | | | <1.0E-12 | 1.19E+05 | <1.0E-07 | 1.6068 |
| GSK | | | 1.01E-09 | 6.32E+05 | 6.36E-04 | 1.554 |
| SCB-1019T(A) | 4D7 | I | 7.20E-10 | 5.10E+04 | 3.68E-05 | 1.2872 |
| SCB-1019T(B) | | | 4.13E-09 | 5.52E+04 | 2.28E-04 | 1.0031 |
| WT-F-Trimer | | | <1.0E-12 | 7.24E+04 | <1.0E-07 | 1.3222 |
| GSK | | | 5.32E-09 | 1.11E+05 | 5.90E-04 | 1.1865 |

| | | | | | | |
|---|---|---|---|---|---|---|
| Notes: * indicates that the response RLU is particularly low and the affinity K_{D} value is inaccurate. | | | | | | |

### Example 9: Immunogenicity of SCB-1019T bivalent vaccine in normal healthy naive mice

Figure 18A shows a schematic diagram of the experimental method: the mice were randomly divided into 2 groups of 10 mice each, and the mice in each group were immunized twice intramuscularly with the RSV virus vaccine candidate on D0 and D21 according to the table below; and blood samples were collected by retroorbital venipuncture on Day 35, and sera were obtained by centrifugation. The bivalent vaccine was SCB-1019T(A) + SCB-1019T(B) at a weight ratio of 1 : 1, with 75 µg of Alum per dose. Figure 18B shows the titer values of neutralizing antibodies induced by the bivalent trimerized fusion peptide vaccine against live hRSV A2 and hRSV B18537 viruses. The detection method for neutralizing antibodies is shown in Figure 12.

Compared with SCB-1019T without an adjuvant, 18 µg of SCB-1019T with an adjuvant induced higher neutralizing antibody titers. A consistent trend was observed for both the A2 and B18537 strains, indicating a strong adjuvant dependence of the vaccine (Figure 18B).

### Example 10: Immunogenicity of SCB-1019T bivalent vaccine in primed mice

Figures 19A-19B show the immunogenicity assays of an exemplary trimerized fusion peptide bivalent vaccine in primed mice. Figure 19A shows a schematic diagram of the experimental method: 6- to 8-week-old SPF female BALB/c mice were intranasally infected with RSV virus to develop natural immunity; after 28 days, the mice were randomly divided into 3 groups of 10 mice each, and the mice in each group were immunized once intramuscularly with 50 µl of the RSV virus vaccine candidate or commercial GSK control vaccine according to the table below; and

blood samples were collected by retroorbital venipuncture on Day 0, Day 14, Day 28, and Day 56, and sera were obtained by centrifugation. The bivalent vaccine was SCB-1019T(A) + SCB-1019T(B) at a weight ratio of 1 : 1, with 75 µg of Alum per dose. Figure 19B shows the titer values of neutralizing antibodies induced by the bivalent trimerized fusion peptide vaccine against live hRSV A2 and hRSV B18537 viruses. The detection method for neutralizing antibodies is shown in Figure 12.

Both SCB-1019T without an adjuvant and SCB-1019T with an adjuvant induced strong neutralizing antibody immune responses, with no difference between the two dosage forms. There was also no difference as compared to the commercial GSK vaccine (Figure 19B).

### Example 11: Efficacy of SCB-1019T bivalent vaccine in cotton rat challenge assays

Figures 20A-20D show cotton rat challenge assays. Figure 20A shows a schematic diagram of the experimental method: a total of 14 cotton rats were used, and 5 cotton rats were intranasally infected with RSV virus to develop natural immunity, designated as Group 1; there were 2 groups of naive mice, and the mice in each group were immunized intramuscularly with 50 µL of the RSV virus vaccine candidate or control vaccine according to the table below; and blood samples were collected by retroorbital venipuncture at Week 9 (i.e., Day 21 post-boost) and 5 days post-challenge and sera were obtained by centrifugation, and mouse viral load and lung pathological sections were detected on Day 68. The bivalent vaccine was SCB-1019T(A) + SCB-1019T(A) at a weight ratio of 1 : 1, with a dose of 90 µg per antigen. Figure 20B shows the titer values of neutralizing antibodies induced by the bivalent trimerized fusion peptide vaccine against live hRSV A2 and hRSV B18537 viruses. Figure 20C shows pathological scores. Figure 20D shows lung viral load.

The detection method for neutralizing antibodies is shown in Figure 12**,** which shows the titer detection of neutralizing antibodies against the RSV A2 and B18537 strains. The detection results in Figure 20B indicated that, compared with the normal saline (PBS) group, high levels of neutralizing antibodies were induced by vaccination with the SCB-1019T bivalent vaccine in both naive and primed cotton rats.

The pathological evaluation method is shown in Figure 15. The results in Figure 20C indicated that, compared with the PBS group, the SCB-1019T bivalent vaccine significantly reduced the RSV viral load in the lung tissues of primed animals, with no significant increases in lung pathology.

The detection method for cotton rat lung viral load is shown in Figure 15. The results in Figure 20D indicated that, compared with the PBS group, the vaccine in both cases significantly reduced the RSV viral load in the lung tissues of primed animals, indicating a strong in vivo anti-RSV immune effect induced by the vaccine.

The present invention is not intended to be limited in scope to the particular disclosed embodiments, which are provided, for example, to illustrate various aspects of the present invention. Various modifications to the compositions and methods described will become apparent from the description and teachings herein. Such variations may be practiced without departing from the true scope and spirit of the present invention and are intended to fall within the scope of the present invention.

### Sequences

| **SEQ ID NO.** | **Sequence** | **Description** |
|---|---|---|
| 1. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKRKRRFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGKRSNGLPGPIGPPGPRGRTGDAGPVGPPGPPGPPGPPGPPS AGFDFSFLPQPPQEKAHDGGRYYRANDANVVRDRDLEVDTTLKSL SQQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A-F-trimer fusion polypeptide (SCB-N25C_A) prototype with signal peptide |
| 2. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCACRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGKRSNGLPGPIGPPGPRGRTGDAGPVGPPGPPGPPGPPGPPS AGFDFSFLPQPPQEKAHDGGRYYRANDANVVRDRDLEVDTTLKSL SQQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A-F-trimer fusion polypeptide (SCB-N25C_A) with signal peptide (R106C,R108C,R 133C,R135C,R13 6C) |
| 3. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRACRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGKSNGLPGPIGPPGPRGRTGDAGPVGPPGPPGPPGPPGPPSA GFDFSFLPQPPQEKAHDGGRYYRANDANVVRDRDLEVDTTLKSLS QQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A-F-trimer fusion polypeptide (SCB-N25C_A) with signal peptide (R108C,R133C,R 135C,R136C) |
| 4. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGKRSNGLPGPIGPPGPRGRTGDAGPVGPPGPPGPPGPPGPPS AGFDFSFLPQPPQEKAHDGGRYYRANDANVVRDRDLEVDTTLKSL SQQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A V-F-trimer fusion polypeptide (SCB-N25C_A) with signal peptide (R133C,R135C,R 136C) |
| 5. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKRKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGKRSNGLPGPIGPPGPRGRTGDAGPVGPPGPPGPPGPPGPPS AGFDFSFLPQPPQEKAHDGGRYYRANDANVVRDRDLEVDTTLKSL SQQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A-F-trimer fusion polypeptide (SCB-N25C_A) with signal peptide (R135C,R136C) |
| 6. | QNITEEFYQSTCSAVSKGYLSALRTGWYTSVITIELSNIKENKCNGT DAKVKLIKQELDKYKNAVTELQLLMQSTPATNNRARRELPRFMNY TLNNAKKTNVTLSKKRKRRFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGKRSNGLPGPIGPPGPR GRTGDAGPVGPPGPPGPPGPPGPPSAGFDFSFLPQPPQEKAHDGGR YYRANDANVVRDRDLEVDTTLKSLSQQIENIRSPEGSRKNPARTCR DLKMCHSDWKSGEYWIDPNQGCNLDAIKVFCNMETGETCVYPTQ PSVAQKNWYISKNPKDKRHVWFGESMTDGFQFEYGGQGSDPADV AIQLTFLRLMSTEASQNITYHCKNSVAYMDQQTGNLKKALLLQGS NEIEIRAEGNSRFTYSVTVDGCTSHTGAWGKTVIEYKTTKTSRLPIID VAPLDVGAPDQEFGFDVGPVCFL | hRSV_subtype A-F-trimer fusion polypeptide (SCB-N25C_A) prototype without signal peptide |
| 7. | QNITEEFYQSTCSAVSKGYLSALRTGWYTSVITIELSNIKENKCNGT DAKVKLIKQELDKYKNAVTELQLLMQSTPATNNCACRELPRFMNY TLNNAKKTNVTLSKKCKCCFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (SCB-N25C_A) without signal peptide (R106C,R108C,R 133C,R135C,R13 6C) |
| 8. | QNITEEFYQSTCSAVSKGYLSALRTGWYTSVITIELSNIKENKCNGT DAKVKLIKQELDKYKNAVTELQLLMQSTPATNNRACRELPRFMNY TLNNAKKTNVTLSKKCKCCFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METETGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (SCB-N25C_A) without signal peptide (R108C,R133C,R 135C,R136C) |
| 9. | QNITEEFYQSTCSAVSKGYLSALRTGWYTSVITIELSNIKENKCNGT DAKVKLIKQELDKYKNAVTELQLLMQSTPATNNRARRELPRFMNY TLNNAKKTNVTLSKKCKCCFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKT KCTASNKNRGIIKTFSNGCDYVSNKGVDTVSVGNTLYYVNKQEGK SLYVKGEPIINFYDPLVFPSDEFDASISQVNEKINQSLAFIRKSDELLH NVNAGKRSNGLPGPIGPPGPRGRTGDAGPVGPPGPPGPPGPPGPPSA GFDFSFLPQPPQEKAHDGGRYYRANDANVVRDRDLEVDTTLKSLS QQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A-F-trimer fusion polypeptide (SCB-N25C_A) without signal peptide (R133C,R135C,R 136C) |
| 10. | QNITEEFYQSTCSAVSKGYLSALRTGWYTSVITIELSNIKENKCNGT DAKVKLIKQELDKYKNAVTELQLLMQSTPATNNRARRELPRFMNY TLNNAKKTNVTLSKKRKCCFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKT KCTASNKNRGIIKTFSNGCDYVSNKGVDTVSVGNTLYYVNKQEGK SLYVKGEPIINFYDPLVFPSDEFDASISQVNEKINQSLAFIRKSDELLH NVNAGKRSNGLPGPIGPPGPRGRTGDAGPVGPPGPPGPPGPPGPPSA GFDFSFLPQPPQEKAHDGGRYYRANDANVVRDRDLEVDTTLKSLS QQIENIRSPEGSRKNPARTCRDLKMCHSDWKSGEYWIDPNQGCNL DAIKVFCNMETGETCVYPTQPSVAQKNWYISKNPKDKRHVWFGES MTDGFQFEYGGQGSDPADVAIQLTFLRLMSTEASQNITYHCKNSV AYMDQQTGNLKKALLLQGSNEIEIRAEGNSRFTYSVTVDGCTSHTG AWGKTVIEYKTTKTSRLPIIDVAPLDVGAPDQEFGFDVGPVCFL | hRSV_subtype A-F-trimer fusion polypeptide (SCB-N25C_A) without signal peptide (R135C,R136C) |
| 11. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKRKRRFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYV VQLPLYGVIDTPCWKLHTSPLCTTNTKEGSNICLTRTDRGWYCDNA GSVSFFPQAETCKVQSNRVFCDTMNSLTLPSEVNLCNVDIFNPKYD CKIMTSKTDVSSSVITSLGAIVSCYGKTKCTASNKNRGIIKTFSNGC DYVSNKGVDTVSVGNTLYYVNKQEGKSLYVKGEPIINFYDPLVFPS DEFDASISQVNEKINQSLAFIRKSDELLHNVNAGK | hRSV_subtype A-F extracellular domain antigen (SCB-N25C_A) prototype with signal peptide |
| 12. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCACRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK | hRSV_subtype A-F extracellular domain antigen (SCB-N25C_A) with signal peptide (R106C,R108C,R 133C,R135C,R13 6C) |
| 13. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRACRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK | hRSV_subtype A-F extracellular domain antigen (SCB-N25C_A) with signal peptide (R108C,R133C,R 135C,R136C) |
| 14. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK | hRSV_subtype A-F extracellular domain antigen (SCB-N25C_A) with signal peptide (R133C,R135C,R 136C) |
| 15. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKRKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK | hRSV_subtype A-F extracellular domain antigen (SCB-N25C_A) with signal peptide (R135C,R136C) |
| 16. | QNITEEFYQSTCSAVSKGYLSALRTGWYTSVITIELSNIKENKCNGT DAKVKLIKQELDKYKNAVTELQLLMQSTPATNNRARRELPRFMNY TLNNAKKTNVTLSKKRKRRFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGK | hRSV_subtype A-F extracellular domain antigen (SCB-N25C_A) prototype without signal peptide |
| 17. | QNITEEFYQSTCSAVSKGYLSALRTGWYTSVITIELSNIKENKCNGT DAKVKLIKQELDKYKNAVTELQLLMQSTPATNNCACRELPRFMNY TLNNAKKTNVTLSKKCKCCFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGK | hRSV_subtype A-F extracellular domain antigen (SCB-N25C_A) without signal peptide (R106C,R108C,R 133C,R135C,R13 6C) |
| 18. | QNITEEFYQSTCSAVSKGYLSALRTGWYTSVITIELSNIKENKCNGT DAKVKLIKQELDKYKNAVTELQLLMQSTPATNNRACRELPRFMNY TLNNAKKTNVTLSKKCKCCFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGK | hRSV_subtype A-F extracellular domain antigen (SCB-N25C_A) without signal peptide (R108C,R133C,R 135C,R136C) |
| 19. | QNITEEFYQSTCSAVSKGYLSALRTGWYTSVITIE LSNIKENKCNGTDAKVKLIKQELDKYKNAVTELQLLMQSTPATNN RARRELPRFMNYTLNNAKKTNVTLSKKCKCCFLGFLLGVGSAIAS GVAVSKVLHLEGEVNKIKSALLSTNKAVVS LSNGVSVLTSKVLDLKNYIDKQLLPIVNKQSCSISNIATVIEFQQKN NRLLEITREFSVNAGVTTPVSTYMLTNSELLSLINDMPITNDQKKLM SNNVQIVRQQSYSIMSIIKEEVLAYV VQLPLYGVIDTPCWKLHTSPLCTTNTKEGSNICLTRTDRGWYCDNA GSVSFFPQAETCKVQSNRVFCDTMNSLTLPSEVNLCNVDIFNPKYD CKIMTSKTDVSSSVITSLGAIVSCYGKT KCTASNKNRGIIKTFSNGCDYVSNKGVDTVSVGNTLYYVNKQEGK SLYVKGEPIINFYDPLVFPSDEFDASISQVNEKINQSLAFIRKSDELLH NVNAGK | hRSV_subtype A-F extracellular domain antigen (SCB-N25C_A) without signal peptide (R133C,R135C,R 136C) |
| 20. | QNITEEFYQSTCSAVSKGYLSALRTGWYTSVITIE LSNIKENKCNGTDAKVKLIKQELDKYKNAVTELQLLMQSTPATNN RARRELPRFMNYTLNNAKKTNVTLSKKRKCCFLGFLLGVGSAIAS GVAVSKVLHLEGEVNKIKSALLSTNKAVVS LSNGVSVLTSKVLDLKNYIDKQLLPIVNKQSCSISNIATVIEFQQKN NRLLEITREFSVNAGVTTPVSTYMLTNSELLSLINDMPITNDQKKLM SNNVQIVRQQSYSIMSIIKEEVLAYV VQLPLYGVIDTPCWKLHTSPLCTTNTKEGSNICLTRTDRGWYCDNA GSVSFFPQAETCKVQSNRVFCDTMNSLTLPSEVNLCNVDIFNPKYD CKIMTSKTDVSSSVITSLGAIVSCYGKT KCTASNKNRGIIKTFSNGCDYVSNKGVDTVSVGNTLYYVNKQEGK SLYVKGEPIINFYDPLVFPSDEFDASISQVNEKINQSLAFIRKSDELLH NVNAGK | hRSV_subtype A-F extracellular domain antigen (SCB-N25C_A) without signal peptide (R135C,R136C) |
| 21. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTINTTKNLNVSISKKRKRRFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYV VQLPIYGVIDTPCWKLHTSPLCTTNIKEGSNICLTRTDRGWYCDNA GSVSFFPQADTCKVQSNRVFCDTMNSLTLPSEVSLCNTDIFNSKYD CKIMTSKTDISSSVITSLGAIVSCYGKTKCTASNKNRGIIKTFSNGCD YVSNKGVDTVSVGNTLYYVNKLEGKNLYVKGEPIINYYDPLVFPS DEFDASISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) prototype with signal peptide |
| 22. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCACREAPQYMNYTIN TTKNLNVSISKKCKCCFLGFLLGVGSAIASGIAVSKVLHLEGEVNKI KNALQLTNKAVVSLSNGVSVLTSRVLDLKNYINNQLLPMVNRQSC RISNIETVIEFQQKNSRLLEITREFSVN AGVTTPLSTYMLTNSELLSLINDMPITNDQKKLMSSNVQIVRQQSY SIMSIIKEEVLAYVVQLPIYGVIDTPCWKLHTSPLCTTNIKEGSNICLT RTDRGWYCDNAGSVSFFPQADTCKVQSNRVFCDTMNSLTLPSEVS LCNTDIFNSKYDCKIMTSKTDISSSVITSLGAIVSCYGKTKCTASNKN RGIIKTFSNGCDYVSNKGVDTVSVGNTLYYVNKLEGKNLYVKGEPI INYYDPLVFPSDEFDASISQVNEKINQSLAFIRRSDELLHNVNTGKRS NGLPGPIGPPGPRGRTGDAGPVGPPGPPGPPGPPGPPSAGFDFSFLPQ PPQEKAHDGGRYYRANDANVVRDRDLEVDTTLKSLSQQIENIRSPE GSRKNPARTCRDLKMCHSDWKSGEYWIDPNQGCNLDAIKVFCNM ETGETCVYPTQPSVAQKNWYISKNPKDKRHVWFGESMTDGFQFEY GGQGSDPADVAIQLTFLRLMSTEASQNITYHCKNSVAYMDQQTGN LKKALLLQGSNEIEIRAEGNSRFTYSVTVDGCTSHTGAWGKTVIEY KTTKTSRLPIIDVAPLDVGAPDQEFGFDVGPVCFL | hRSV_subtype B-F-trimer fusion polypeptide (SCB-N25C_B) with signal peptide (R106C,R108C,R 133C,R135C,R13 6C) |
| 23. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRACREAPQYMNYTINTTKNLNVSISKKCKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) with signal peptide (R108C,R133C,R 135C,R136C) |
| 24. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTINTTKNLNVSISKKCKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) with signal peptide (R133C,R135C,R 136C) |
| 25. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTINTTKNLNVSISKKRKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) with signal peptide (R135C,R136C) |
| 26. | QNITEEFYQSTCSAVSRGYLSALRTGWYTSVITIELSNIKETKCNGT DTKVKLIKQELDKYKNAVTELQLLMQNTPAVNNRARREAPQYMN YTINTTKNLNVSISKKRKRRFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) prototype without signal peptide |
| 27. | QNITEEFYQSTCSAVSRGYLSALRTGWYTSVITIELSNIKETKCNGT DTKVKLIKQELDKYKNAVTELQLLMQNTPAVNNCACREAPQYMN YTINTTKNLNVSISKKCKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVN AGVTTPLSTYMLTNSELLSLINDMPITNDQKKLMSSNVQIVRQQSY SIMSIIKEEVLAYVVQLPIYGVIDTPCWKLHTSPLCTTNIKEGSNICLT RTDRGWYCDNAGSVSFFPQADTCKVQSNRVFCDTMNSLTLPSEVS LCNTDIFNSKYDCKIMTSKTDISSSVITSLGAIVSCYGKTKCTASNKN RGIIKTFSNGCDYVSNKGVDTVSVGNTLYYVNKLEGKNLYVKGEPI INYYDPLVFPSDEFDASISQVNEKINQSLAFIRRSDELLHNVNTGKRS NGLPGPIGPPGPRGRTGDAGPVGPPGPPGPPGPPGPPSAGFDFSFLPQ PPQEKAHDGGRYYRANDANVVRDRDLEVDTTLKSLSQQIENIRSPE GSRKNPARTCRDLKMCHSDWKSGEYWIDPNQGCNLDAIKVFCNM ETGETCVYPTQPSVAQKNWYISKNPKDKRHVWFGESMTDGFQFEY GGQGSDPADVAIQLTFLRLMSTEASQNITYHCKNSVAYMDQQTGN LKKALLLQGSNEIEIRAEGNSRFTYSVTVDGCTSHTGAWGKTVIEY KTTKTSRLPIIDVAPLDVGAPDQEFGFDVGPVCFL | hRSV_subtype B-F-trimer fusion polypeptide (SCB-N25C_B) without signal peptide (R106C,R108C,R 133C,R135C,R13 6C) |
| 28. | QNITEEFYQSTCSAVSRGYLSALRTGWYTSVITIELSNIKETKCNGT DTKVKLIKQELDKYKNAVTELQLLMQNTPAVNNRACREAPQYMN YTINTTKNLNVSISKKCKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) without signal peptide (R108C,R133C,R 135C,R136C) |
| 29. | QNITEEFYQSTCSAVSRGYLSALRTGWYTSVITIELSNIKETKCNGT DTKVKLIKQELDKYKNAVTELQLLMQNTPAVNNRARREAPQYMN YTINTTKNLNVSISKKCKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) without signal peptide (R133C,R135C,R 136C) |
| 30. | QNITEEFYQSTCSAVSRGYLSALRTGWYTSVITIELSNIKETKCNGT DTKVKLIKQELDKYKNAVTELQLLMQNTPAVNNRARREAPQYMN YTINTTKNLNVSISKKRKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (SCB-N25C_B) without signal peptide (R135C,R136C) |
| 31. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTINTTKNLNVSISKKRKRRFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMIIKEEVLAYV VQLPIYGVIDTPCWKLHTSPLCTTNIKEGSNICLTRTDRGWYCDNA GSVSFFPQADTCKVQSNRVFCDTMNSLTLPSEVSLCNTDIFNSKYD CKIMTSKTDISSSVITSLGAIVSCYGKTKCTASNKNRGIIKTFSNGCD YVSNKGVDTVSVGNTLYYVNKLEGKNLYVKGEPIINYYDPLVFPS DEFDASISQVNEKINQSLAFIRRSDELLHNVNTGK | hRSV_subtype B-F extracellular domain antigen (SCB-N25C_B) prototype with signal peptide |
| 32. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCACREAPQYMNYTINTTKNLNVSISKKCKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDP LVFPSDEFDASISQVNEKINQSLAFIRRSDELLHNVNTGK | hRSV_subtype B-F extracellular domain antigen (SCB-N25C_B) with signal peptide (R106C,R108C,R 133C,R135C,R13 6C) |
| 33. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRACREAPQYMNYTINTTKNLNVSISKKCKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK | hRSV_subtype B-F extracellular domain antigen (SCB-N25C_B) with signal peptide (R108C,R133C,R 135C,R136C) |
| 34. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTIN TTKNLNVSISKKCKCCFLGFLLGVGSAIASGIAVSKVLHLEGEVNKI KNALQLTNKAVVSLSNGVSVLTSRVLDLKNYINNQLLPMVNRQSC RISNIETVIEFQQKNSRLLEITREFSVNAGVTTPLSTYMLTNSELLSLI NDMPITNDQKKLMSSNVQIVRQQSYSIMSIIKEEVLAYVVQLPIYGV IDTPCWKLHTSPLCTTNIKEGSNICLTRTDRGWYCDNAGSVSFFPQA DTCKVQSNRVFCDTMNSLTLPSEVSLCNTDIFNSKYDCKIMTSKTDI SSSVITSLGAIVSCYGKTKCTASNKNRGIIKTFSNGCDYVSNKGVDT VSVGNTLYYVNKLEGKNLYVKGEPIINYYDPLVFPSDEFDASISQV NEKINQSLAFIRRSDELLHNVNTGK | hRSV_subtype B-F extracellular domain antigen (SCB-N25C_B) with signal peptide (R133C,R135C,R 136C) |
| 35. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTINTTKNLNVSISKKRKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK | hRSV_subtype B-F extracellular domain antigen (SCB-N25C_B) with signal peptide (R135C,R136C) |
| 36. | QNITEEFYQSTCSAVSRGYLSALRTGWYTSVITIELSNIKETKCNGT DTKVKLIKQELDKYKNAVTELQLLMQNTPAVNNRARREAPQYMN YTINTTKNLNVSISKKRKRRFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK | hRSV_subtype B-F extracellular domain antigen (SCB-N25C_B) prototype without signal peptide |
| 37. | QNITEEFYQSTCSAVSRGYLSALRTGWYTSVITIELSNIKETKCNGT DTKVKLIKQELDKYKNAVTELQLLMQNTPAVNNCACREAPQYMN YTINTTKNLNVSISKKCKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK | hRSV_subtype B-F extracellular domain antigen (SCB-N25C_B) without signal peptide (R106C,R108C,R 133C,R135C,R13 6C) |
| 38. | QNITEEFYQSTCSAVSRGYLSALRTGWYTSVITIELSNIKETKCNGT DTKVKLIKQELDKYKNAVTELQLLMQNTPAVNNRACREAPQYMN YTINTTKNLNVSISKKCKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK | hRSV_subtype B-F extracellular domain antigen (SCB-N25C_B) without signal peptide (R108C,R133C,R 135C,R136C) |
| 39. | QNITEEFYQSTCSAVSRGYLSALRTGWYTSVITIELSNIKETKCNGT DTKVKLIKQELDKYKNAVTELQLLMQNTPAVNNRARREAPQYMN YTINTTKNLNVSISKKCKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK | hRSV_subtype B-F extracellular domain antigen (SCB-N25C_B) without signal peptide (R133C,R135C,R 136C) |
| 40. | QNITEEFYQSTCSAVSRGYLSALRTGWYTSVITIELSNIKETKCNGT DTKVKLIKQELDKYKNAVTELQLLMQNTPAVNNRARREAPQYMN YTINTTKNLNVSISKKRKCCFLGFLLGVGSAIASGIAVSKVLHLEGE VNKIKNALQLTNKAVVSLSNGVSVLTSRVLDLKNYINNQLLPMVN RQSCRISNIETVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK | hRSV_subtype B-F extracellular domain antigen (SCB-N25C_B) without signal peptide (R135C,R136C) |
| 41. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCGGGGSAIASGVAVSKVLHLEGEVNKIKSALLSTN KAVVSLSNGVSVLTSKVLDLKNYIDKQLLPIVNKQSCSISNIATVIEF QQKNNRLLEITREFSVNAGVTTPVSTYMLTNSELLSLINDMPITNDQ KKLMSNNVQIVRQQSYSIMSIIKEEVLAYVVQLPLYGVIDTPCWKL HTSPLCTTNTKEGSNICLT RTDRGWYCDNAGSVSFFPQAETCKVQSNRVFCDTMNSLTLPSEVN LCNVDIFNPKYDCKIMTSKTDVSSSVITSLGAIVSCYGKTKCTASNK NRGIIKTFSNGCDYVSNKGVDTVSVGNTLYYVNKQEGKSLYVKGE PIINFYDPLVFPSDEFDASISQVNEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) with signal peptide, with the linker peptide CGGG |
| 42. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCGGGELPRFMNYTLNNAKKTNVTLSKKRKRRFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) with signal peptide, with the linker peptide CGGG replacing furin site I |
| 43. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCGGGFLGFLLGVGSAIASGVAVSKVLHLEGEVNKI KSALLSTNKAVVSLSNGVSVLTSKVLDLKNYIDKQLLPIVNKQSCSI SNIATVIEFQQKNNRLLEITREFSVNAGVTTPVSTYMLTNSELLSLIN DMPITNDQKKLMSNNVQIVRQQSYSIMSIIKEEVLAYVVQLPLYGVI DTPCWKLHTSPLCTTNTKEGSNICLTRTDRGWYCDNAGSVSFFPQA ETCKVQSNRVFCDTMNSLTLPSEVNLCNVDIFNPKYDCKIMTSKTD VSSSVITSLGAIVSCYGKTKCTASNKNRGIIKTFSNGCDYVSNKGVD TVSVGNTLYYVNKQEGKSLYVKGEPIINFYDPLVFPSDEFDASISQV NEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) with signal peptide, with the linker peptide CGGG replacing furin site I + pep27 peptide |
| 44. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRCGGGFLGFLLGVGSAIASGVAVSKVLHLEG EVNKIKSALLSTNKAVVSLSNGVSVLTSKVLDLKNYIDKQLLPIVN KQSCSISNIATVIEFQQKNNRLLEITREFSVNAGVTTPVSTYMLTNSE LLSLINDMPITNDQKKLMSNNVQIVRQQSYSIMSIIKEEVLAYVVQL PLYGVIDTPCWKLHTSPLCTTNTKEGSNICLTRTDRGWYCDNAGSV SFFPQAETCKVQSNRVFCDTMNSLTLPSEVNLCNVDIFNPKYDCKI MTSKTDVSSSVITSLGAIVSCYGKTKCTASNKNRGIIKTFSNGCDYV SNKGVDTVSVGNTLYYVNKQEGKSLYVKGEPIINFYDPLVFPSDEF DASISQVNEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) with signal peptide, with the linker peptide CGGG replacing pep27 peptide |
| 45. | QNITEEFYQSTCSAVSKGYLSALRTGWYTSVITIELSNIKENKCNGT DAKVKLIKQELDKYKNAVTELQLLMQSTPATNNCGGGGSAIASGV AVSKVLHLEGEVNKIKSALLSTNKAVVSLSNGVSVLTSKVLDLKN YIDKQLLPIVNKQSCSISNIATVIEFQQKNNRLLEITREFSVNAGVTT PVSTYMLTNSELLSLINDMPITNDQKKLMSNNVQIVRQQSYSIMSII KEEVLAYVVQLPLYGVIDTPCWKLHTSPLCTTNTKEGSNICLTRTD RGWYCDNAGSVSFFPQAETCKVQSNRVFCDTMNSLTLPSEVNLCN VDIFNPKYDCKIMTSKTDVSSSVITSLGAIVSCYGKTKCTASNKNRG IIKTFSNGCDYVSNKGVDTVSVGNTLYYVNKQEGKSLYVKGEPIIN FYDPLVFPSDEFDASISQVNEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) without signal peptide, with the linker peptide CGGG |
| 46. | QNITEEFYQSTCSAVSKGYLSALRTGWYTSVITIELSNIKENKCNGT DAKVKLIKQELDKYKNAVTELQLLMQSTPATNNCGGGELPRFMN YTLNNAKKTNVTLSKKRKRRFLGFLLGVGSAIASGVAVSKVLHLE GEVNKIKSALLSTNKAVVSLSNGVSVLTSKVLDLKNYIDKQLLPIV NKQSCSISNIATVIEFQQKNNRLLEITREFSVNAGVTTPVSTYMLTNS ELLSLINDMPITNDQKKLMSNNVQIVRQQSYSIMSIIKEEVLAYVVQ LPLYGVIDTPCWKLHTSPLCTTNTKEGSNICLTRTDRGWYCDNAGS VSFFPQAETCKVQSNRVFCDTMNSLTLPSEVNLCNVDIFNPKYDCK IMTSKTDVSSSVITSLGAIVSCYGKTKCTASNKNRGIIKTFSNGCDY VSNKGVDTVSVGNTLYYVNKQEGKSLYVKGEPIINFYDPLVFPSDE FDASISQVNEKINQSLAFIRKSDELLHNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) without signal peptide, with the linker peptide CGGG replacing furin site I |
| 47. | QNITEEFYQSTCSAVSKGYLSALRTGWYTSVITIELSNIKENKCNGT DAKVKLIKQELDKYKNAVTELQLLMQSTPATNNCGGGFLGFLLGV GSAIASGVAVSKVLHLEGEVNKIKSALLSTNKAVVSLSNGVSVLTS KVLDLKNYIDKQLLPIVNKQSCSISNIATVIEFQQKNNRLLEITREFS VNAGVTTPVSTYMLTNSELLSLINDMPITNDQKKLMSNNVQIVRQQ SYSIMSIIKEEVLAYVVQLPLYGVIDTPCWKLHTSPLCTTNTKEGSNI CLTRTDRGWYCDNAGSVSFFPQAETCKVQSNRVFCDTMNSLTLPS EVNLCNVDIFNPKYDCKIMTSKTDVSSSVITSLGAIVSCYGKTKCTA SNKNRGIIKTFSNGCDYVSNKGVDTVSVGNTLYYVNKQEGKSLYV KGEPIINFYDPLVFPSDEFDASISQVNEKINQSLAFIRKSDELLHNVN AGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) without signal peptide, with the linker peptide CGGG replacing furin site I + pep27 peptide |
| 48. | QNITEEFYQSTCSAVSKGYLSALRTGWYTSVITIELSNIKENKCNGT DAKVKLIKQELDKYKNAVTELQLLMQSTPATNNRARRCGGGFLGF LLGVGSAIASGVAVSKVLHLEGEVNKIKSALLSTNKAVVSLSNGVS VLTSKVLDLKNYIDKQLLPIVNKQSCSISNIATVIEFQQKNNRLLEIT REFSVNAGVTTPVSTYMLTNSELLSLINDMPITNDQKKLMSNNVQI VRQQSYSIMSIIKEEVLAYVVQLPLYGVIDTPCWKLHTSPLCTTNTK EGSNICLTRTDRGWYCDNAGSVSFFPQAETCKVQSNRVFCDTMNS LTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype A-F-trimer fusion polypeptide (3.1SCB-N25C_A) without signal peptide, with the linker peptide CGGG replacing pep27 peptide |
| 49. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCGGGGSAIASGVAVSKVLHLEGEVNKIKSALLSTN KAVVSLSNGVSVLTSKVLDLKNYIDKQLLPIVNKQSCSISNIATVIEF QQKNNRLLEITREFSVNAGVTTPVSTYMLTNSELLSLINDMPITNDQ KKLMSNNVQIVRQQSYSIMSIIKEEVLAYVVQLPLYGVIDTPCWKL HTSPLCTTNTKEGSNICLT RTDRGWYCDNAGSVSFFPQAETCKVQSNRVFCDTMNSLTLPSEVN LCNVDIFNPKYDCKIMTSKTDVSSSVITSLGAIVSCYGKTKCTASNK NRGIIKTFSNGCDYVSNKGVDTVSVGNTLYYVNKQEGKSLYVKGE PIINFYDPLVFPSDEFDASISQVNEKINQSLAFIRKSDELLHNVNAGK | hRSV_subtype A-fusion glycoprotein F extracellular domain antigen (3.1SCB-N25C_A) with signal peptide, with the linker peptide CGGG |
| 50. | QNITEEFYQSTCSAVSKGYLSALRTGWYTSVITIELSNIKENKCNGT DAKVKLIKQELDKYKNAVTELQLLMQSTPATNNCGGGGSAIASGV AVSKVLHLEGEVNKIKSALLSTNKAVVSLSNGVSVLTSKVLDLKN YIDKQLLPIVNKQSCSISNIATVIEFQQKNNRLLEITREFSVNAGVTT PVSTYMLTNSELLSLINDMPITNDQKKLMSNNVQIVRQQSYSIMSII KEEVLAYVVQLPLYGVIDTPCWKLHTSPLCTTNTKEGSNICLTRTD RGWYCDNAGSVSFFPQAETCKVQSNRVFCDTMNSLTLPSEVNLCN VDIFNPKYDCKIMTSKTDVSSSVITSLGAIVSCYGKTKCTASNKNRG IIKTFSNGCDYVSNKGVDTVSVGNTLYYVNKQEGKSLYVKGEPIIN FYDPLVFPSDEFDASISQVNEKINQSLAFIRKSDELLHNVNAGK | hRSV_subtype A-fusion glycoprotein F extracellular domain antigen (3.1SCB-N25C_A) without signal peptide, with the linker peptide CGGG |
| 51. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNN | hRSV_subtype A-fusion glycoprotein F2 fragment (3.1SCB-N25C_A) with signal peptide |
| 52. | QNITEEFYQSTCSAVSKGYLSALRTGWYTSVITIELSNIKENKCNGT DAKVKLIKQELDKYKNAVTELQLLMQSTPATNN | hRSV_subtype A-fusion glycoprotein F2 fragment (3.1SCB-N25C_A) without signal peptide |
| 53. | GSAIASGVAVSKVLHLEGEVNKIKSALLSTNKAVVSLSNGVSVLTS KVLDLKNYIDKQLLPIVNKQSCSISNIATVIEFQQKNNRLLEITREFS VNAGVTTPVSTYMLTNSELLSLINDMPITNDQKKLMSNNVQIVRQQ SYSIMSIIKEEVLAYVVQLPLYGVIDTPCWKLHTSPLCTTNTKEGSNI CLTRTDRGWYCDNAGSVSFFPQAETCKVQSNRVFCDTMNSLTLPS EVNLCNVDIFNPKYDCKIMTSKTDVSSSVITSLGAIVSCYGKTKCTA SNKNRGIIKTFSNGCDYVSNKGVDTVSVGNTLYYVNKQEGKSLYV KGEPIINFYDPLVFPSDEFDASISQVNEKINQSLAFIRKSDELLHNVN AGK | hRSV_subtype A-fusion glycoprotein F1 fragment (3.1SCB-N25C_A) |
| 54. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCGGGGSAIASGIAVSKVLHLEGEVNKIKNALQLT NKAVVSLSNGVSVLTSRVLDLKNYINNQLLPMVNRQSCRISNIATVI EFQQKNSRLLEITREFSVNAGVTTPLSTYMLTNSELLSLINDMPITN DQKKLMSSNVQIVRQQSYSIMSIIKEEVLAYVVQLPIYGVIDTPCWK LHTSPLCTTNIKEGSNICLT RTDRGWYCDNAGSVSFFPQADTCKVQSNRVFCDTMNSLTLPSEVS LCNTDIFNSKYDCKIMTSKTDISSSVITSLGAIVSCYGKTKCTASNKN RGIIKTFSNGCDYVSNKGVDTVSVGNTLYYVNKLEGKNLYVKGEPI INYYDPLVFPSDEFDASISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) with signal peptide, with the linker peptide CGGG |
| 55. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCGGGEAPQYMNYTIN121TTKNLNVSISKKRKRRF LGFLLGVGSAIASGIAVSKVLHLEGEVNKIKNALQLTNKAVVSLSN GVSVLTSRVLDLKNYINNQLLPMVNRQSCRISNIETVIEFQQKNSRL LEITREFSVNAGVTTPLSTYMLTNSELLSLINDMPITNDQKKLMSSN VQIVRQQSYSIMSIIKEEVLAYVVQLPIYGVIDTPCWKLHTSPLCTTN IKEGSNICLTRTDRGWYCDNAGSVSFFPQADTCKVQSNRVFCDTM NSLTLPSEVSLCNTDIFNSKYDCKIMTSKTDISSSVITSLGAIVSCYG KTKCTASNKNRGIIKTFSNGCDYVSNKGVDTVSVGNTLYYVNKLE GKNLYVKGEPIINYYDPLVFPSDEFDASISQVNEKINQSLAFIRRSDE LLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) with signal peptide, with the linker peptide CGGG replacing furin site I |
| 56. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCGGGFLGFLLGVGSAIASGIAVSKVLHLEGEVNKI KNALQLTNKAVVSLSNGVSVLTSRVLDLKNYINNQLLPMVNRQSC RISNIATVIEFQQKNSRLLEITREFSVNAGVTTPLSTYMLTNSELLSLI NDMPITNDQKKLMSSNVQIVRQQSYSIMSIIKEEVLAYVVQLPIYGV IDTPCWKLHTSPLCTTNIKEGSNICLTRTDRGWYCDNAGSVSFFPQA DTCKVQSNRVFCDTMNSLTLPSEVSLCNTDIFNSKYDCKIMTSKTDI SSSVITSLGAIVSCYGKTKCTASNKNRGIIKTFSNGCDYVSNKGVDT VSVGNTLYYVNKLEGKNLYVKGEPIINYYDPLVFPSDEFDASISQV NEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) with signal peptide, with the linker peptide CGGG replacing furin site I + pep27 peptide |
| 57. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARRCGGGFLGFLLGVGSAIASGIAVSKVLHLEGE VNKIKNALQLTNKAVVSLSNGVSVLTSRVLDLKNYINNQLLPMVN RQSCRISNIATVIEFQQKNSRLLEITREFSVNAGVTTPLSTYMLTNSE LLSLINDMPITNDQKKLMSSNVQIVRQQSYSIMSIIKEEVLAYVVQL PIYGVIDTPCWKLHTSPLCTTNIKEGSNICLTRTDRGWYCDNAGSVS FFPQADTCKVQSNRVFCDTMNSLTLPSEVSLCNTDIFNSKYDCKIM TSKTDISSSVITSLGAIVSCYGKTKCTASNKNRGIIKTFSNGCDYVSN KGVDTVSVGNTLYYVNKLEGKNLYVKGEPIINYYDPLVFPSDEFDA SISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) with signal peptide, with the linker peptide CGGG replacing pep27 peptide |
| 58. | QNITEEFYQSTCSAVSRGYLSALRTGWYTSVITIELSNIKETKCNGT DTKVKLIKQELDKYKNAVTELQLLMQNTPAVNNCGGGGSAIASGI AVSKVLHLEGEVNKIKNALQLTNKAVVSLSNGVSVLTSRVLDLKN YINNQLLPMVNRQSCRISNIATVIEFQQKNSRLLEITREFSVNAGVTT PLSTYMLTNSELLSLINDMPITNDQKKLMSSNVQIVRQQSYSIMSIIK EEVLAYVVQLPIYGVIDTPCWKLHTSPLCTTNIKEGSNICLTRTDRG WYCDNAGSVSFFPQADTCKVQSNRVFCDTMNSLTLPSEVSLCNTDI FNSKYDCKIMTSKTDISSSVITSLGAIVSCYGKTKCTASNKNRGIIKT FSNGCDYVSNKGVDTVSVGNTLYYVNKLEGKNLYVKGEPIINYYD PLVFPSDEFDASISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) without signal peptide, with the linker peptide CGGG |
| 59. | QNITEEFYQSTCSAVSRGYLSALRTGWYTSVITIELSNIKETKCNGT DTKVKLIKQELDKYKNAVTELQLLMQNTPAVNNCGGGEAPQYMN YTIN121TTKNLNVSISKKRKRRFLGFLLGVGSAIASGIAVSKVLHLE GEVNKIKNALQLTNKAVVSLSNGVSVLTSRVLDLKNYINNQLLPM VNRQSCRISNIATVIEFQQKNSRLLEITREFSVNAGVTTPLSTYMLTN SELLSLINDMPITNDQKKLMSSNVQIVRQQSYSIMSIIKEEVLAYVV QLPIYGVIDTPCWKLHTSPLCTTNIKEGSNICLTRTDRGWYCDNAGS VSFFPQADTCKVQSNRVFCDTMNSLTLPSEVSLCNTDIFNSKYDCKI MTSKTDISSSVITSLGAIVSCYGKTKCTASNKNRGIIKTFSNGCDYVS NKGVDTVSVGNTLYYVNKLEGKNLYVKGEPIINYYDPLVFPSDEFD ASISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) without signal peptide, with the linker peptide CGGG replacing furin site I |
| 60. | QNITEEFYQSTCSAVSRGYLSALRTGWYTSVITIELSNIKETKCNGT DTKVKLIKQELDKYKNAVTELQLLMQNTPAVNNCGGGFLGFLLGV GSAIASGIAVSKVLHLEGEVNKIKNALQLTNKAVVSLSNGVSVLTS RVLDLKNYINNQLLPMVNRQSCRISNIATVIEFQQKNSRLLEITREFS VNAGVTTPLSTYMLTNSELLSLINDMPITNDQKKLMSSNVQIVRQQ SYSIMSIIKEEVLAYVVQLPIYGVIDTPCWKLHTSPLCTTNIKEGSNI CLTRTDRGWYCDNAGSVSFFPQADTCKVQSNRVFCDTMNSLTLPS EVSLCNTDIFNSKYDCKIMTSKTDISSSVITSLGAIVSCYGKTKCTAS NKNRGIIKTFSNGCDYVSNKGVDTVSVGNTLYYVNKLEGKNLYVK GEPIINYYDPLVFPSDEFDASISQVNEKINQSLAFIRRSDELLHNVNT GK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) without signal peptide, with the linker peptide CGGG replacing furin site I + pep27 peptide |
| 61. | QNITEEFYQSTCSAVSRGYLSALRTGWYTSVITIELSNIKETKCNGT DTKVKLIKQELDKYKNAVTELQLLMQNTPAVNNRARRCGGGFLG FLLGVGSAIASGIAVSKVLHLEGEVNKIKNALQLTNKAVVSLSNGV SVLTSRVLDLKNYINNQLLPMVNRQSCRISNIATVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | hRSV_subtype B-F-trimer fusion polypeptide (3.1SCB-N25C_B) without signal peptide, with the linker peptide CGGG replacing pep27 peptide |
| 62. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCGGGGSAIASGIAVSKVLHLEGEVNKIKNALQLT NKAVVSLSNGVSVLTSRVLDLKNYINNQLLPMVNRQSCRISNIATVI EFQQKNSRLLEITREFSVNAGVTTPLSTYMLTNSELLSLINDMPITN DQKKLMSSNVQIVRQQSYSIMSIIKEEVLAYVVQLPIYGVIDTPCWK LHTSPLCTTNIKEGSNICLT RTDRGWYCDNAGSVSFFPQADTCKVQSNRVFCDTMNSLTLPSEVS LCNTDIFNSKYDCKIMTSKTDISSSVITSLGAIVSCYGKTKCTASNKN RGIIKTFSNGCDYVSNKGVDTVSVGNTLYYVNKLEGKNLYVKGEPI INYYDPLVFPSDEFDASISQVNEKINQSLAFIRRSDELLHNVNTGK | hRSV_subtype B-fusion glycoprotein F extracellular domain antigen (3.1SCB-N25C_B) with signal peptide, with the linker peptide CGGG |
| 63. | QNITEEFYQSTCSAVSRGYLSALRTGWYTSVITIELSNIKETKCNGT DTKVKLIKQELDKYKNAVTELQLLMQNTPAVNNCGGGGSAIASGI AVSKVLHLEGEVNKIKNALQLTNKAVVSLSNGVSVLTSRVLDLKN YINNQLLPMVNRQSCRISNIATVIEFQQKNSRLLEITREFSVNAGVTT PLSTYMLTNSELLSLINDMPITNDQKKLMSSNVQIVRQQSYSIMSIIK EEVLAYVVQLPIYGVIDTPCWKLHTSPLCTTNIKEGSNICLTRTDRG WYCDNAGSVSFFPQADTCKVQSNRVFCDTMNSLTLPSEVSLCNTDI FNSKYDCKIMTSKTDISSSVITSLGAIVSCYGKTKCTASNKNRGIIKT FSNGCDYVSNKGVDTVSVGNTLYYVNKLEGKNLYVKGEPIINYYD PLVFPSDEFDASISQVNEKINQSLAFIRRSDELLHNVNTGK | hRSV_subtype B-fusion glycoprotein F extracellular domain antigen (3.1SCB-N25C_B) without signal peptide, with the linker peptide CGGG |
| 64. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNN | hRSV_subtype B-fusion glycoprotein F2 fragment (3.1SCB-N25C_B) with signal peptide |
| 65. | QNITEEFYQSTCSAVSRGYLSALRTGWYTSVITIELSNIKETKCNGT DTKVKLIKQELDKYKNAVTELQLLMQNTPAVNN | hRSV_subtype B-fusion glycoprotein F2 fragment (3.1SCB-N25C_B) without signal peptide |
| 66. | GSAIASGIAVSKVLHLEGEVNKIKNALQLTNKAVVSLSNGVSVLTS RVLDLKNYINNQLLPMVNRQSCRISNIATVIEFQQKNSRLLEITREFS VNAGVTTPLSTYMLTNSELLSLINDMPITNDQKKLMSSNVQIVRQQ SYSIMSIIKEEVLAYVVQLPIYGVIDTPCWKLHTSPLCTTNIKEGSNI CLTRTDRGWYCDNAGSVSFFPQADTCKVQSNRVFCDTMNSLTLPS EVSLCNTDIFNSKYDCKIMTSKTDISSSVITSLGAIVSCYGKTKCTAS NKNRGIIKTFSNGCDYVSNKGVDTVSVGNTLYYVNKLEGKNLYVK GEPIINYYDPLVFPSDEFDASISQVNEKINQSLAFIRRSDELLHNVNT GK | hRSV_subtype B-fusion glycoprotein F1 fragment (3.1SCB-N25C_B) |
| 67. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNRARRELPRFMNYTLNNAKKTNVTLSKKRKRRFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYV VQLPLYGVIDTPCWKLHTSPLCTTNTKEGSNICLTRTDRGWYCDNA GSVSFFPQAETCKVQSNRVFCDTMNSLTLPSEVNLCNVDIFNPKYD CKIMTSKTDVSSSVITSLGAIVSCYGKTKCTASNKNRGIIKTFSNGC DYVSNKGVDTVSVGNTLYYVNKQEGKSLYVKGEPIINFYDPLVFPS DEFDASISQVNEKINQSLAFIRKSDELLHNVNAGK STTNIMITTIIIVIIVILLSLIAVGLLLYCKARSTPVTLSKDQLSGINNIA FSN | RSV_subtype A fusion glycoprotein F0, 574aa |
| 68. | MELLILKANAITTILTAVTFCFASGQNITEEFYQSTCSAVSKGYLSAL RTGWYTSVITIELSNIKENKCNGTDAKVKLIKQELDKYKNAVTELQ LLMQSTPATNNCACRELPRFMNYTLNNAKKTNVTLSKKCKCCFLG FLLGVGSAIASGVAVSKVLHLEGEVNKIKSALLSTNKAVVSLSNGV SVLTSKVLDLKNYIDKQLLPIVNKQSCSISNIATVIEFQQKNNRLLEI TREFSVNAGVTTPVSTYMLTNSELLSLINDMPITNDQKKLMSNNVQ IVRQQSYSIMSIIKEEVLAYVVQLPLYGVIDTPCWKLHTSPLCTTNT KEGSNICLTRTDRGWYCDNAGSVSFFPQAETCKVQSNRVFCDTMN SLTLPSEVNLCNVDIFNPKYDCKIMTSKTDVSSSVITSLGAIVSCYGK TKCTASNKNRGIIKTFSNGCDYVSNKGVDTVSVGNTLYYVNKQEG KSLYVKGEPIINFYDPLVFPSDEFDASISQVNEKINQSLAFIRKSDELL HNVNAGK STINIMITTIIIVIIVILLSLIAVGLLLYCKARSTPVTLSKDQLSGINNIA FSN | RSV _subtype A fusion glycoprotein F0, 574aa (R106C,R108C,R 133C,R135C,R13 6C) |
| 69. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNRARREAPQYMNYTINTTKNLNVSISKKRKRRFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK STTNIMITAIIIVIIVVLLSLIAIGLLLYCKAKNTPVTLSKDQLSGINNI AFSK | RSV _subtype B fusion glycoprotein F0 |
| 70. | MELLIHRSSAIFLTLAINALYLTSSQNITEEFYQSTCSAVSRGYLSAL RTGWYTSVITIELSNIKETKCNGTDTKVKLIKQELDKYKNAVTELQ LLMQNTPAVNNCACREAPQYMNYTINTTKNLNVSISKKCKCCFLG FLLGVGSAIASGIAVSKVLHLEGEVNKIKNALQLTNKAVVSLSNGV SVLTSRVLDLKNYINNQLLPMVNRQSCRISNIETVIEFQQKNSRLLEI TREFSVNAGVTTPLSTYMLTNSELLSLINDMPITNDQKKLMSSNVQI VRQQSYSIMSIIKEEVLAYVVQLPIYGVIDTPCWKLHTSPLCTTNIKE GSNICLTRTDRGWYCDNAGSVSFFPQADTCKVQSNRVFCDTMNSL TLPSEVSLCNTDIFNSKYDCKIMTSKTDISSSVITSLGAIVSCYGKTK CTASNKNRGIIKTFSNGCDYVSNKGVDTVSVGNTLYYVNKLEGKN LYVKGEPIINYYDPLVFPSDEFDASISQVNEKINQSLAFIRRSDELLH NVNTGK STTNIMITAIIIVIIVVLLSLIAIGLLLYCKAKNTPVTLSKDQLSGINNI AFSK | RSV _subtype B fusion glycoprotein F0 (R106C,R108C,R 133C,R135C,R13 6C) |
| 71. | MELLILKANAITTILTAVTFCFASG | RSV_subtype A fusion glycoprotein F signal peptide, 25aa (1-25 of SEQ ID NO: 1 or 2) |
| 72. | MELLIHRSSAIFLTLAINALYLTSS | RSV_subtype B fusion glycoprotein F signal peptide, 25aa (1-25 of SEQ ID NO: 21 or 22) |
| 73. | QNITEEFYQSTCSAVSKGYLSALRTGWYTSVITIELSNIKENKCNGT DAKVKLIKQELDKYKNAVTELQLLMQSTPATNNRARR | RSV_subtype A fusion glycoprotein F2 (26-109 of SEQ ID NO: 1) |
| 74. | QNITEEFYQSTCSAVSKGYLSALRTGWYTSVITIELSNIKENKCNGT DAKVKLIKQELDKYKNAVTELQLLMQSTPATNNCACR | RSV_subtype A fusion glycoprotein F2 mutant (26-109 of SEQ ID NO: 2) (R106C,R108C) |
| 75. | RARR | RSV_subtype A&B furin site I (106-109 of SEQ ID NO: 1) |
| 76. | CARR | RSV_subtype A&B furin site I mutant 1 (R106C) |
| 77. | RACR | RSV_subtype A&B furin site I mutant 2 (R108C) |
| 78. | CACR | RSV_subtype A&B furin site I mutant (R106C and R108C) |
| 79. | ELPRFMNYTLNNAKKTNVTLSKKRKRR | RSV_subtype A fusion glycoprotein Fpep27 (110-136 of SEQ ID NO: 1) |
| 80. | ELPRFMNYTLNNAKKTNVTLSKKCKCC | RSV_subtype A fusion glycoprotein Fpep27 mutant (R133C,R135C,R 136C) |
| 81. | EAPQYMNYTINTTKNLNVSISKKRKRR | RSV_subtype B fusion glycoprotein Fpep27 |
| 82. | EAPQYMNYTINTTKNLNVSISKKCKCC | RSV_subtype B fusion glycoprotein Fpep27 mutant (R133C,R135C,R 136C) |
| 83. | KKRKRR | RSV_subtype A&B furin site II (11-16 of SEQ ID NO: 1) |
| 84. | KKCKRR | RSV_subtype A&B furin site II mutant 1 (R133C) |
| 85. | KKRKCR | RSV_subtype A&B furin site II mutant 2 (R135C) |
| 86. | KKRKRC | RSV_subtype A&B furin site II mutant 3 (R136C) |
| 87. | KKCKCR | RSV_subtype A&B furin site II mutant 4 (R133C,R135C) |
| 88. | KKCKRC | RSV_subtype A&B furin site II mutant 5 (R133C,R136C) |
| 89. | KKRKCC | RSV_subtype A&B furin site II mutant 6 (R135C,R136C) |
| 90. | KKCKCC | RSV_subtype A&B furin site II mutant 7 (R133C,R135C,R 136C) |
| 91. | FLGFLLGVGSAIASGVAVS | RSV_subtype A RSV fusion glycoprotein FFP (137-155 of SEQ ID NO: 1 or 2) |
| 92. | FLGFLLGVGSAIASGIAVS | RSV_subtype B RSV fusion glycoprotein FFP (137-155 of SEQ ID NO: 21 or 22) |
| 93. | FLGFLLGV | RSV_subtype A&B RSV fusion glycoprotein F1 fragment, with F2-F1 linker peptide head being substituted |
| 94. | FLGFLLGVGSAIASGVAVSKVLHLEGEVNKIKSALLSTNKAVVSLS NGVSVLTSKVLDLKNYIDKQLLPIVNKQSCSISNIATVIEFQQKNNR LLEITREFSVNAGVTTPVSTYMLTNSELLSLINDMPITNDQKKLMSN NVQIVRQQSYSIMSIIKEEVLAYVVQLPLYGVIDTPCWKLHTSPLCT TNTKEGSNICLTRTDRGWYCDNAGSVSFFPQAETCKVQSNRVFCD TMNSLTLPSEVNLCNVDIFNPKYDCKIMTSKTDVSSSVITSLGAIVS CYGKTKCTASNKNRGIIKTFSNGCDYVSNKGVDTVSVGNTLYYVN KQEGKSLYVKGEPIINFYDPLVFPSDEFDASISQVNEKINQSLAFIRK SDELLHNVNAGKSTIN | RSV_subtype A fusion glycoprotein F1 |
| 95. | FLGFLLGVGSAIASGVAVSKVLHLEGEVNKIKSALLSTNKAVVSLS NGVSVLTSKVLDLKNYIDKQLLPIVNKQSCSISNIATVIEFQQKNNR LLEITREFSVNAGVTTPVSTYMLTNSELLSLINDMPITNDQKKLMSN NVQIVRQQSYSIMSIIKEEVLAYVVQLPLYGVIDTPCWKLHTSPLCT TNTKEGSNICLTRTDRGWYCDNAGSVSFFPQAETCKVQSNRVFCD TMNSLTLPSEVNLCNVDIFNPKYDCKIMTSKTDVSSSVITSLGAIVS CYGKTKCTASNKNRGIIKTFSNGCDYVSNKGVDTVSVGNTLYYVN KQEGKSLYVKGEPIINFYDPLVFPSDEFDASISQVNEKINQSLAFIRK SDELLHNVNAGK | RSV_subtype A fusion glycoprotein F1 fragment |
| 96. | FLGFLLGVGSAIASGIAVSKVLHLEGEVNKIKNALQLTNKAVVS LSNGVSVLTSRVLDLKNYINNQLLPMVNRQSCRISNIETVIEFQQKN SRLLEITREFSVNAGVTTPLSTYMLTNSELLSLINDMPITNDQKKLM SSNVQIVRQQSYSIMSIIKEEVLAYVVQLPIYGVIDTPCWKLHTSPLC TTNIKEGSNICLTRTDRGWYCDNAGSVSFFPQADTCKVQSNRVFCD TMNSLTLPSEVSLCNTDIFNSKYDCKIMTSKTDISSSVITSLGAIVSC YGKTKCTASNKNRGIIKTFSNGCDYVSNKGVDTVSVGNTLYYVNK LEGKNLYVKGEPIINYYDPLVFPSDEFDASISQVNEKINQSLAFIRRS DELLHNVNTGKSTTN | RSV_subtype B fusion glycoprotein F1 |
| 97. | FLGFLLGVGSAIASGIAVSKVLHLEGEVNKIKNALQLTNKAVVS LSNGVSVLTSRVLDLKNYINNQLLPMVNRQSCRISNIETVIEFQQKN SRLLEITREFSVNAGVTTPLSTYMLTNSELLSLINDMPITNDQKKLM SSNVQIVRQQSYSIMSIIKEEVLAYVVQLPIYGVIDTPCWKLHTSPLC TTNIKEGSNICLTRTDRGWYCDNAGSVSFFPQADTCKVQSNRVFCD TMNSLTLPSEVSLCNTDIFNSKYDCKIMTSKTDISSSVITSLGAIVSC YGKTKCTASNKNRGIIKTFSNGCDYVSNKGVDTVSVGNTLYYVNK LEGKNLYVKGEPIINYYDPLVFPSDEFDASISQVNEKINQSLAFIRRS DELLHNVNTGK | RSV_subtype B fusion glycoprotein F1 fragment |
| 98. | IMITTIIIVIIVILLSLIAVGLLLYC | RSV_subtype A fusion glycoprotein F transmembrane domain (525-550 of SEQ ID NO: 67 or 2) |
| 99. | KARSTPVTLSKDQLSGINNIAFSN | RSV_subtype A fusion glycoprotein F cytoplasmic domain (551-574 of SEQ ID NO: 67 or 2) |
| 100. | IMITAIIIVIIVVLLSLIAIGLLLYC | RSV_subtype B fusion glycoprotein F transmembrane domain (525-550 of SEQ ID NO: 69 or 2) |
| 101. | KAKNTPVTLSKDQLSGINNIAFSK | RSV_subtype B fusion glycoprotein F cytoplasmic domain (551-574 of SEQ ID NO: 69) |
| 102. | CGGG | Linker peptide between the fusion glycoproteins F 1 and F2 |
| 103. | ANVVRDRDLEVDTTLKSLSQQIENIRSPEGSRKNPARTCRDLKMCH SDWKSGEYWIDPNQGCNLDAIKVFCNMETGETCVYPTQPSVAQKN WYISKNPKDKRHVWFGESMTDGFQFEYGGQGSDPADVAIQLTFLR LMSTEASQNITYHCKNSVAYMDQQTGNLKKALLLQGSNEIEIRAEG NSRFTYSVTVDGCTSHTGAWGKTVIEYKTTKTSRLPIIDVAPLDVG APDQEFGFDVGPVCFL | Trimerization peptide (type I), QT version |
| 104. | RSANVVRDRDLEVDTTLKSLSQQIENIRSPEGSRKNPARTCRDLKM CHSDWKSGEYWIDPNQGCNLDAIKVFCNMETGETCVYPTQPSVAQ KNWYISKNPKDKRHVWFGESMTDGFQFEYGGQGSDPADVAIQLTF LRLMSTEASQNITYHCKNSVAYMDQQTGNLKKALLLQGSNEIEIRA EGNSRFTYSVTVDGCTSHTGAWGKTVIEYKTTKTSRLPIIDVAPLD VGAPDQEFGFDVGPVCFL | Trimerization peptide (type I), QT version |
| 105. | NGLPGPIGPPGPRGRTGDAGPVGPPGPPGPPGPPSAGFDFSFLPQ PPQEKAHDGGRYYRANDANVVRDRDLEVDTTLKSLSQQIENIRSPE GSRKNPARTCRDLKMCHSDWKSGEYWIDPNQGCNLDAIKVFCNM ETGETCVYPTQPSVAQKNWYISKNPKDKRHVWFGESMTDGFQFEY GGQGSDPADVAIQLTFLRLMSTEASQNITYHCKNSVAYMDQQTGN LKKALLLQGSNEIEIRAEGNSRFTYSVTVDGCTSHTGAWGKTVIEY KTTKTSRLPIIDVAPLDVGAPDQEFGFDVGPVCFL | Trimerization peptide (type I) with glycine-X-Y repeats and D→N mutation at BMP-1 site, QT version |
| 106. | NGLPGPIGPPGPRGRTGDAGPVGPPGPPGPPGPPGPPSAGFDFSFLPQ PPQEKAHDGGRYYRNDDANVVRDRDLEVDTTLKSLSQQIENIRSPE GSRKNPARTCRDLKMCHSDWKSGEYWIDPNQGCNLDAIKVFCNM ETGETCVYPTQPSVAQKNWYISKNPKDKRHVWFGESMTDGFQFEY GGQGSDPADVAIQLTFLRLMSTEASQNITYHCKNSVAYMDQQTGN LKKALLLQGSNEIEIRAEGNSRFTYSVTVDGCTSHTGAWGKTVIEY KTTKTSRLPIIDVAPLDVGAPDQEFGFDVGPVCFL | Trimerization peptide (type I) with glycine-X-Y repeats and A→N mutation at BMP-1 site, QT version |
| 107. | RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | Trimerization peptide (type I) with glycine-X-Y repeats and D→N mutation at BMP-1 site, QT version |
| 108. | GSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVI EYKTTKTSRLPIIDVAPLDVGAPDQEFGFDVGPVCFL | Trimerization peptide (type I) with glycine-X-Y repeats and D→N mutation at BMP-1 site, QT version |
| 109. | ANVVRDRDLEVDTTLKSLSQQIENIRSPEGSRKNPARTCRDLKMCH SDWKSGEYWIDPNQGCNLDAIKVFCNMETGETCVYPTQPSVAQKN WYISKNPKDKRHVWFGESMTDGFQFEYGGQGSDPADVAIQLTFLR LMSTEASQNITYHCKNSVAYMDQQTGNLKKALLLKGSNEIEIRAEG NSRFTYSVTVDGCTSHTGAWGKTVIEYKTTKSSRLPIIDVAPLDVG APDQEFGFDVGPVCFL | Trimerization peptide (type I), KS version |
| 110. | RSANVVRDRDLEVDTTLKSLSQQIENIRSPEGSRKNPARTCRDLKM CHSDWKSGEYWIDPNQGCNLDAIKVFCNMETGETCVYPTQPSVAQ KNWYISKNPKDKRHVWFGESMTDGFQFEYGGQGSDPADVAIQLTF LRLMSTEASQNITYHCKNSVAYMDQQTGNLKKALLLKGSNEIEIRA EGNSRFTYSVTVDGCTSHTGAWGKTVIEYKTTKSSRLPIIDVAPLDV GAPDQEFGFDVGPVCFL | Trimerization peptide (type I), KS version |
| 111. | NGLPGPIGPPGPRGRTGDAGPVGPPGPPGPPGPPGPPSAGFDFSFLPQ PPQEKAHDGGRYYRANDANVVRDRDLEVDTTLKSLSQQIENIRSPE GSRKNPARTCRDLKMCHSDWKSGEYWIDPNQGCNLDAIKVFCNM ETGETCVYPTQPSVAQKNWYISKNPKDKRHVWFGESMTDGFQFEY GGQGSDPADVAIQLTFLRLMSTEASQNITYHCKNSVAYMDQQTGN LKKALLLKGSNEIEIRAEGNSRFTYSVTVDGCTSHTGAWGKTVIEY KTTKSSRLPIIDVAPLDVGAPDQEFGFDVGPVCFL | Trimerization peptide (type I) with glycine-X-Y repeats and D→N mutation at BMP-1 site, KS version |
| 112. | NGLPGPIGPPGPRGRTGDAGPVGPPGPPGPPGPPGPPSAGFDFSFLPQ PPQEKAHDGGRYYRNDDANVVRDRDLEVDTTLKSLSQQIENIRSPE GSRKNPARTCRDLKMCHSDWKSGEYWIDPNQGCNLDAIKVFCNM ETGETCVYPTQPSVAQKNWYISKNPKDKRHVWFGESMTDGFQFEY GGQGSDPADVAIQLTFLRLMSTEASQNITYHCKNSVAYMDQQTGN LKKALLLKGSNEIEIRAEGNSRFTYSVTVDGCTSHTGAWGKTVIEY KTTKSSRLPIIDVAPLDVGAPDQEFGFDVGPVCFL | Trimerization peptide (type I) with glycine-X-Y repeats and A→N mutation at BMP-1 site, KS version |
| 113. | RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLKGSNEIEIRAEGNSRFTYSVTVDGCTSHTGAWGKTVI EYKTTKSSRLPIIDVAPLDVGAPDQEFGFDVGPVCFL | Trimerization peptide (type I) with glycine-X-Y repeats and D→N mutation at BMP-1 site, KS version |
| 114. | GSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQF EYGGQGSDPADVAIQLTFLRLMSTEASQNITYHCKNSVAYMDQQT GNLKKALLLKGSNEIEIRAEGNSRFTYSVTVDGCTSHTGAWGKTVI EYKTTKSSRLPIIDVAPLDVGAPDQEFGFDVGPVCFL | Trimerization peptide (type I) with glycine-X-Y repeats and D→N mutation at BMP-1 site, KS version |
| 115. | DEIMTSLKSVNGQIESLISPDGSRKNPARNCRDLKFCHPELKSGEYW VDPNQGCKLDAIKVFCNMETGETCISANPLNVPRKHWWTDSSAEK KHVWFGESMDGGFQFSYGNPELPEDVLDVQLAFLRLLSSRASQNIT YHCKNSIAYMDQASGNVKKALKLMGSNEGEFKAEGNSKFTYTVL EDGCTKHTGEWSKTVFEYRTRKAVRLPIVDIAPYDIGGPDQEFGVD VGPVCF | Trimerization peptide (type III) |
| 116. | EPMDFKINTDEIMTSLKSVNGQIESLISPDGSRKNPARNCRDLKFCH PELKSGEYWVDPNQGCKLDAIKVFCNMETGETCISANPLNVPRKH WWTDSSAEKKHVWFGESMDGGFQFSYGNPELPEDVLDVQLAFLR LLSSRASQNITYHCKNSIAYMDQASGNVKKALKLMGSNEGEFKAE GNSKFTYTVLEDGCTKHTGEWSKTVFEYRTRKAVRLPIVDIAPYDI GGPDQEFGVDVGPVCFL | Trimerization peptide (type III) |
| 117. | SEPMDFKINTDEIMTSLKSVNGQIESLISPDGSRKNPARNCRDLKFC HPELKSGEYWVDPNQGCKLDAIKVFCNMETGETCISANPLNVPRK HWWTDSSAEKKHVWFGESMDGGFQFSYGNPELPEDVLDVQLAFL RLLSSRASQNITYHCKNSIAYMDQASGNVKKALKLMGSNEGEFKA EGNSKFTYTVLEDGCTKHTGEWSKTVFEYRTRKAVRLPIVDIAPYD IGGPDQEFGVDVGPVCFL | Trimerization peptide (type III) |
| 118. | RSEPMDFKINTDEIMTSLKSVNGQIESLISPDGSRKNPARNCRDLKF CHPELKSGEYWVDPNQGCKLDAIKVFCNMETGETCISANPLNVPR KHWWTDSSAEKKHVWFGESMDGGFQFSYGNPELPEDVLDVQLAF LRLLSSRASQNITYHCKNSIAYMDQASGNVKKALKLMGSNEGEFK AEGNSKFTYTVLEDGCTKHTGEWSKTVFEYRTRKAVRLPIVDIAPY DIGGPDQEFGVDVGPVCFL | Trimerization peptide (type III) |
| 119. | QNITEEFYQSTCSAVSRGYLSALRTGWYTSVITIELSNIKETKCNGT DTKVKLIKQELDKYKNAVTELQLLMQNTPAVNNRARR | RSV _subtype B fusion glycoprotein F2 (26-109 of SEQ ID NO: 1) |
| 120. | QNITEEFYQSTCSAVSRGYLSALRTGWYTSVITIELSNIKETKCNGT DTKVKLIKQELDKYKNAVTELQLLMQNTPAVNNCACR | RSV _subtype B fusion glycoprotein F2 mutant (26-109 of SEQ ID NO: 2) (R106C,R108C) |
| 139 | MELLILKANAITTILTAVTFCFASGQNITEEFYQSTCSAVSKGYLSAL RTGWYTSVITIELSNIKENKCNGTDAKVKLIKQELDKYKNAVTELQL LMQSTPATNNAAARELPRFMNYTLNNAKKTNVTLSKKAKAAFLGF LLGVGSAIASGVAVSKVLHLEGEVNKIKSALLSTNKAVVSLSNGVS VLTSKVLDLKNYIDKQLLPIVNKQSCSISNIATVIEFQQKNNRLLEITR EFSVNAGVTTPVSTYMLTNSELLSLINDMPITNDQKKLMSNNVQIVR QQSYSIMSIIKEEVLAYVVQLPLYGVIDTPCWKLHTSPLCTTNTKEGS NICLTRTDRGWYCDNAGSVSFFPQAETCKVQSNRVFCDTMNSLTLP SEVNLCNVDIFNPKYDCKIMTSKTDVSSSVITSLGAIVSCYGKTKCT ASNKNRGIIKTFSNGCDYVSNKGVDTVSVGNTLYYVNKQEGKSLY VKGEPIINFYDPLVFPSDEFDASISQVNEKINQSLAFIRKSDELLHNVN AGKRSNGLPGPIGPPGPRGRTGDAGPVGPPGPPGPPGPPGPPSAGFDF SFLPQPPQEKAHDGGRYYRANDANVVRDRDLEVDTTLKSLSQQIEN IRSPEGSRKNPARTCRDLKMCHSDWKSGEYWIDPNQGCNLDAIKVF CNMETGETCVYPTQPSVAQKNWYISKNPKDKRHVWFGESMTDGF QFEYGGQGSDPADVAIQLTFLRLMSTEASQNITYHCKNSVAYMDQ QTGNLKKALLLQGSNEIEIRAEGNSRFTYSVTVDGCTSHTGAWGKT VIEYKTTKTSRLPIIDVAPLDVGAPDQEFGFDVGPVCFL | SCB-N25A_A (R106A,R108A, R133A,R135A, R136A) |
| 140 | MELLILKANAITTILTAVTFCFASGQNITEEFYQSTCSAVSKGYLSAL RTGWYTSVITIELSNIKENKCNGTDAKVKLIKQELDKYKNAVTELQL LMQSTPATNNTATRELPRFMNYTLNNAKKTNVTLSKKTKTTFLGFL LGVGSAIASGVAVSKVLHLEGEVNKIKSALLSTNKAVVSLSNGVSV LTSKVLDLKNYIDKQLLPIVNKQSCSISNIATVIEFQQKNNRLLEITRE FSVNAGVTTPVSTYMLTNSELLSLINDMPITNDQKKLMSNNVQIVR QQSYSIMSIIKEEVLAYVVQLPLYGVIDTPCWKLHTSPLCTTNTKEGS NICLTRTDRGWYCDNAGSVSFFPQAETCKVQSNRVFCDTMNSLTLP SEVNLCNVDIFNPKYDCKIMTSKTDVSSSVITSLGAIVSCYGKTKCT ASNKNRGIIKTFSNGCDYVSNKGVDTVSVGNTLYYVNKQEGKSLY VKGEPIINFYDPLVFPSDEFDASISQVNEKINQSLAFIRKSDELLHNVN AGKRSNGLPGPIGPPGPRGRTGDAGPVGPPGPPGPPGPPGPPSAGFDF SFLPQPPQEKAHDGGRYYRANDANVVRDRDLEVDTTLKSLSQQIEN IRSPEGSRKNPARTCRDLKMCHSDWKSGEYWIDPNQGCNLDAIKVF CNMETGETCVYPTQPSVAQKNWYISKNPKDKRHVWFGESMTDGF QFEYGGQGSDPADVAIQLTFLRLMSTEASQNITYHCKNSVAYMDQ QTGNLKKALLLQGSNEIEIRAEGNSRFTYSVTVDGCTSHTGAWGKT VIEYKTTKTSRLPIIDVAPLDVGAPDQEFGFDVGPVCFL | SCB-N25T_A (R106T,R108T, R133T,R135T,R 136T) |
| 141 | MELLILKANAITTILTAVTFCFASGQNITEEFYQSTCSAVSKGYLSAL RTGWYTSVITIELSNIKENKCNGTDAKVKLIKQELDKYKNAVTELQL LMQSTPATNNYAYRELPRFMNYTLNNAKKTNVTLSKKYKYYFLGF LLGVGSAIASGVAVSKVLHLEGEVNKIKSALLSTNKAVVSLSNGVS VLTSKVLDLKNYIDKQLLPIVNKQSCSISNIATVIEFQQKNNRLLEITR EFSVNAGVTTPVSTYMLTNSELLSLINDMPITNDQKKLMSNNVQIVR QQSYSIMSIIKEEVLAYVVQLPLYGVIDTPCWKLHTSPLCTTNTKEGS NICLTRTDRGWYCDNAGSVSFFPQAETCKVQSNRVFCDTMNSLTLP SEVNLCNVDIFNPKYDCKIMTSKTDVSSSVITSLGAIVSCYGKTKCT ASNKNRGIIKTFSNGCDYVSNKGVDTVSVGNTLYYVNKQEGKSLY VKGEPIINFYDPLVFPSDEFDASISQVNEKINQSLAFIRKSDELLHNVN AGKRSNGLPGPIGPPGPRGRTGDAGPVGPPGPPGPPGPPGPPSAGFDF SFLPQPPQEKAHDGGRYYRANDANVVRDRDLEVDTTLKSLSQQIEN IRSPEGSRKNPARTCRDLKMCHSDWKSGEYWIDPNQGCNLDAIKVF CNMETGETCVYPTQPSVAQKNWYISKNPKDKRHVWFGESMTDGF QFEYGGQGSDPADVAIQLTFLRLMSTEASQNITYHCKNSVAYMDQ QTGNLKKALLLQGSNEIEIRAEGNSRFTYSVTVDGCTSHTGAWGKT VIEYKTTKTSRLPIIDVAPLDVGAPDQEFGFDVGPVCFL | SCB-N25Y_A (R106Y,R108Y, R133Y,R135Y, R136Y) |
| 142 | MELLILKANAITTILTAVTFCFASGQNITEEFYQSTCSAVSKGYLSAL RTGWYTSVITIELSNIKENKCNGTDAKVKLIKQELDKYKNAVTELQL LMQSTPATNNGAGRELPRFMNYTLNNAKKTNVTLSKKGKGGFLGF LLGVGSAIASGVAVSKVLHLEGEVNKIKSALLSTNKAVVSLSNGVS VLTSKVLDLKNYIDKQLLPIVNKQSCSISNIATVIEFQQKNNRLLEITR EFSVNAGVTTPVSTYMLTNSELLSLINDMPITNDQKKLMSNNVQIVR QQSYSIMSIIKEEVLAYVVQLPLYGVIDTPCWKLHTSPLCTTNTKEGS NICLTRTDRGWYCDNAGSVSFFPQAETCKVQSNRVFCDTMNSLTLP SEVNLCNVDIFNPKYDCKIMTSKTDVSSSVITSLGAIVSCYGKTKCT ASNKNRGIIKTFSNGCDYVSNKGVDTVSVGNTLYYVNKQEGKSLY VKGEPIINFYDPLVFPSDEFDASISQVNEKINQSLAFIRKSDELLHNVN AGKRSNGLPGPIGPPGPRGRTGDAGPVGPPGPPGPPGPPGPPSAGFDF SFLPQPPQEKAHDGGRYYRANDANVVRDRDLEVDTTLKSLSQQIEN IRSPEGSRKNPARTCRDLKMCHSDWKSGEYWIDPNQGCNLDAIKVF CNMETGETCVYPTQPSVAQKNWYISKNPKDKRHVWFGESMTDGF QFEYGGQGSDPADVAIQLTFLRLMSTEASQNITYHCKNSVAYMDQ QTGNLKKALLLQGSNEIEIRAEGNSRFTYSVTVDGCTSHTGAWGKT VIEYKTTKTSRLPIIDVAPLDVGAPDQEFGFDVGPVCFL | SCB-N25G_A (R106G,R108G, R133G,R135G, R136G) |
| 143 | MELLILKANAITTILTAVTFCFASGQNITEEFYQSTCSAVSKGYLSAL RTGWYTSVITIELSNIKENKCNGTDAKVKLIKQELDKYKNAVTELQL LMQSTPATNNSASRELPRFMNYTLNNAKKTNVTLSKKSKSSFLGFL LGVGSAIASGVAVSKVLHLEGEVNKIKSALLSTNKAVVSLSNGVSV LTSKVLDLKNYIDKQLLPIVNKQSCSISNIATVIEFQQKNNRLLEITRE FSVNAGVTTPVSTYMLTNSELLSLINDMPITNDQKKLMSNNVQIVR QQSYSIMSIIKEEVLAYVVQLPLYGVIDTPCWKLHTSPLCTTNTKEGS NICLTRTDRGWYCDNAGSVSFFPQAETCKVQSNRVFCDTMNSLTLP SEVNLCNVDIFNPKYDCKIMTSKTDVSSSVITSLGAIVSCYGKTKCT ASNKNRGIIKTFSNGCDYVSNKGVDTVSVGNTLYYVNKQEGKSLY VKGEPIINFYDPLVFPSDEFDASISQVNEKINQSLAFIRKSDELLHNVN AGKRSNGLPGPIGPPGPRGRTGDAGPVGPPGPPGPPGPPGPPSAGFDF SFLPQPPQEKAHDGGRYYRANDANVVRDRDLEVDTTLKSLSQQIEN IRSPEGSRKNPARTCRDLKMCHSDWKSGEYWIDPNQGCNLDAIKVF CNMETGETCVYPTQPSVAQKNWYISKNPKDKRHVWFGESMTDGF QFEYGGQGSDPADVAIQLTFLRLMSTEASQNITYHCKNSVAYMDQ QTGNLKKALLLQGSNEIEIRAEGNSRFTYSVTVDGCTSHTGAWGKT VIEYKTTKTSRLPIIDVAPLDVGAPDQEFGFDVGPVCFL | SCB-N25S_A (R106S,R108S, R133S,R135S,R 136S) |
| 144 | MELLIHRSSAIFLTLAINALYLTSSQNITEEFYQSTCSAVSRGYLSALR TGWYTSVITIELSNIKETKCNGTDTKVKLIKQELDKYKNAVTELQLL MQNTPAVNNAAAREAPQYMNYTINTTKNLNVSISKKAKAAFLGFL LGVGSAIASGIAVSKVLHLEGEVNKIKNALQLTNKAVVSLSNGVSV LTSRVLDLKNYINNQLLPMVNRQSCRISNIETVIEFQQKNSRLLEITR EFSVNAGVTTPLSTYMLTNSELLSLINDMPITNDQKKLMSSNVQIVR QQSYSIMSIIKEEVLAYVVQLPIYGVIDTPCWKLHTSPLCTTNIKEGS NICLTRTDRGWYCDNAGSVSFFPQADTCKVQSNRVFCDTMNSLTLP SEVSLCNTDIFNSKYDCKIMTSKTDISSSVITSLGAIVSCYGKTKCTAS NKNRGIIKTFSNGCDYVSNKGVDTVSVGNTLYYVNKLEGKNLYVK GEPIINYYDPLVFPSDEFDASISQVNEKINQSLAFIRRSDELLHNVNTG KRSNGLPGPIGPPGPRGRTGDAGPVGPPGPPGPPGPPGPPSAGFDFSF LPQPPQEKAHDGGRYYRANDANVVRDRDLEVDTTLKSLSQQIENIR SPEGSRKNPARTCRDLKMCHSDWKSGEYWIDPNQGCNLDAIKVFC NMETGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVIE YKTTKTSRLPIIDVAPLDVGAPDQEFGFDVGPVCFL | SCB-N25A_B (R106A,R108A, R133A,R135A, R136A) |
| 145 | MELLIHRSSAIFLTLAINALYLTSSQNITEEFYQSTCSAVSRGYLSALR TGWYTSVITIELSNIKETKCNGTDTKVKLIKQELDKYKNAVTELQLL MQNTPAVNNTATREAPQYMNYTINTTKNLNVSISKKTKTTFLGFLL GVGSAIASGIAVSKVLHLEGEVNKIKNALQLTNKAVVSLSNGVSVL TSRVLDLKNYINNQLLPMVNRQSCRISNIETVIEFQQKNSRLLEITRE FSVNAGVTTPLSTYMLTNSELLSLINDMPITNDQKKLMSSNVQIVRQ QSYSIMSIIKEEVLAYVVQLPIYGVIDTPCWKLHTSPLCTTNIKEGSNI CLTRTDRGWYCDNAGSVSFFPQADTCKVQSNRVFCDTMNSLTLPSE VSLCNTDIFNSKYDCKIMTSKTDISSSVITSLGAIVSCYGKTKCTASN KNRGIIKTFSNGCDYVSNKGVDTVSVGNTLYYVNKLEGKNLYVKG EPIINYYDPLVFPSDEFDASISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQFE YGGQGSDPADVAIQLTFLRLMSTEASQNITYHCKNSVAYMDQQTG NLKKALLLQGSNEIEIRAEGNSRFTYSVTVDGCTSHTGAWGKTVIEY KTTKTSRLPIIDVAPLDVGAPDQEFGFDVGPVCFL | SCB-N25T_B (R106T,R108T, R133T,R135T,R 136T) |
| 146 | MELLIHRSSAIFLTLAINALYLTSSQNITEEFYQSTCSAVSRGYLSALR TGWYTSVITIELSNIKETKCNGTDTKVKLIKQELDKYKNAVTELQLL MQNTPAVNNYAYREAPQYMNYTINTTKNLNVSISKKYKYYFLGFL LGVGSAIASGIAVSKVLHLEGEVNKIKNALQLTNKAVVSLSNGVSV LTSRVLDLKNYINNQLLPMVNRQSCRISNIETVIEFQQKNSRLLEITR EFSVNAGVTTPLSTYMLTNSELLSLINDMPITNDQKKLMSSNVQIVR QQSYSIMSIIKEEVLAYVVQLPIYGVIDTPCWKLHTSPLCTTNIKEGS NICLTRTDRGWYCDNAGSVSFFPQADTCKVQSNRVFCDTMNSLTLP SEVSLCNTDIFNSKYDCKIMTSKTDISSSVITSLGAIVSCYGKTKCTAS NKNRGIIKTFSNGCDYVSNKGVDTVSVGNTLYYVNKLEGKNLYVK GEPIINYYDPLVFPSDEFDASISQVNEKINQSLAFIRRSDELLHNVNTG KRSNGLPGPIGPPGPRGRTGDAGPVGPPGPPGPPGPPGPPSAGFDFSF LPQPPQEKAHDGGRYYRANDANVVRDRDLEVDTTLKSLSQQIENIR SPEGSRKNPARTCRDLKMCHSDWKSGEYWIDPNQGCNLDAIKVFC NMETGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVIE YKTTKTSRLPIIDVAPLDVGAPDQEFGFDVGPVCFL | SCB-N25Y_B (R106Y,R108Y, R133Y,R135Y, R136Y) |
| 147 | MELLIHRSSAIFLTLAINALYLTSSQNITEEFYQSTCSAVSRGYLSALR TGWYTSVITIELSNIKETKCNGTDTKVKLIKQELDKYKNAVTELQLL MQNTPAVNNGAGREAPQYMNYTINTTKNLNVSISKKGKGGFLGFL LGVGSAIASGIAVSKVLHLEGEVNKIKNALQLTNKAVVSLSNGVSV LTSRVLDLKNYINNQLLPMVNRQSCRISNIETVIEFQQKNSRLLEITR EFSVNAGVTTPLSTYMLTNSELLSLINDMPITNDQKKLMSSNVQIVR QQSYSIMSIIKEEVLAYVVQLPIYGVIDTPCWKLHTSPLCTTNIKEGS NICLTRTDRGWYCDNAGSVSFFPQADTCKVQSNRVFCDTMNSLTLP SEVSLCNTDIFNSKYDCKIMTSKTDISSSVITSLGAIVSCYGKTKCTAS NKNRGIIKTFSNGCDYVSNKGVDTVSVGNTLYYVNKLEGKNLYVK GEPIINYYDPLVFPSDEFDASISQVNEKINQSLAFIRRSDELLHNVNTG KRSNGLPGPIGPPGPRGRTGDAGPVGPPGPPGPPGPPGPPSAGFDFSF LPQPPQEKAHDGGRYYRANDANVVRDRDLEVDTTLKSLSQQIENIR SPEGSRKNPARTCRDLKMCHSDWKSGEYWIDPNQGCNLDAIKVFC NMETGETCVYPTQPSVAQKNWYISKNPKDKRHVWFGESMTDGFQF EYGGQGSDPADVAIQLTFLRLMSTEASQNITYHCKNSVAYMDQQT GNLKKALLLQGSNEIEIRAEGNSRFTYSVTVDGCTSHTGAWGKTVIE YKTTKTSRLPIIDVAPLDVGAPDQEFGFDVGPVCFL | SCB-N25G_B (R106G,R108G, R133G,R135G, R136G) |
| 148 | MELLIHRSSAIFLTLAINALYLTSSQNITEEFYQSTCSAVSRGYLSALR TGWYTSVITIELSNIKETKCNGTDTKVKLIKQELDKYKNAVTELQLL MQNTPAVNNSASREAPQYMNYTINTTKNLNVSISKKSKSSFLGFLL GVGSAIASGIAVSKVLHLEGEVNKIKNALQLTNKAVVSLSNGVSVL TSRVLDLKNYINNQLLPMVNRQSCRISNIETVIEFQQKNSRLLEITRE FSVNAGVTTPLSTYMLTNSELLSLINDMPITNDQKKLMSSNVQIVRQ QSYSIMSIIKEEVLAYVVQLPIYGVIDTPCWKLHTSPLCTTNIKEGSNI CLTRTDRGWYCDNAGSVSFFPQADTCKVQSNRVFCDTMNSLTLPSE VSLCNTDIFNSKYDCKIMTSKTDISSSVITSLGAIVSCYGKTKCTASN KNRGIIKTFSNGCDYVSNKGVDTVSVGNTLYYVNKLEGKNLYVKG EPIINYYDPLVFPSDEFDASISQVNEKINQSLAFIRRSDELLHNVNTGK RSNGLPGPIGPPGPRGRTGDAGPVGPPGPPGPPGPPGPPSAGFDFSFL PQPPQEKAHDGGRYYRANDANVVRDRDLEVDTTLKSLSQQIENIRS PEGSRKNPARTCRDLKMCHSDWKSGEYWIDPNQGCNLDAIKVFCN METGETCVYPTQPSVAQKNWYISKNPKDKRHVWFGESMTDGFQFE YGGQGSDPADVAIQLTFLRLMSTEASQNITYHCKNSVAYMDQQTG NLKKALLLQGSNEIEIRAEGNSRFTYSVTVDGCTSHTGAWGKTVIEY KTTKTSRLPIIDVAPLDVGAPDQEFGFDVGPVCFL | SCB-N25S_B (R106S,R108S, R133S,R135S,R 136S) |

## Claims

1. A fusion protein comprising a soluble RSV viral surface antigen linked to a collagen trimerization domain, **characterized in that** the soluble RSV viral surface antigen comprises a recombinant F peptide, or a fragment or epitope thereof.

2. The fusion protein according to claim 1, **characterized in that** RSV virus is of subtype A and/or subtype B.

3. The fusion protein according to claim 1 or 2, **characterized in that** the collagen trimerization domain is a collagen C-terminal propeptide.

4. The fusion protein according to any one of claims 1-3, **characterized in that** the collagen trimerization domain comprises a C-terminal polypeptide of proα1(I), proα1(II), proα1(III), proα1(V), proα1(XI), proα2(I), proα2(V), proα2(XI), or proα3(XI), or a fragment thereof.

5. The fusion protein according to any one of claims 1-4, **characterized in that** the collagen trimerization domain comprises the sequence of any one of SEQ ID NOs: 103-118, or an amino acid sequence having at least 90% identity thereto, capable of forming inter-polypeptide disulfide bonds and trimerizing the fusion protein.

6. The fusion protein according to any one of claims 1-5, **characterized in that** the recombinant F peptide, or a fragment or epitope thereof has one or more mutation sites, and the F peptide, or a fragment or epitope thereof comprises a sulfur-containing amino acid or a sulfur-containing amino acid derivative at the one or more mutation sites.

7. The fusion protein according to claim 6, **characterized in that** the recombinant F peptide, or a fragment or epitope thereof comprises an F2 peptide, or a fragment or epitope thereof, and an F1 peptide, or a fragment or epitope thereof.

8. The fusion protein according to claim 6 or 7, **characterized in that** the recombinant F peptide, or a fragment or epitope thereof comprises a pep27 peptide.

9. The fusion protein according to any one of claims 6-8, **characterized in that** the recombinant F peptide, or a fragment or epitope thereof comprises one or more mutation sites in the F2 peptide and/or pep27 peptide.

10. The fusion protein according to any one of claims 6-9, **characterized in that** the recombinant F peptide, or a fragment or epitope thereof comprises an amino acid substitution at one or more or all of amino acid residues 106, 108, 133, 135, and 136.

11. The fusion protein according to any one of claims 6-10, **characterized in that** the sulfur-containing amino acid is cysteine.

12. The fusion protein according to any one of claims 6-11, **characterized in that** the recombinant F peptide, or a fragment or epitope thereof comprises the amino acid substitutions R106C, R108C, R133C, R135C, and R136C.

13. The fusion protein according to any one of claims 6-12, **characterized in that** the recombinant F peptide, or a fragment or epitope thereof comprises the sequence of any one of SEQ ID NOs: 11-20 and 31-40, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 11-20 and 31-40.

14. The fusion protein according to any one of claims 6-13, **characterized in that** the fusion protein comprises the sequence of any one of SEQ ID NOs: 1-10 and 21-30, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 1-10 and 21-30.

15. The fusion protein according to any one of claims 1-5, **characterized in that** the recombinant F peptide, or a fragment or epitope thereof comprises an F1 peptide and an F2 peptide of the F peptide, wherein the F1 peptide and the F2 peptide are linked by an artificially introduced linker, the artificially introduced linker being the amino acid sequence CGGG (SEQ ID NO: 138).

16. The fusion protein according to claim 15, **characterized in that** the F2 peptide comprises an amino acid sequence from amino acid residues at position 26 to position 105 of the F0 precursor, or an amino acid sequence from amino acid residues at position 1 to position 105 of the F0 precursor.

17. The fusion protein according to claim 15 or 16, **characterized in that** the F1 peptide comprises an amino acid sequence from amino acid residue at position 105 to any one selected from amino acid residues at positions 513-524 of the F0 precursor.

18. The fusion protein according to any one of claims 15-17, **characterized in that** the F0 precursor comprises the sequence of SEQ ID NO: 67 or 69, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of SEQ ID NO: 67 or 69.

19. The fusion protein according to any one of claims 15-18, **characterized in that** the recombinant F peptide, or a fragment or epitope thereof comprises the sequence of any one of SEQ ID NOs: 49-50 and 62-63, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 49-50 and 62-63.

20. The fusion protein according to any one of claims 15-19, **characterized in that** the fusion protein comprises the sequence of any one of SEQ ID NOs: 41-48 and 54-61, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 41-48 and 54-61.

21. A polynucleotide encoding the fusion protein according to any one of claims 1-20.

22. A vector comprising the polynucleotide according to claim 21.

23. A host cell comprising the polynucleotide according to claim 21 or the vector according to claim 22.

24. An immunogenic composition comprising the fusion protein according to any one of claims 1-20.

25. The immunogenic composition according to claim 24, which comprises one or more adjuvants, or does not comprise an adjuvant.

26. The immunogenic composition according to claim 25, **characterized in that** the adjuvant is alum.

27. The immunogenic composition according to any one of claims 24-26, which is multivalent, and comprises two or more of the fusion proteins, each fusion protein being a fusion protein formed by linking a soluble RSV viral surface antigen of a different RSV virus subtype or strain to a collagen trimerization domain.

28. The immunogenic composition according to claim 27, which comprises a fusion protein formed by linking a soluble RSV viral surface antigen of RSV subtype A to a collagen trimerization domain, and a fusion protein formed by linking a soluble RSV viral surface antigen of RSV subtype B to a collagen trimerization domain.

29. The immunogenic composition according to claim 28, **characterized in that** the fusion protein formed by linking a soluble RSV viral surface antigen of RSV subtype A to a collagen trimerization domain comprises the sequence of any one of SEQ ID NOs: 1-10 and 41-48, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 1-10 and 41-48.

30. The immunogenic composition according to claim 28 or 29, **characterized in that** the fusion protein formed by linking a soluble RSV viral surface antigen of RSV subtype B to a collagen trimerization domain comprises the sequence of any one of SEQ ID NOs: 21-30 and 54-61, or an amino acid sequence having at least about 80%, 81%, 82%, 83%, 84%, 85%, 86%, 87%, 88%, 89%, 90%, 91%, 92%, 93%, 94%, 95%, 96%, 97%, 98%, or 99% sequence identity to the sequence of any one of SEQ ID NOs: 21-30 and 54-61.

31. A method for preventing or treating respiratory syncytial virus (RSV) infection, comprising administering to a subject the fusion protein according to any one of claims 1-20, the polynucleotide according to claim 21, the vector according to claim 22, the host cell according to claim 23, or the immunogenic composition according to any one of claims 24-30.

32. The method according to claim 31, **characterized in that** the administration is performed by intramuscular injection for immunization or by intranasal spray for immunization.

33. The method according to claim 31 or 32, **characterized in that** the administration is performed in a single dose or in a series of doses separated by intervals of weeks or months for immunization.
